# EUROPÄISCHE PATENTSCHRIFT

(11) **EP 1 181 295 B1**
(45) Veröffentlichungstag und Bekanntmachung des Hinweises auf die Patenterteilung: **18.02.2004**
(21) Anmeldenummer: 00940273.6
(22) Anmeldetag: 26.05.2000
(51) Int. Cl.: C07D 495/04, C07D 498/04, A01N 43/90

(54) **TRICYCLISCHE BENZOYLCYCLOHEXANDION-DERIVATE**
TRICYCLIC BENZOYLCYCLOHEXANEDIONE DERIVATIVES
DERIVES TRICYCLIQUES DE BENZOYLCYCLOHEXANEDIONES

(30) Priorität: 01.06.1999 DE 19925103
(43) Veröffentlichungstag der Anmeldung: 27.02.2002
(73) Patentinhaber: BASF AKTIENGESELLSCHAFT, 67056 Ludwigshafen (DE)
(72) Erfinder: WITSCHEL, Matthias, 67098 Bad Dürkheim (DE); KUDIS, Steffen, D-68167 Mannheim (DE); LANGEMANN, Klaus, D-67551 Worms (DE); BAUMANN, Ernst, D-67373 Dudenhofen (DE); DEYN, Wolfgang von, D-67435 Neustadt (DE); MAYER, Guido, D-67433 Neustadt (DE); MISSLITZ, Ulf, D-67433 Neustadt (DE); NEIDLEIN, Ulf, D-68165 Mannheim (DE); OTTEN, Martina, D-67069 Ludwigshafen (DE); WESTPHALEN, Karl-Otto, D-67346 Speyer (DE); WALTER, Helmut, D-67283 Obrigheim (DE)
(86) Internationale Anmeldenummer: PCT/EP2000/004806
(87) Internationale Veröffentlichungsnummer: WO 2000/073311

(56) Entgegenhaltungen:
- EP-A- 0 283 261
- EP-A- 0 860 441
- WO-A-97/19087

## Beschreibung

Die vorliegende Erfindung betrifft neue tricyclische Benzoylcyclohexandion-Derivate der Formel I 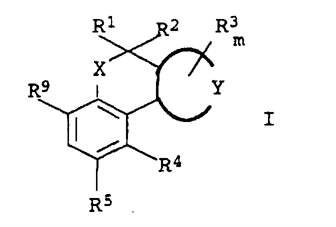 in der die Variablen folgende Bedeutungen haben:
- X: Sauerstoff, Schwefel, S=O oder S(=O)₂;
- Y: bildet gemeinsam mit den beiden Kohlenstoffen, an die es gebunden ist, einen gesättigten, partiell gesättigten oder ungesättigten 5-gliedrigen Heterocyclus, der ein bis drei gleiche oder verschiedene Heteroatome, ausgewählt aus folgender Gruppe: Sauerstoff, Schwefel oder Stickstoff, enthält;
- R¹,R²: Wasserstoff, C₁-C₆-Alkyl, C₁-C₆-Halogenalkyl, C₁-C₆-Alkoxy oder C₁-C₆-Halogenalkoxy;
- R³: Halogen, C₁-C₆-Alkyl, C₁-C₆-Halogenalkyl, C₁-C₆-Alkoxy oder C₁-C₆-Halogenalkoxy;
- R⁴: Nitro, Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, Aminosulfonyl, N-(C₁-C₆-Alkyl)-aminosulfonyl, N,N-Di-(C₁-C₆-alkyl)-aminosulfonyl, N-(C₁-C₆-Alkylsulfonyl)-amino, N-(C₁-C₆-Halogenalkylsulfonyl)-amino, N-(C₁-C₆-Alkyl)-N-(C₁-C₆-alkylsulfonyl)-amino oder N-(C₁-C₆-Alky)-N-(C₁-C₆-halogenalkylsulfonyl)-amino;
- R⁵: Wasserstoff, C₁-C₆-Alkyl oder Halogen;
- m: 0, 1 oder 2;
- R⁹: ein Rest IIa oder IIb 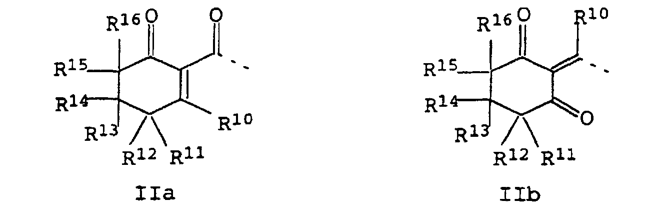
wobei die Variablen folgende Bedeutung haben:
- R¹⁰: Hydroxy;
- R¹¹, R¹⁵: Wasserstoff, C₁-C₄-Alkyl oder C₁-C₄-Alkoxycarbonyl;
- R¹², R¹⁴, R¹⁶: Wasserstoff oder C₁-C₄-Alkyl;
- R¹³: Wasserstoff, Halogen, Hydroxy, C₁-C₆-Alkyl, C₁-C₆-Halogenalkyl, Di-(C₁-C₆-alkoxy)-methyl, (C₁-C₆-Alkoxy)-(C₁-C₆-alkylthio)-methyl, Di-(C₁-C₆-alkylthio)methyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, C₁-C₆-Alkoxycarbonyl, C₁-C₆-Halogenalkoxycarbonyl;
1,3-Dioxolan-2-yl, 1,3-Dioxan-2-yl, 1,3-Oxathiolan-2-yl, 1,3-Oxathian-2-yl, 1,3-Dithiolan-2-yl oder 1,3-Dithian-2-yl, wobei die sechs letztgenannten Reste durch einen bis drei C₁-C₄-Alkylreste substituiert sein können;
oder
R¹² und R¹³ oder R¹³ und R¹⁶ bilden gemeinsam eine π-Bindung oder eine C₁-C₅-Alkylkette, die einen bis drei Reste aus folgender Gruppe tragen kann: Halogen, Cyano, C₁-C₄-Alkyl, C₁-C₄-Halogenalkyl oder C₁-C₄-Alkoxycarbonyl, aus;
oder
R¹² und R¹⁶ bilden gemeinsam eine C₁-C₄-Alkylkette, die einen bis drei Reste aus folgender Gruppe tragen kann: Halogen, Cyano, C₁-C₄-Alkyl. C₁-C₄-Halogenalkyl oder C₁-C₄-Alkoxycarbonyl, aus;
oder
R¹³ und R¹⁴ bilden gemeinsam eine -O-(CH₂)ₚ-O-, -O-(CH₂)ₚ-S-, -S-(CH₂)ₚ-S-, -O-(CH₂)_{q}- oder -S-(CH₂)_{q}-Kette, die durch einen bis drei Reste aus folgender Gruppe substituiert sein kann: Halogen, Cyano, C₁-C₄-Alkyl, C₁-C₄-Halogenalkyl oder C₁-C₄-Alkoxycarbonyl;
oder
R¹³ und R¹⁴ bilden gemeinsam mit dem Kohlenstoff, an dem sie gebunden sind eine Carbonylgruppe aus;
- p: 2, 3 oder 4;
- q: 1, 2, 3, 4 oder 5
sowie deren landwirtschaftlich brauchbaren Salze.

Außerdem betrifft die Erfindung Verfahren zur Herstellung von Verbindungen der Formel I, Mittel welche diese enthalten, sowie die Verwendung dieser Derivate oder diese enthaltende Mittel zur Schadpflanzenbekämpfung.

Aus WO 97/19087 und EP-A 860 441 sind tricyclische Verbindungen bekannt, die dadurch charakterisiert sind, daß die jeweils enthaltene Benzoyleinheit über die Positionen 3 und 4 mit einem Bicyclus anelliert ist. Die herbiziden Eigenschaften der bisher bekannten Verbindungen sowie die Verträglichkeiten gegenüber Kulturpflanzen können jedoch nur bedingt befriedigen. Es lag daher dieser Erfindung die Aufgabe zugrunde, neue, biologisch, insbesondere herbizid wirksame, Verbindungen mit verbesserten Eigenschaften zu finden.

Demgemäß wurden die tricyclischen Benzoylcyclohexandion-Derivate der Formel I sowie deren herbizide Wirkung gefunden.

Ferner wurden Verfahren zur Synthese der Verbindungen der Formel I gefunden. Ebenso wurden herbizide Mittel gefunden, die die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Die Verbindungen der Formel I können je nach Substitutionsmuster ein oder mehrere Chiralitätszentren enthalten und liegen dann als Enantiomeren oder Diastereomerengemische vor. Gegenstand der Erfindung sind sowohl die reinen Enantiomeren oder Diastereomeren als auch deren Gemische.

Die Verbindungen der Formel I können auch in Form ihrer landwirtschaftlich brauchbaren Salze vorliegen, wobei es auf die Art des Salzes in der Regel nicht ankommt. Im Allgemeinen kommen die Salze derjenigen Kationen oder die Säureadditionssalze derjenigen Säuren in Betracht, deren Kationen, beziehungsweise Anionen, die herbizide Wirkung der Verbindungen I nicht negativ beeinträchtigen.

Es kommen als Kationen insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschtenfalls ein bis vier Wasserstoffatome durch C₁-C₄-Alkyl, Hydroxy-C₁-C₄-alkyl, C₁-C₄-Alkoxy-C₁-C₄-alkyl, Hydroxy-C₁-C₄-alkoxy-C₁-C₄-alkyl, Phenyl oder Benzyl ersetzt sein können, vorzugsweise Ammonium, Dimethylammonium, Diisopropylammonium, Tetramethylammonium, Tetrabutylammonium, 2-(2-Hydroxyeth-1-oxy)eth-1-ylammonium, Di(2-hydroxyeth-1-yl)ammonium, Trimethylbenzylammonium, des weiteren Phosphoniumionen, Sulfoniumionen, vorzugsweise Tri(C₁-C₄-alkyl)sulfonium und Sulfoxoniumionen, vorzugsweise Tri(C₁-C₄-alkyl)sulfoxonium, in Betracht.

Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat und Butyrat.

Im Falle von R¹⁰ = Hydroxy steht IIa auch stellvertretend für die tautomeren Formen IIa', IIa'' und IIa''' 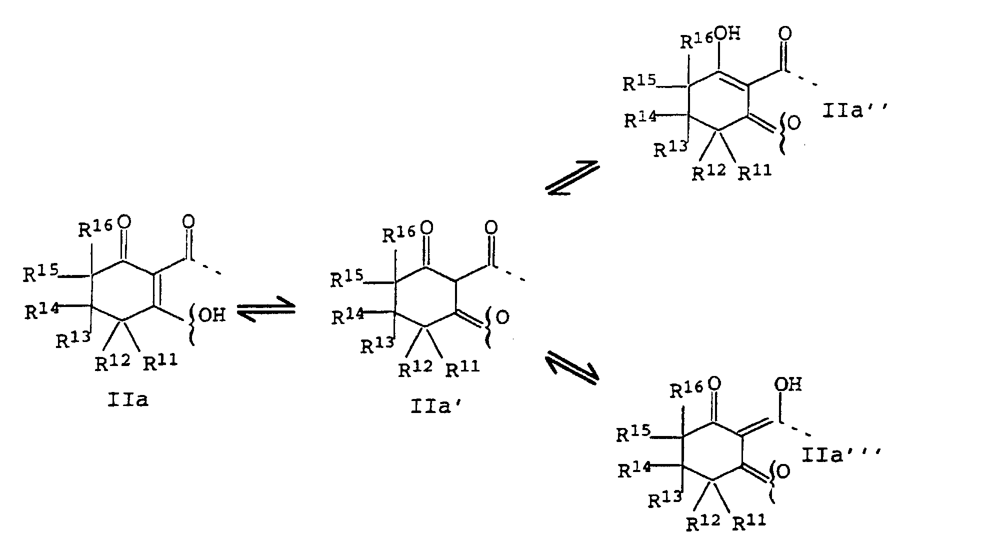 bzw. IIb auch stellvertretend für die tautomeren Formen IIb', IIb'' und IIb'''.

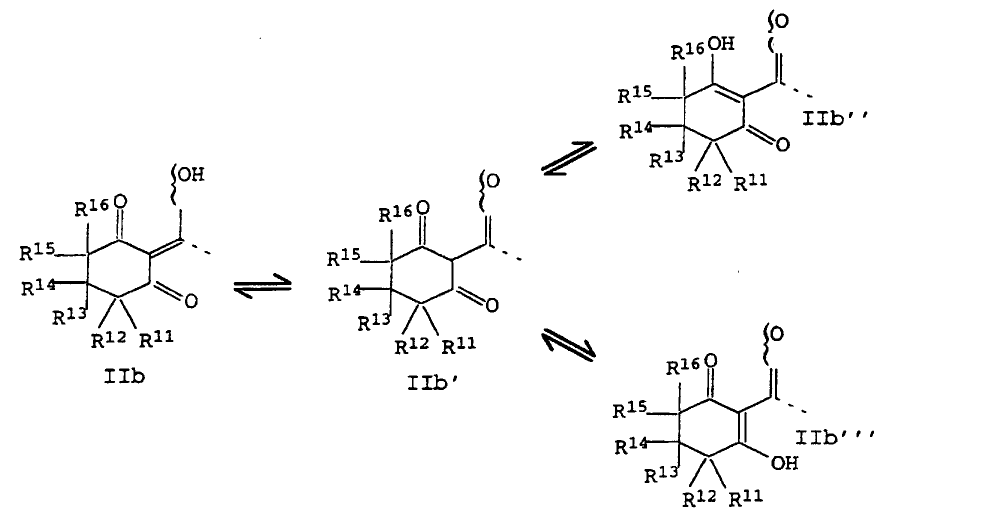

Die für die Substituenten R¹-R¹⁶ oder als Reste an Phenyl- und Heterocyclyl-Resten genannten organischen Molekülteile, sowie alle weiteren in dieser Anmeldung aufgeführten Reste stellen Sammelbegriffe für individuelle Aufzählungen der einzelnen Gruppenmitglieder dar. Sämtliche Kohlenwasserstoffketten, also alle Alkyl-, Halogenalkyl-, Hydroxyalkyl, Di(alkoxy)methyl, (Alkoxy)(alkylthio)methyl, Di(alkylthio)methyl, Alkoxy-, Halogenalkoxy-, Alkylthio-, Halogenalkylthio-, Alkylsulfinyl-, Halogenalkylsulfinyl-, Alkylsulfonyl-, Halogenalkylsulfonyl-, N-Alkylaminosulfonyl-, N,N-Dialkylaminosulfonyl-, Trialkylsulfonium, Trialkylsulfoxonium, N-Alkylamino-, N,N-Dialkylamino-, Alkylcarbonylamino, N-Alkylsulfor-ylamino-, N-Halogenalkylsulfonylamino-, N-Alkyl-N-alkylsulfonylamino-, N-Alkyl-N-halogenalkylsulfonylamino-, Alkylcarbonyl-, Alkoxycarbonyl-, Halogenalkyoxycarbonyl, Alkylthiocarbonyl-, Alkylcarbonyloxy-, Alkylaminocarbonyl-, Dialkylaminocarbonyl-, Dialkylaminothiocarbonyl-, Alkoxyalkyl-, Hydroxyalkoxyalkyl, Alkoxyiminoalkyl-, Phenylalkenylcarbonyl-, Heterocyclylalkenylcarbonyl-, N-Alkoxy-N-alkylaminocarbonyl-, N-Alkyl-N-phenylaminocarbonyl-, N-Alkyl-N-heterocyclylaminocarbonyl-, Phenylalkyl-, Heterocyclylalkyl-, Phenylcarbonylalkyl-, Heterocyclylcarbonylalkyl-, Alkoxyalkoxycarbonyl-, Alkenylcarbonyl-, Alkenyloxycarbonyl-, Alkenylaminocarbonyl-, N-Alkenyl-N-alkylaminocarbonyl-, N-Alkenyl-N-alkoxyaminocarbonyl-, Alkinylcarbonyl-, Alkinyloxycarbonyl-, Alkinylaminocarbonyl-, N-Alkinyl-N-alkylaminocarbonyl-, N-Alkinyl-N-alkoxyaminocarbonyl-, Alkenyl-, Alkinyl-, Halogenalkenyl-, Halogenalkinyl-, Alkenyloxyund Alkinyloxy-Teile können geradkettig oder verzweigt sein. Sofern nicht anders angegeben tragen halogenierte Substituenten vorzugsweise ein bis fünf gleiche oder verschiedene Halogenatome. Die Bedeutung Halogen steht jeweils für Fluor, Chlor, Brom oder Iod.

Ferner bedeuten beispielsweise:
- C₁-C₄-Alkyl sowie die Alkylteile von Hydroxy-C₁-C₄-alkyl, Tri-(C₁-C₄-alkyl)sulfonium und Tri-(C₁-C₄-alkyl)sulfoxonium: z.B. Methyl, Ethyl, Propyl, 1-Methylethyl, Butyl, 1-Methylpropyl, 2-Methylpropyl oder 1,1-Dimethylethyl;
- C₁-C₆-Alkyl, sowie die Alkylteile von, C₁-C₆-Alkoxyimino-C₁-C₆-alkyl, N-(C₁-C₆-Alkoxy)-N-(C₁-C₆-alkyl)-aminocarbonyl, N-(C₃-C₆-Alkenyl)-N-(C₁-C₆-alkyl)-aminocarbonyl, N-(C₃-C₆-Alkinyl)-N-(C₁-C₆-alkyl)-aminocarbonyl, N-(C₁-C₆-Alkyl)-N-phenylaminocarbonyl, N-(C₁-C₆-Alkyl)-N-heterocyclylaminocarbonyl, Phenyl-C₁-C₆-alkyl, N-(C₁-C₆-Alkyl)-N-(C₁-C₆-alkylsulfonyl)-amino, N-(C₁-C₆-Alkyl)-N-(C₁-C₆-halogenalkylsulfonyl)-amino, Heterocyclyl-C₁-C₆-alkyl, Phenylcarbonyl-C₁-C₆-alkyl, Heterocyclylcarbonyl-C₁-C₆-alkyl: C₁-C₄-Alkyl, wie voranstehend genannt, sowie z.B. Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl oder 1-Ethyl-3-methylpropyl;
- C₁-C₄-Halogenalkyl: einen C₁-C₄-Alkylrest, wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl oder Nonafluorbutyl;
- C₁-C₆-Halogenalkyl: C₁-C₄-Halogenalkyl, wie voranstehend genannt, sowie z.B. 5-Fluorpentyl, 5-Chlorpentyl, 5-Brompentyl, 5-Iodpentyl, Undecafluorpentyl, 6-Fluorhexyl, 6-Chlorhexyl, 6-Bromhexyl, 6-Iodhexyl oder Dodecafluorhexyl;
- C₁-C₄-Alkoxy: z.B. Methoxy, Ethoxy, Propoxy, 1-Methylethoxy, Butoxy, 1-Methylpropoxy, 2-Methylpropoxy oder 1,1-Dimethylethoxy;
- C₁-C₆-Alkoxy, sowie die Alkoxyteile von Di-(C₁-C₆-alkoxy)-methyl, (C₁-C₆-Alkoxy) (C₁-C₆-alkylthio)methyl, C₁-C₆-Alkoxyimino-C₁-C₆-alkyl, N-(C₁-C₆-Alkoxy)-N-(C₁-C₆-alkyl)-aminocarbonyl, N-(C₃-C₆-Alkenyl)-N-(C₁-C₆-alkoxy)-amiziocarbonyl und N-(C₃-C₆-Alkinyl)-N-(C₁-C₆-alkoxy)-aminocarbonyl: C₁-C₄-Alkoxy, wie voranstehend genannt, sowie z.B. Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy oder 1-Ethyl-2-methylpropoxy;
- C₁-C₄-Halogenalkoxy: einen C₁-C₄-Alkoxyrest, wie voranstehend genannt, der partiell oder vollständig durch Fluor, Chlor, Brom und/oder Iod substituiert ist, also z.B. Fluormethoxy, Difluormethoxy, Trifluormethoxy, Chlordifluormethoxy, Bromdifluormethoxy, 2-Fluorethoxy, 2-Chlorethoxy, 2-Brommethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy oder Nonafluorbutoxy;
- C₁-C₆-Halogenalkoxy: C₁-C₄-Halogenalkoxy, wie voranstehend genannt, sowie z.B. 5-Fluorpentoxy, 5-Chlorpentoxy, 5-Brompentoxy, 5-Iodpentoxy, Undecafluorpentoxy, 6-Fluorhexoxy, 6-Chlorhexoxy, 6-Bromhexoxy, 6-Iodhexoxy oder Dodecafluorhexoxy;
- C₁-C₄-Alkylthio: z.B. Methylthio, Ethylthio, Propylthio, 1-Methylethylthio, Butylthio, 1-Methylpropylthio, 2-Methylpropylthio oder 1,1-Dimethylethylthio;
- C₁-C₆-Alkylthio, sowie die Alkylthioteile von (C₁-C₆-Alkoxy)(C₁-C₆-alkylthio)methyl, Di(C₁-C₆-alkylthio)methyl und C₁-C₆-Alkylthiocarbonyl: C₁-C₄-Alkylthio, wie voranstehend genannt, sowie z.B. Pentylthio, 1-Methylbutylthio, 2-Methylbutylthio, 3-Methylbutylthio, 2,2-Dimethylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio oder 1-Ethyl-2-methylpropylthio;
- C₁-C₄-Halogenalkylthio: einen C₁-C₄-Alkylthiorest, wie voranstehend genannt, der partiell oder vollständig durch Fluor, Chlor, Brom und/oder Iod substituiert ist, also z.B. Fluormethylthio, Difluormethylthio, Trifluormethylthio, Chlordifluormethylthio, Bromdifluormethylthio, 2-Fluorethylthio, 2-Chlorethylthio, 2-Bromethylthio, 2-Iodethylthio, 2,2-Difluorethylthio, 2,2,2-Trifluorethylthio, 2,2,2-Trichlorethylthio, 2-Chlor-2-fluorethylthio, 2-Chlor-2,2-difluorethylthio, 2,2-Dichlor-2-fluorethylthio, Pentafluorethylthio, 2-Fluorpropylthio, 3-Fluorpropylthio, 2-Chlorpropylthio, 3-Chlorpropylthio, 2-Brompropylthio, 3-Brompropylthio, 2,2-Difluorpropylthio, 2,3-Difluorpropylthio, 2,3-Dichlorpropylthio, 3,3,3-Trifluorpropylthio, 3,3,3-Trichlorpropylthio, 2,2,3,3,3-Pentafluorpropylthio, Heptafluorpropylthio, 1-(Fluormethyl)-2-fluorethylthio, 1-(Chlormethyl)-2-chlorethylthio, 1-(Brommethyl)-2-bromethylthio, 4-Fluorbutylthio, 4-Chlorbutylthio, 4-Brombutylthio oder Nonafluorbutylthio;
- C₁-C₆-Halogenalkylthio: C₁-C₄-Halogenalkylthioo wie voranstehend genannt, sowie z.B. 5-Fluorpentylthio, 5-Chlorpentylthio, 5-Brompentylthio, 5-Iodpentylthio, Undecafluorpentylthio, 6-Fluorhexylthio, 6-Chlorhexylthio, 6-Bromhexylthio, 6-Iodhexylthio oder Dodecafluorhexylthio;
- C₁-C₆-Alkylsulfinyl (C₁-C₆-Alkyl-S(=O)-): z.B. Methylsulfinyl, Ethylsulfinyl, Propylsulfinyl, 1-Methylethylsulfinyl, Butylsulfinyl, 1-Methylpropylsulfinyl, 2-Methylpropylsulfinyl, 1,1-Dimethylethylsulfinyl, Pentylsulfinyl, 1-Methylbutylsulfinyl, 2-Methylbutylsulfinyl, 3-Methylbutylsulfinyl, 2,2-Dimethylpropylsulfinyl, 1-Ethylpropylsulfinyl, 1,1-Dimethylpropylsulfinyl, 1,2-Dimethylpropylsulfinyl, Hexylsulfinyl, 1-Methylpentylsulfinyl, 2-Methylpentylsulfinyl, 3-Methylpentylsulfinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl oder 1-Ethyl-2-methylpropylsulfinyl;
- C₁-C₆-Halogenalkylsulfinyl: C₁-C₆-Alkylsulfinylrest, wie voranstehend genannt, der partiell oder vollständig durch Fluor, Chlor, Brom und/oder Iod substituiert ist, also z.B. Fluormethylsulfinyl, Difluormethylsulfinyl, Trifluormethylsulfinyl, Chlordifluormethylsulfinyl, Bromdifluormethylsulfinyl, 2-Fluorethylsulfinyl, 2-Chlorethylsulfinyl, 2-Bromethylsulfinyl, 2-Iodethylsulfinyl, 2,2-Difluorethylsulfinyl, 2,2,2-Trifluorethylsulfinyl, 2,2,2-Trichlorethylsulfinyl, 2-Chlor-2-fluorethylsulfinyl, 2-Chlor-2,2-difluorethylsulfinyl, 2,2-Dichlor-2-fluorethylsulfinyl, Pentafluorethylsulfinyl, 2-Fluorpropylsulfinyl, 3-Fluorpropylsulfinyl, 2-Chlorpropylsulfinyl, 3-Chlorpropylsulfinyl, 2-Brompropylsulfinyl, 3-Brompropylsulfinyl, 2,2-Difluorpropylsulfinyl, 2,3-Difluorpropylsulfinyl, 2,3-Dichlorpropylsulfinyl, 3,3,3-Trifluorpropylsulfinyl, 3,3,3-Trichlorpropylsulfinyl, 2,2,3,3,3-Pentafluorpropylsulfinyl, Heptafluorpropylsulfinyl, 1-(Fluormethyl)-2-fluorethylsulfinyl, 1-(Chlormethyl)-2-chlorethylsulfinyl, 1-(Brommethyl)-2-bromethylsulfinyl, 4-Fluorbutylsulfinyl, 4-Chlorbutylsulfinyl, 4-Brombutylsulfinyl, Nonafluorbutylsulfinyl, 5-Fluorpentylsulfinyl, 5-Chlorpentylsulfinyl, 5-Brompentylsulfinyl, 5-Iodpentylsulfinyl, Undecafluorpentylsulfinyl, 6-Fluorhexylsulfinyl, 6-Chlorhexylsulfinyl, 6-Bromhexylsulfinyl, 6-Iodhexylsulfinyl oder Dodecafluorhexylsulfinyl;
- C₁-C₆-Alkylsulfonyl (C₁-C₆-Alkyl-S(=O)₂-), sowie die Alkylsulfonylreste von N-(C₁-C₆-Alkylsulfonyl)-amino und N-(C₁-C₆-Alkyl)-N-(C₁-C₆-alkylsulfonyl)-amino: z.B. Methylsulfonyl, Ethylsulfonyl, Propylsulfonyl, 1-Methylethylsulfonyl, Butylsulfonyl, 1-Methylpropylsulfonyl, 2-Methylpropylsulfonyl, 1,1-Dimethylethylsulfonyl, Pentylsulfonyl, 1-Methylbutylsulfonyl, 2-Methylbutylsulfonyl, 3-Methylbutylsulfonyl, 1,1-Dimethylpropylsulfonyl, 1,2-Dimethylpropylsulfonyl, 2,2-Dimethylpropylsulfonyl, 1-Ethylpropylsulfonyl, Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl oder 1-Ethyl-2-methylpropylsulfonyl;
- C₁-C₆-Halogenalkylsulfonyl, sowie die Halogenalkylsulfonylreste von N-(C₁-C₆-Halogenalkylsulfonyl)-amino und N-(C₁-C₆-Alkyl)-N-(C₁-C₆-halogenalkylsulfonyl)-amino: einen C₁-C₆-Alkylsulfonylrest, wie voranstehend genannt, der partiell oder vollständig durch Fluor, Chlor, Brom und/oder Iod substituiert ist, also z.B. Fluormethylsulfonyl, Difluormethylsulfonyl, Trifluormethylsulfonyl, Chlordifluormethylsulfonyl, Bromdifluormethylsulfonyl, 2-Fluorethylsulfonyl, 2-Chlorethylsulfonyl, 2-Bromethylsulfonyl, 2-Iodethylsulfonyl, 2,2-Difluorethylsulfonyl, 2,2,2-Trifluorethylsulfonyl, 2-Chlor-2-fluorethylsulfonyl, 2-Chlor-2,2-difluorethylsulfonyl, 2,2-Dichlor-2-fluorethylsulfonyl, 2,2,2-Trichlorethylsulfonyl, Pentafluorethylsulfonyl, 2-Fluorpropylsulfonyl, 3-Fluorpropylsulfonyl, 2-Chlorpropylsulfonyl, 3-Chlorpropylsulfonyl, 2-Brompropylsulfonyl, 3-Brompropylsulfonyl, 2,2-Difluorpropylsulfonyl, 2,3-Difluorpropylsulfonvl, 2,3-Dichlorpropylsulfonyl, 3,3,3-Trifluorpropylsulfonyl, 3,3,3-Trichlorpropylsulfonyl, 2,2,3,3,3-Pentafluorpropylsulfonyl, Heptafluorpropylsulfonyl, 1-(Fluormethyl)-2-fluorethylsulfonyl, 1-(Chlormethyl)-2-chlorethylsulfonyl, 1-(Brommethyl)-2-bromethylsulfonyl, 4-Fluorbutylsulfonyl, 4-Chlorbutylsulfonyl, 4-Brombutylsulfonyl, Nonafluorbutylsulfonyl, 5-Fluorpentylsulfonyl, 5-Chlorpentylsulfonyl, 5-Brompentylsulfonyl, 5-Iodpentylsulfonyl, 6-Fluorhexylsulfonyl, 6-Bromhexylsulfonyl, 6-Iodhexylsulfonyl oder Dodecafluorhexylsulfonyl;
- C₁-C₆-Alkylamino: z.B. Methylamino, Ethylamino, Propylamino, 1-Methylethylamino, Butylamino, 1-Methylpropylamino, 2-Methylpropylamino, 1,1-Dimethylethylamino, Pentylamino, 1-Methylbutylamino, 2-Methylbutylamino, 3-Methylbutylamino, 2,2-Dimethylpropylamino, 1-Ethylpropylamino, Hexylamino, 1,1-Dimethylpropylamino, 1,2-Dimethylpropylamino, 1-Methylpentylamino, 2-Methylpentylamino, 3-Methylpentylamino, 4-Methylpentylamino, 1,1-Dimethylbutylamino, 1,2-Dimethylbutylamino, 1,3-Dimethylbutylamino, 2,2-Dimethylbutylamino, 2,3-Dimethylbutylamino, 3,3-Dimethylbutylamino, 1-Ethylbutylamino, 2-Ethylbutylamino, 1,1,2-Trimethylpropylamino, 1,2,2-Trimethylpropylamino, 1-Ethyl-1-methylpropylamino oder 1-Ethyl-2-methylpropylamino;
- (C₁-C₆-Alkylamino)sulfonyl: z.B. Methylaminosulfonyl, Ethylaminosulfonyl, Propylaminosulfonyl, 1-Methylethylaminosulfonyl, Butylaminosulfonyl, 1-Methylpropylaminosulfonyl, 2-Methylpropylaminosulfonyl, 1,1-Dimethylethylaminosulfonyl, Pentylaminosulfonyl, 1-Methylbutylaminosulfonyl, 2-Methylbutylaminosulfonyl, 3-Methylbutylaminosulfonyl, 2,2-Dimethylpropylaminosulfonyl, 1-Ethylpropylaminosulfonyl, Hexylaminosulfonyl, 1,1-Dimethylpropylaminosulfonyl, 1,2-Dimethylpropylaminosulfonyl, 1-Methylpentylaminosulfonyl, 2-Methylpentylaminosulfonyl, 3-Methylpentylaminosulfonyl, 4-Methylpentylaminosulfonyl, 1,1-Dimethylbutylaminosulfonyl, 1,2-Dimethylbutylaminosulfonyl, 1,3-Dimethylbutylaminosulfonyl, 2,2-Dimethylbutylaminosulfonyl, 2,3-Dimethylbutylaminosulfonyl, 3,3-Dimethylbutylaminosulfonyl, 1-Ethylbutylaminosulfonyl, 2-Ethylbutylaminosulfonyl, 1,1,2-Trimethylpropylaminosulfonyl, 1,2,2-Trimethylpropylaminosulfonyl, 1-Ethyl-1-methylpropylaminosulfonyl oder 1-Ethyl-2-methylpropylaminosulfonyl;
- Di-(C₁-C₆-alkyl)-aminosulfonyl: z.B. N,N-Dimethylaminosulfonyl, N,N-Diethylaminosulfonyl, N,N-Di-(1-methylethyl)aminosulfonyl, N,N-Dipropylaminosulfonyl, N,N-Dibutylaminosulfonyl, N,N-Di-(1-methylpropyl)-aminosulfonyl, N,N-Di-(2-methylpropyl)-aminosulfonyl, N,N-Di-(1,1-dimethylethyl)-aminosulfonyl, N-Ethyl-N-methylaminosulfonyl, N-Methyl-N-propylaminosulfonyl, N-Methyl-N-(1-methylethyl)-aminosulfonyl, N-Butyl-N-methylaminosulfonyl, N-Methyl-N-(1-methylpropyl)-aminosulfonyl, N-Methyl-N-(2-methylpropyl)-aminosulfonyl, N-(1,1-Dimethylethyl)-N-methylaminosulfonyl, N-Ethyl-N-propylaminosulfonyl, N-Ethyl-N-(1-methylethyl)-aminosulfonyl, N-Butyl-N-ethylaminosulfonyl, N-Ethyl-N-(1-methylpropyl)-aminosulfonyl, N-Ethyl-N-(2-methylpropyl)-aminosulfonyl, N-Ethyl-N-(1,1-dimethylethyl)-aminosulfonyl, N-(1-Methylethyl)-N-propylaminosulfonyl, N-Butyl-N-propylaminosulfonyl, N-(1-Methylpropyl)-N-propylaminosulfonyl, N-(2-Methylpropyl)-N-propylaminosulfonyl, N-(1,1-Dimethylethyl)-N-propylaminosulfonyl, N-Butyl-N-(1-methylethyl)-aminosulfonyl, N-(1-Methylethyl)-N-(1-methylpropyl)-aminosulfonyl, N-(1-Methylethyl)-N-(2-methylpropyl)-aminosulfonyl, N-(1,1-Dimethylethyl)-N-(1-methylethyl)-aminosulfonyl, N-Butyl-N-(1-methylpropyl)-aminosulfonyl, N-Butyl-N-(2-methylpropyl)-aminosulfonyl, N-Butyl-N-(1,1-dimethylethyl)-aminosulfonyl, N-(1-Methylpropyl)-N-(2-methylpropyl)-aminosulfonyl, N-(1,1-Dimethylethyl)-N-(1-methylpropyl)-aminosulfonyl, N-(1,1-Dimethylethyl)-N-(2-methylpropyl)-aminosulfonyl, N-Methyl-N-pentylaminosulfonyl, N-Methyl-N-(1-methylbutyl)-aminosulfonyl, N-Methyl-N-(2-methylbutyl)-aminosulfonyl, N-Methyl-N-(3-methylbutyl)-aminosulfonyl, N-Methyl-N-(2,2-dimethylpropyl)-aminosulfonyl, N-Methyl-N-(1-ethylpropyl)-aminosulfonyl, N-Methyl-N-hexylaminosulfonyl, N-Methyl-N-(1,1-dimethylpropyl)-aminosulfonyl, N-Methyl-N-(1,2-dimethylpropyl)-aminosulfonyl, N-Methyl-N-(1-methylpentyl)-aminosulfonyl, N-Methyl-N-(2-methylpentyl)-aminosulfonyl, N-Methyl-N-(3-methylpentyl)-aminosulfonyl, N-Methyl-N-(4-methylpentyl)-aminosulfonyl, N-Methyl-N-(1,1-dimethylbutyl)-aminosulfonyl, N-Methyl-N-(1,2-dimethylbutyl)-aminosulfonyl, N-Methyl-N-(1,3-dimethylbutyl)-aminosulfonyl, N-Methyl-N-(2,2-dimethylbutyl)-aminosulfonyl, N-Methyl-N-(2,3-dimethylbutyl)-aminosulfonyl, N-Methyl-N-(3,3-dimethylbutyl)-aminosulfonyl, N-Methyl-N-(1-ethylbutyl)-aminosulfonyl, N-Methyl-N-(2-ethylbutyl)-aminosulfonyl, N-Methyl-N-(1,1,2-trimethylpropyl)-aminosulfonyl, N-Methyl-N-(1,2,2-trimethylpropyl)-aminosulfonyl, N-Methyl-N-(1-ethyl-1-methylpropyl)-aminosulfonyl, N-Methyl-N-(1-ethyl-2-methylpropyl)-aminosulfonyl, N-Ethyl-N-pentylaminosulfonyl, N-Ethyl-N-(1-methylbutyl)-aminosulfonyl, N-Ethyl-N-(2-methylbutyl)-aminosulfonyl, N-Ethyl-N-(3-methylbutyl)-aminosulfonyl, N-Ethyl-N-(2,2-dimethylpropyl)-aminosulfonyl, N-Ethyl-N-(1-ethylpropyl)-aminosulfonyl, N-Ethyl-N-hexylaminosulfonyl, N-Ethyl-N-(1,1-dimethylpropyl)-aminosulfonyl, N-Ethyl-N-(1,2-dimethylpropyl)-aminosulfonyl, N-Ethyl-N-(2-methylpentyl)-aminosulfonyl, N-Ethyl-N-(2-methylpentyl)-aminosulfonyl, N-Ethyl-N-(3-methylpentyl)-aminosulfonyl, N-Ethyl-N-(4-methylpentyl)-aminosulfonyl, N-Ethyl-N-(1,1-dimethylbutyl)-aminosulfonyl, N-Ethyl-N-(1,2-dimethylbutyl)-aminosulfonyl, N-Ethyl-N-(1,3-dimethylbutyl)-aminosulfonyl, N-Ethyl-N-(2,2-dimethylbutyl)-aminosulfonyl, N-Ethyl-N-(2,3-dimethylbutyl)-aminosulfonyl, N-Ethyl-N-(3,3-dimethylbutyl)-aminosulfonyl, N-Ethyl-N-(1-ethylbutyl)-aminosulfonyl, N-Ethyl-N-(2-ethylbutyl)-aminosulfonyl, N-Ethyl-N-(1,1,2-trimethylpropyl)-aminosulfonyl, N-Ethyl-N-(1,2,2-trimethylpropyl)-aminosulfonyl, N-Ethyl-N-(1-ethyl-1-methylpropyl)-aminosulfonyl, N-Ethyl-N-(1-ethyl-2-methylpropyl)-aminosulfonyl, N-Propyl-N-pentylaminosulfonyl, N-Butyl-N-pentylaminosulfonyl, N,N-Dipentylaminosulfonyl, N-Propyl-N-hexylaminosulfonyl, N-Butyl-N-hexylaminosulfonyl, N-Pentyl-N-hexylaminosulfonyl oder N,N-Dihexylaminosulfonyl;
- Di-(C₁-C₄-alkyl)amino: z.B. N,N-Dimethylamino, N,N-Diethylamino, N,N-Dipropylamino, N,N-Di-(1-methylethyl)-amino, N,N-Dibutylamino, N,N-Di-(1-methylpropyl)amino, N,N-Di-(2-methylpropyl)amino, N,N-Di-(1,1-dimethylethyl)-amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl)amino, N-(1,1-Dimethylethyl)-N-methylamino, N-Ethyl-N-propylamino, N-Ethyl-N-(1-methylethyl)amino, N-Butyl-N-ethylamino, N-Ethyl-N-(1-methylpropyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methylpropyl)amino, N-(1,1-Dimethylethyl)-N-(1-methylethyl)amino, N-Butyl-N-(1-methylpropyl)-amino, N-Butyl-N-(2-methylpropyl)amino, N-Butyl-N-(1,1-dimethylethyl)amino, N-(1-Methylpropyl)-N-(2-methylpropyl)-amino, N-(1,1-Dimethylethyl)-N-(1-methylpropyl)-amino oder N-(1,1-Dimethylethyl)-N-(2-methylpropyl)amino;
- Di-(C₁-C₆-alkyl)amino: Di-(C₁-C₄-alkyl)amino wie voranstehend genannt, sowie N,N-Dipentylamino, N,N-Dihexylamino, N-Methyl-N-pentylamino, N-Ethyl-N-pentylamino, N-Methyl-N-hexylamino oder N-Ethyl-N-hexylamino.
- C₁-C₄-Alkylcarbonyl: z.B. Methylcarbonyl, Ethylcarbonyl, Propylcarbonyl, 1-Methylethylcarbonyl, Butylcarbonyl, 1-Methylpropylcarbonyl, 2-Methylpropylcarbonyl oder 1,1-Dimethylethylcarbonyl;
- C₁-C₆-Alkylcarbonyl, sowie die Alkylcarbonylreste von C₁-C₆-Alkylcarbonylamino: C₁-C₄-Alkylcarbonyl, wie voranstehend genannt, sowie z.B. Pentylcarbonyl, 1-Methylbutylcarbonyl, 2-Methylbutylcarbonyl, 3-Methylbutylcarbonyl, 2,2-Dimethylpropylcarbonyl, 1-Ethylpropylcarbonyl, Hexylcarbonyl, 1,1-Dimethylpropylcarbonyl, 1,2-Dimethylpropylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2,-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl oder 1-Ethyl-2-methylpropylcarbonyl;
- C₁-C₂₀-Alkylcarbonyl: C₁-C₆-Alkylcarbonyl, wie voranstehend genannt, sowie Heptylcarbonyl, Octylcarbonyl, Pentadecylcarbonyl oder Heptadecylcarbonyl;
- C₁-C₄-Alkoxycarbonyl: z.B. Methoxycarbonyl, Ethoxycarbonyl, Propoxycarbonyl, 1-Methylethoxycarbonyl, Butoxycarbonyl, 1-Methylpropoxycarbonyl, 2-Methylpropoxycarbonyl oder 1,1-Dimethylethoxycarbonyl;
- (C₁-C₆-Alkoxy)carbonyl: (C₁-C₄-Alkoxy)carbonyl, wie vorstehend genannt, sowie z.B. Pentoxycarbonyl, 1-Methylbutoxycarbonyl, 2-Methylbutoxycarbonyl, 3-Methylbutoxycarbonyl, 2,2-Dimethylpropoxycarbonyl, 1-Ethylpropoxycarbonyl, Hexoxycarbonyl, 1,1-Dimethylpropoxycarbonyl, 1,2-Dimethylpropoxycarbonyl, 1-Methylpentoxycarbonyl, 2-Methylpentoxycarbonyl, 3-Methylpentoxycarbonyl, 4-Methylpentoxycarbonyl, 1,1-Dimethylbutoxycarbonyl, 1,2-Dimethylbutoxycarbonyl, 1,3-Dimethylbutoxycarbonyl, 2,2-Dimethylbutoxycarbonyl, 2,3-Dimethylbutoxycarbonyl, 3,3-Dimethylbutoxycarbonyl, 1-Ethylbutoxycarbonyl, 2-Ethylbucoxycarbonyl, 1,1,2-Trimethylpropoxycarbonyl, 1,2,2-Trimethylpropoxycarbonyl, 1-Ethyl-1-methyl-propoxycarbonyl oder 1-Ethyl-2-methyl-propoxycarbonyl;
- C₁-C₄-Halogenalkoxycarbonyl: ein C₁-C₄-Alkoxycarbonylrest, wie voranstehend genannt, der partiell oder vollständig durch Fluor, Chlor, Brom und/oder Iod substituiert ist, also z.B. Fluormethoxycarbonyl, Difluormethoxycarbonyl, Trifluormethoxycarbonyl, Chlordifluormethoxycarbonyl, Bromdifluormethoxycarbonyl, 2-Fluorethoxycarbonyl, 2-Chlorethoxycarbonyl, 2-Bromethoxycarbonyl, 2-Iodethoxycarbonyl, 2,2-Difluorethoxycarbonyl, 2,2,2-Trifluorethoxycarbonyl, 2-Chlor-2-fluorethoxycarbonyl, 2-Chlor-2,2-difluorethoxycarbonyl, 2,2-Dichlor-2-fluorethoxycarbonyl, 2,2,2-Trichlorethoxycarbonyl, Pentafluorethoxycarbonyl, 2-Fluorpropoxycarbonyl, 3-Fluorpropoxycarbonyl, 2-Chlorpropoxycarbonyl, 3-Chlorpropoxycarbonyl, 2-Brompropoxycarbonyl, 3-Brompropoxycarbonyl, 2,2-Difluorpropoxycarbonyl, 2,3-Difluorpropoxycarbonyl, 2,3-Dichlorpropoxycarbonyl, 3,3,3-Trifluorpropoxycarbonyl, 3,3,3-Trichlorpropoxycarbonyl, 2,2,3,3,3-Pentafluorpropoxycarbonyl, Heptafluorpropoxycarbonyl, 1-(Fluormethyl)-2-fluorethoxycarbonyl, 1-(Chlormethyl)-2-chlorethoxycarbonyl, 1-(Brommethyl)-2-bromethoxycarbonyl, 4-Fluorbutoxycarbonyl, 4-Chlorbutoxycarbonyl, 4-Brombutoxycarbonyl oder Nonafluorbutoxycarbonyl;
- C₁-C₆-Halogenalkoxycarbonyl: C₁-C₄-Halogenalkoxycarbanyl wie voranstehend genannt, sowie 5-Fluorpentoxycarbonyl, 5-Chlorpentoxycarbonyl, 5-Brompentoxycarbonyl, 5-Iodpentoxycarbonyl, 6-Fluorhexoxycarbonyl, 6-Bromhexoxycarbonyl, 6-Iodhexoxycarbonyl oder Dodecafluorhexoxycarbonyl;
- (C₁-C₄-Alkyl)carbonyloxy: Acetyloxy, Ethylcarbonyloxy, Propylcarbonyloxy, 1-Methylethylcarbonyloxy, Butylcarbonyloxy, 1-Methylpropylcarbonyloxy, 2-Methylpropylcarbonyloxy oder 1,1-Dimethylethylcarbonyloxy;
- (C₁-C₄-Alkylamino)carbonyl: z.B. Methylaminocarbonyl, Ethylaminocarbonyl, Propylaminocarbonyl, 1-Methylethylaminocarbonyl, Butylaminocarbonyl, 1-Methylpropylaminocarbonyl, 2-Methylpropylaminocarbonyl oder 1,1-Dimethylethylaminocarbonyl;
- (C₁-C₆-Alkylamino)carbonyl: (C₁-C₄-Alkylamino)carbonyl, wie vorstehend genannt, sowie z.B. Pentylaminocarbonyl, 1-Methylbutylaminocarbonyl, 2-Methylbutylaminocarbonyl, 3-Methylbutylaminocarbonyl, 2,2-Dimethylpropylaminocarbonyl, 1-Ethylpropylaminocarbonyl, Hexylaminocarbonyl, 1,1-Dimethylpropylaminocarbonyl, 1,2-Dimethylpropylaminocarbonyl, 1-Methylpentylaminocarbonyl, 2-Methylpentylaminocarbonyl, 3-Methylpentylaminocarbonyl, 4-Methylpentylaminocarbonyl, 1,1-Dimethylbutylaminocarbonyl, 1,2-Dimethylbutylaminocarbonyl, 1,3-Dimethylbutylaminocarbonyl, 2,2-Dimethylbutylaminocarbonyl, 2,3-Dimethylbutylaminocarbonyl, 3,3-Dimethylbutylaminocarbonyl, 1-Ethylbutylaminocarbonyl, 2-Ethylbutylaminocarbonyl, 1,1,2-Trimethylpropylaminocarbonyl, 1,2,2-Trimethylpropylaminocarbonyl, 1-Ethyl-1-methylpropylaminocarbonyl oder 1-Ethyl-2-methylpropylaminocarbonyl;
- Di-(C₁-C₄-alkyl)-aminocarbonyl: z.B. N,N-Dimethylaminocarbonyl, N,N-Diethylaminocarbonyl, N,N-Di-(1-methylethyl)aminocarbonyl, N,N-Dipropylaminocarbonyl, N,N-Dibutylaminocarbonyl, N,N-Di-(1-methylpropyl)-aminocarbonyl, N,N-Di-(2-methylpropyl)-aminocarbonyl, N,N-Di-(1,1-dimethylethyl)-aminocarbonyl, N-Ethyl-N-methylaminocarbonyl, N-Methyl-N-propylaminocarbonyl, N-Methyl-N-(1-methylethyl)-aminocarbonyl, N-Butyl-N-methylaminocarbonyl, N-Methyl-N-(1-methylpropyl)-aminocarbonyl, N-Methyl-N-(2-methylpropyl)-aminocarbonyl, N-(1,2-Dimethylethyl)-N-methylaminocarbonyl, N-Ethyl-N-propylaminocarbonyl, N-Ethyl-N-(1-methylethyl)-aminocarbonyl, N-Butyl-N-ethylaminocarbonyl, N-Ethyl-N-(1-methylpropyl)-aminocarbonyl, N-Ethyl-N-(2-methylpropyl)-aminocarbonyl, N-Ethyl-N-(1,1-dimethylethyl)-aminocarbonyl, N-(1-Methylethyl)-N-propylaminocarbonyl, N-Butyl-N-propylaminocarbonyl, N-(1-Methylpropyl)-N-propylaminocarbonyl, N-(2-Methylpropyl)-N-propylaminocarbonyl, N-(1,1-Dimethylethyl)-N-propylaminocarbonyl, N-Butyl-N-(1-methylethyl)-aminocarbonyl, N-(1-Methylethyl)-N-(1-methylpropyl)-aminocarbonyl, N-(1-Methylethyl)-N-(2-methylpropyl)-aminocarbonyl, N-(1,1-Dimethylethyl)-N-(1-methylethyl)-aminocarbonyl, N-Butyl-N-(1-methylpropyl)-aminocarbonyl, N-Butyl-N-(2-methylpropyl)-aminocarbonyl, N-Butyl-N-(1,1-dimethylethyl)-aminocarbonyl, N-(1-Methylpropyl)-N-(2-methylpropyl)-aminocarbonyl, N-(1,1-Dimethylethyl)-N-(1-methylpropyl)-aminocarbonyl oder N-(1,1-Dimethylethyl)-N-(2-methylpropyl)-aminocarbonyl;
- Di-(C₁-C₆-alkyl)-aminocarbonyl: Di-(C₁-C₄-alkyl)-aminocarbonyl, wie voranstehend genannt, sowie z.B. N-Methyl-N-pentylaminocarbonyl, N-Methyl-N-(1-methylbutyl)-aminocarbonyl, N-Methyl-N-(2-methylbutyl)-aminocarbonyl, N-Methyl-N-(3-methylbutyl)-aminocarbonyl, N-Methyl-N-(2,2-dimethylpropyl)-aminocarbonyl, N-Methyl-N-(1-ethylpropyl)-aminocarbonyl, N-Methyl-N-hexylaminocarbonyl, N-Methyl-N-(1,1-dimethylpropyl)-aminocarbonyl, N-Methyl-N-(1,2-dimethylpropyl)-aminocarbonyl, N-Methyl-N-(1-methylpentyl)-aminocarbonyl, N-Methyl-N-(2-methylpentyl)-aminocarbonyl, N-Methyl-N-(3-methylpentyl)-aminocarbonyl, N-Methyl-N-(4-methylpentyl)-aminocarbonyl, N-Methyl-N-(1,1-dimethylbutyl)-aminocarbonyl, N-Methyl-N-(1,2-dimethylbutyl)-aminocarbonyl, N-Methyl-N-(1,3-dimethylbutyl)-aminocarbonyl, N-Methyl-N-(2,2-dimethylbutyl)-aminocarbonyl, N-Methyl-N-(2,3-dimethylbutyl)-aminocarbonyl, N-Methyl-N-(3,3-dimethylbutyl)-aminocarbonyl, N-Methyl-N-(1-ethylbutyl)-aminocarbonyl, N-Methyl-N-(2-ethylbutyl)-aminocarbonyl, N-Methyl-N-(1,1,2-trimethylpropyl)-aminocarbonyl, N-Methyl-N-(1,2,2-trimethylpropyl)-aminocarbonyl, N-Methyl-N-(1-ethyl-1-methylpropyl)-aminocarbonyl, N-Methyl-N-(1-ethyl-2-methylpropyl)-aminocarbonyl, N-Ethyl-N-pentylaminocarbonyl, N-Ethyl-N-(1-methylbutyl)-aminocarbonyl, N-Ethyl-N-(2-methylbutyl)-aminocarbonyl, N-Ethyl-N-(3-methylbutyl)-aminocarbonyl, N-Ethyl-N-(2,2-dimethylpropyl)-aminocarbonyl, N-Ethyl-N-(1-ethylpropyl)-aminocarbonyl, N-Ethyl-N-hexylaminocarbonyl, N-Ethyl-N-(1,1-dimethylpropyl)-aminocarbonyl, N-Ethyl-N-(1,2-dimethylpropyl)-aminocarbonyl, N-Ethyl-N-(1-methylpentyl)-aminocarbonyl, N-Ethyl-N-(2-methylpentyl)-aminocarbonyl, N-Ethyl-N-(3-methylpentyl)-aminocarbonyl, N-Ethyl-N-(4-methylpentyl)-aminocarbonyl, N-Ethyl-N-(1,1-dimethylbutyl)-aminocarbonyl, N-Ethyl-N-(1,2-dimethylbutyl)-aminocarbonyl, N-Ethyl-N-(1,3-dimethylbutyl)-aminocarbonyl, N-Ethyl-N-(2,2-dimethylbutyl)-aminocarbonyl, N-Ethyl-N-(2,3-dimethylbutyl)-aminocarbonyl, N-Ethyl-N-(3,3-dimethylbutyl)-aminocarbonyl, N-Ethyl-N-(1-ethylbutyl)-aminocarbonyl, N-Ethyl-N-(2-ethylbutyl)-aminocarbonyl, N-Ethyl-N-(1,1,2-trimethylpropyl)-aminocarbonyl, N-Ethyl-N-(1,2,2-trimethylpropyl)-aminocarbonyl, N-Ethyl-N-(1-ethyl-1-methylpropyl)-aminocarbonyl, N-Ethyl-N-(1-ethyl-2-methylpropyl)-aminocarbonyl, N-Propyl-N-pentylaminocarbonyl, N-Butyl-N-pentylaminocarbonyl, N,N-Dipentylaminocarbonyl, N-Propyl-N-hexylaminocarbonyl, N-Butyl-N-hexylaminocarbonyl, N-Pentyl-N-hexylaminocarbonyl oder N,N-Dihexylaminocarbonyl;
- Di-(C₁-C₆-alkyl)-aminothiocarbonyl: z.B. N,N-Dimethylaminothiocarbonyl, N,N-Diethylaminothiocarbonyl, N,N-Di-(1-methylethyl)aminothiocarbonyl, N,N-Dipropylaminothiocarbonyl, N,N-Dibutylaminothiocarbonyl, N,N-Di-(1-methylpropyl)-aminothiocarbonyl, N,N-Di-(2-methylpropyl)-aminothiocarbonyl, N,N-Di-(1,1-dimethylethyl)-aminothiocarbonyl, N-Ethyl-N-methylaminothiocarbonyl, N-Methyl-N-propylaminothiocarbonyl, N-Methyl-N-(2-methylethyl)-aminothiocarbonyl, N-Butyl-N-methylaminothiocarbonyl, N-Methyl-N-(1-methylpropyl)-aminothiocarbonyl, N-Methyl-N-(2-methylpropyl)-aminothiocarbonyl, N-(1,1-Dimethyl-ethyl)-N-methylaminothiocarbonyl, N-Ethyl-N-propylaminothiocarbonyl, N-Ethyl-N-(1-methylethyl)-aminothiocarbonyl, N-Butyl-N-ethylaminothiocarbonyl, N-Ethyl-N-(1-methylpropyl)-aminothiocarbonyl, N-Ethyl-N-(2-methylpropyl)-aminothiocarbonx', N-Ethyl-N-(1,1-dimethylethyl)-aminothiocarbonyl, N-(1-Methylethyl)-N-propylaminothiocarbonyl, N-Butyl-N-propylaminothiocarbonyl, N-(1-Methylpropyl)-N-propylaminothiocarbonyl, N-(2-Methylpropyl)-N-propylaminothiocarbonyl, N-(1,1-Dimethylethyl)-N-propylaminothiocarbonyl, N-Butyl-N-(1-methylethyl)-aminothiocarbonyl, N-(1-Methylethyl)-N-(1-methylpropyl)-aminothiocarbonyl, N-(1-Methylethyl)-N-(2-methylpropyl)-aminothiocarbonyl, N-(1,1-Dimethylethyl)-N-(1-methylethyl)-aminothiocarbonyl, N-Butyl-N-(1-methylpropyl)-aminothiocarbonyl, N-Butyl-N-(2-methylpropyl)-aminothiocarbonyl, N-Butyl-N-(1,1-dimethylethyl)-aminothiocarbonyl, N-(1-Methylpropyl)-N-(2-methylpropyl)-ciminothiocarbonyl, N-(1,1-Dimethylethyl)-N-(1-methylpropyl)-aminothiocarbonyl, N-(1,1-Dimethylethyl)-N-(2-methylpropyl)-aminothiocarbonyl, N-Methyl-N-pentylaminothiocarbonyl, N-Methyl-N-(1-methylbutyl)-aminothiocarbonyl, N-Methyl-N-(2-methylbutyl)-aminothiocarbonyl, N-Methyl-N-(3-methylbutyl)-aminothiocarbonyl, N-Methyl-N-(2,2-dimethylpzopyl)-aminothiocarbonyl, N-Methyl-N-(1-ethylpropyl)-aminothiocarbonyl, N-Methyl-N-hexylaminothiocarbonyl, N-Methyl-N-(1,1-dimethylpropyl)-aminothiocarbonyl, N-Methyl-N-(1,2-dimethylpropyl)-aminothiocarbonyl, N-Methyl-N-(1-methylpentyl)-aminothiocarbonyl, N-Methyl-N-(2-methylpentyl)-aminothiocarbonyl, N-Methyl-N-(3-methylpentyl)-aminothiocarbonyl, N-Methyl-N-(4-methylpentyl)-aminothiocarbonyl, N-Methyl-N-(1,1-dimethylbutyl)-aminothiocarbonyl, N-Methyl-N-(1,2-dimethylbutyl)-aminothiocarbonyl, N-Methyl-N-(1,3-dimethylbutyl)-aminothiocarbonyl, N-Methyl-N-(2,2-dimethylbutyl)-aminothiocarbonyl, N-Methyl-N-(2,3-dimethylbutyl)-aminothiocarbonyl, N-Methyl-N-(3,3-dimethylbutyl)-aminothiocarbonyl, N-Methyl-N-(1-ethylbutyl)-aminothiocarbonyl, N-Methyl-N-(2-ethylbutyl)-aminothiocarbonyl, N-Methyl-N-ethyl-N-(1,1,2-trimethylpropyl)-aminothiocarbonyl, N-Methyl-N-(1,2,2-trimethylpropyl)-aminothiocarbonyl, N-Methyl-N-(1-ethyl-1-methylpropyl)-aminothiocarbonyl, N-Methyl-N-(1-ethyl-2-methylpropyl)-aminothiocarbonyl, N-Ethyl-N-pentylaminothiocarbonyl, N-Ethyl-N-(1-methylbutyl)-aminothiocarbonyl, N-Ethyl-N-(2-methylbutyl)-aminothiocarbonyl, N-Ethyl-N-(3-methylbutyl)-aminothiocarbonyl, N-Ethyl-N-(2,2-dimethylpropyl)-aminothiocarbonyl, N-Ethyl-N-(1-ethylpropyl)-aminothiocarbonyl, N-Ethyl-N-hexylaminothiocarbonyl, N-Ethyl-N-(1,1-dimethylpropyl)-aminothiocarbonyl, N-Ethyl-N-(1,2-dimethylpropyl)-aminothiocarbonyl, N-Ethyl-N-(1-methylpentyl)-aminothiocarbonyl, N-Ethyl-N-(2-methylpentyl)-aminothiocarbonyl, N-Ethyl-N-(3-methylpentyl)-aminothiocarbonyl, N-Ethyl-N-(4-methylpentyl)-aminothiocarbonyl, N-Ethyl-N-(1,1-dimethylbutyl)-aminothiocarbonyl, N-Ethyl-N-(1,2-dimethylbutyl)-aminothiocarbonyl, N-Ethyl-N-(1,3-dimethylbutyl)-aminothiocarbonyl, N-Ethyl-N-(2,2-dimethylbutyl)-aminothiocarbonyl, N-Ethyl-N-(2,3-dimethylbutyl)-aminothiocarbonyl, N-Ethyl-N-(3,3-dimethylbutyl)-aminothiocarbonyl, N-Ethyl-N-(1-ethylbutyl)-aminothiocarbonyl, N-Ethyl-N-(2-ethylbutyl)-aminothiocarbonyl, N-Ethyl-N-(1,1,2-trimethylpropyl)-aminothiocarbonyl, N-Ethyl-N-(1,2,2-trimethylpropyl)-aminothiocarbonyl, N-Ethyl-N-(1-ethyl-1-methylpropyl)-aminothiocarbonyl, N-Ethyl-N-(1-ethyl-2-methylpropyl)-aminothiocarbonyl, N-Propyl-N-pentylaminothiocarbonyl, N-Butyl-N-pentylaminothiocarbonyl, N,N-Dipentylaminothiocarbonyl, N-Propyl-N-hexylaminothiocarbonyl, N-Butyl-N-hexylaminothiocarbonyl, N-Pentyl-N-hexylaminothiocarbonyl oder N,N-Dihexylaminothiocarbonyl;
- C₁-C₄-Alkoxy-C₁-C₄-alkyl sowie die Alkoxyalkylteile von Hydroxy-C₁-C₄-alkoxy-C₁-C₄-alkyl: durch C₁-C₄-Alkoxy, wie vorstehend genannt, substituiertes C₁-C₄-Alkyl, also z.B. für Methoxymethyl, Ethoxymethyl, Propoxymethyl, (1-Methylethoxy)methyl, Butoxymethyl, (1-Methylpropoxy)methyl, (2-Methylpropoxy)-methyl, (1,1-Dimethylethoxy)methyl, 2-(Methoxy)ethyl, 2-(Ethoxy)ethyl, 2-(Propoxy)ethyl, 2-(1-Methylethoxy)ethyl, 2-(Butoxy)ethyl, 2-(1-Methylpropoxy)ethyl, 2-(2-Methylpropoxy)ethyl, 2-(1,1-Dimethylethoxy)ethyl, 2-(Methoxy)-propyl, 2-(Ethoxy)propyl, 2-(Propoxy)propyl, 2-(1-Methylethoxy)-propyl, 2-(Butoxy)propyl, 2-(1-Methylpropoxy)propyl, 2-(2-Methylpropoxy)propyl, 2-(1,1-Dimethylethoxy)propyl, 3-(Methoxy)propyl, 3-(Ethoxy)-propyl, 3-(Propoxy)propyl, 3-(1-Methylethoxy)propyl, 3-(Butoxy)propyl, 3-(1-Methylpropoxy)propyl, 3-(2-Methylpropoxy)propyl, 3-(1,1-Dimethylethoxy)propyl, 2-(Methoxy)butyl, 2-(Ethoxy)butyl, 2-(Propoxy)butyl, 2-(1-Methylethoxy)butyl, 2-(Butoxy)butyl, 2-(1-Methylpropoxy)butyl, 2-(2-Methylpropoxy)butyl, 2-(1,1-Dimethylethoxy)butyl, 3-(Methoxy)butyl, 3-(Ethoxy)butyl, 3-(Propoxy)butyl, 3-(1-Methylethoxy)butyl, 3-(Butoxy)-butyl, 3-(1-Methylpropoxy)butyl, 3-(2-Methylpropoxy)butyl, 3-(1,1-Dimethylethoxy)butyl, 4-(Methoxy)butyl, 4-(Ethoxy)-butyl, 4-(Propoxy)butyl, 4-(1-Methylethoxy)butyl, 4-(Butoxy)-butyl, 4-(1-Methylpropoxy)butyl, 4-(2-Methylpropoxy)butyl oder 4-(1,1-Dimethylethoxy)butyl;
- C₁-C₄-Alkoxy-C₁-C₄-alkoxy als Alkoxyalkoxyteile von C₁-C₄-Alkoxy-C₁-C₄-alkoxycarbonyl: durch C₁-C₄-Alkoxy, wie vorstehend genannt, substituiertes C₁-C₄-Alkoxy, also z.B. für Methoxymethoxy, Ethoxymethoxy, Propoxymethoxy, (1-Methylethoxy)methoxy, Butoxymethoxy, (1-Methylpropoxy)methoxy, (2-Methylpropoxy)methoxy, (1,1-Dimethylethoxy)methoxy, 2-(Methoxy)ethoxy, 2-(Ethoxy)ethoxy, 2-(Propoxy)ethoxy, 2-(1-Methylethoxy)ethoxy, 2-(Butoxy)ethoxy, 2-(1-Methylpropoxy)ethoxy, 2-(2-Methylpropoxy)ethoxy, 2-(1,1-Dimethylethoxy)ethoxy, 2-(Methoxy)propoxy, 2-(Ethoxy)propoxy, 2-(Propoxy)propoxy, 2-(1-Methylethoxy)propoxy, 2-(Butoxy)-propoxy, 2-(1-Methylpropoxy)propoxy, 2-(2-Methylpropoxy)propoxy, 2-(1,1-Dimethylethoxy)propoxy, 3-(Methoxy)-propoxy, 3-(Ethoxy)propoxy, 3-(Propoxy)propoxy, 3-(1-Methylethoxy)propoxy, 3-(Butoxy)propoxy, 3-(1-Methylpropoxy)-propoxy, 3-(2-Methylpropoxy)propoxy, 3-(1,1-Dimethylethoxy)propoxy, 2-(Methoxy)butoxy, 2-(Ethoxy)butoxy, 2-(Propoxy)butoxy, 2-(1-Methylethoxy)butoxy, 2-(Butoxy)-butoxy, 2-(1-Methylpropoxy)butoxy, 2-(2-Methylpropoxy)butoxy, 2-(1,1-Dimethylethoxy)butoxy, 3-(Methoxy)butoxy, 3-(Ethoxy)-butoxy, 3-(Propoxy)butoxy, 3-(1-Methylethoxy)butoxy, 3-(Butoxy)butoxy, 3-(1-Methylpropoxy)butoxy, 3-(2-Methylpropoxy)butoxy, 3-(1,1-Dimethylethoxy)butoxy, 4-(Methoxy)-butoxy, 4-(Ethoxy)butoxy, 4-(Propoxy)butoxy, 4-(1-Methylethoxy)butoxy, 4-(Butoxy)butoxy, 4-(1-Methylpropoxy)butoxy, 4-(2-Methylpropoxy)butoxy oder 4-(1,1-Dimethylethoxy)butoxy;
- C₃-C₆-Alkenyl, sowie die Alkenylteile von C₃-C₆-Alkenylcarbonyl, C₃-C₆-Alkenyloxy, C₃-C₆-Alkenyloxycarbonyl, C₃-C₆-Alkenylaminocarbonyl, N-(C₃-C₆-Alkenyl)-N-(C₁-C₆)alkylaminocarbonyl und N-(C₃-C₆-Alkenyl)-N-(C₁-C₆-alkoxy)aminocarbonyl: z.B. Prop-2-en-1-yl, But-1-en-4-yl, 1-Methylprop-2-en-1-yl, 2-Methyl-prop-2-en-1-yl, 2-Buten-1-yl, 1-Penten-3-yl, 1-Penten-4-yl, 2-Penten-4-yl, 1-Methylbut-2-en-1-yl, 2-Methyl-but-2-en-1-yl, 3-Methylbut-2-en-1-yl, 1-Methyl-but-3-en-1-yl, 2-Methylbut-3-en-1-yl, 3-Methyl-but-3-en-1-yl, 1,1-Dimethylprop-2-en-1-yl, 1,2-Dimethyl-prop-2-en-1-yl, 1-Ethylprop-2-en-1-yl, Hex-3-en-1-yl, Hex-4-en-1-yl, Hex-5-en-1-yl, 1-Methyl-pent-3-en-1-yl, 2-Methyl-pent-3-en-1-yl, 3-Methylpent-3-en-1-yl, 4-Methyl-pent-3-en-1-yl, 1-Methylpent-4-en-1-yl, 2-Methyl-pent-4-en-1-yl, 3-Methylpent-4-en-1-yl, 4-Methyl-pent-4-en-1-yl, 1,1-Dimethylbut-2-en-1-yl, 1,1-Dimethyl-but-3-en-1-yl, 1,2-Dimethylbut-2-en-1-yl, 1,2-Dimethyl-but-3-en-1-yl, 1,3-Dimethylbut-2-en-1-yl, 1,3-Dimethyl-but-3-en-1-yl, 2,2-Dimethylbut-3-en-1-yl, 2,3-Dimethyl-but-2-en-1-yl, 2,3-Dimethylbut-3-en-1-yl, 3,3-Dimethyl-but-2-en-1-yl, 1-Ethyl-but-2-en-1-yl, 1-Ethyl-but-3-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl oder 1-Ethyl-2-methylprop-2-en-1-yl;
- C₂-C₆-Alkenyl, sowie die Alkenylteile von C₂-C₆-Alkenylcarbonyl, Phenyl-C₂-C₆-alkenylcarbonyl und Heterocyclyl-C₂-C₆-alkenylcarbonyl: C₃-C₆-Alkenyl, wie voranstehend genannt, sowie Ethenyl;
- C₃-C₆-Halogenalkenyl: einen C₃-C₆-Alkenylrest, wie vorstehend genannt, der partiell oder vollständig durch Fluor, Chlor, Brom und/oder Iod substituiert ist, also z.B. 2-Chlorallyl, 3-Chlorallyl, 2,3-Dichlorallyl, 3,3-Dichlorallyl, 2,3,3-Trichlorallyl, 2,3-Dichlorbut-2-enyl, 2-Bromallyl, 3-Bromallyl, 2,3-Dibromallyl, 3,3-Dibromallyl, 2,3,3-Tribromallyl oder 2,3-Dibrombut-2-enyl;
- C₃-C₆-Alkinyl, sowie die Alkinylteile von C₃-C₆-Alkinylcarbonyl, C₃-C₆-Alkinyloxy, C₃-C₆-Alkinyloxycarbony, C₃-C₆-Alkinylaminocarbonyl, N-(C₃-C₆-Alkinyl)-N-(C₁-C₆alkyl)-aminocarbonyl und N-(C₃-C₆-Alkinyl)-N-(C₁-C₆-alkoxyaminocarbonyl: z.B. Propargyl, But-1-in-3-yl, But-1-in-4-yl, But-2-in-1-yl, Pent-1-in-3-yl, Pent-1-in-4-yl, Pent-1-in-5-yl, Pent-2-in-1-yl, Pent-2-in-4-yl, Pent-2-in-5-yl, 3-Methyl-but-1-in-3-yl, 3-Methyl-but-1-in-4-yl, Hex-1-in-3-yl, Hex-1-in-4-yl, Hex-1-in-5-yl, Hex-1-in-6-yl, Hex-2-in-1-yl, Hex-2-in-4-yl, Hex-2-in-5-yl, Hex-2-in-6-yl, Hex-3-in-1-yl, Hex-3-in-2-yl, 3-Methyl-pent-1-in-3-yl, 3-Methyl-pent-1-in-4-yl, 3-Methyl-pent-1-in-5-yl, 4-Methyl-pent-2-in-4-yl oder 4-Methyl-pent-2-in-5-yl;
- C₂-C₆-Alkinyl, sowie die Alkinylteile von C₂-C₆-Alkinylcarbonyl: C₃-C₆-Alkinyl, wie voranstehend genannt, sowie Ethinyl;
- C₃-C₆-Halogenalkinyl: einen C₃-C₆-Alkinylrest, wie vorstehend genannt, der partiell oder vollständig durch Fluor, Chlor, Brom und/oder Iod substituiert ist, also z.B. 1,1-Difluorprop-2-in-1-yl, 3-Iod-prop-2-in-1-yl, 4-Fluorbut-2-in-1-yl, 4-Chlorbut-2-in-1-yl, 1,1-Difluorbut-2-in-1-yl, 4-Iodbut-3-in-1-yl, 5-Fluorpent-3-in-1-yl, 5-Iod-pent-4-in-1-yl, 6-Fluor-hex-4-in-1-yl oder 6-Iod-hex-5-in-1-yl;
- C₃-C₆-Cycloalkyl, sowie die Cycloalkylteile von C₃-C₆-Cycloalkylcarbonyl: z.B. Cyclopropyl, Cyclobutyl, Cyclopentyl oder Cyclohexyl;
- Heterocyclyl, sowie Heterocyclylteile von Heterocyclylcarbonyl, Heterocyclyl-C₁-C₆-alkyl, Heterocyclyloxycarbonyl, Heterocyclyloxythiocarbonyl, Heterocyclyl-C₂-C₆alkenylcarbonyl, Heterocyclylcarbonyl-C₁-C₆-alkyl, N-(C₁-C₆-Alkyl)-N-(heterocyclyl)-aminocarbonyl, Heterocyclylaminocarbonyl: ein gesättigter, partiell gesättigter oder ungesättigter 5- oder 6-gliedriger, C-gebundener, heterocyclischer Ring, der ein bis vier gleiche oder verschiedene Heteroatome, ausgewählt aus folgender Gruppe: Sauerstoff, Schwefel oder Stickstoff, enthält, also z.B. 5-gliedrige Ringe mit beispielsweise einem Heteroatom, mit zwei Heteroatomen, mit drei Heteroatomen oder mit vier Heteroatomen oder z.B. 6-gliedrige Ringe mit beispielsweise einem Heteroatom, mit zwei Heteroatomen, mit drei Heteroatomen oder mit vier Heteroatomen, also 5-gliedrige Ringe: mit einem Heteroatom wie:
   Tetrahydrofuran-2-yl, Tetrahydrofuran-3-yl, Tetrahydrothien-2-yl, Tetrahydrothien-3-yl,Tetrahydropyrrol-2-yl, Tetrahydropyrrol-3-yl, 2,3-Dihydrofuran-2-yl, 2,3-Dihydrofuran-3-yl, 2,5-Dihydrofuran-2-yl, 2,5-Dihydrofuran-3-yl, 4,5-Dihydrofuran-2-yl, 4,5-Dihydrofuran-3-yl, 2,3-Dihydrothien-2-yl, 2,3-Dihydrothien-3-yl, 2,5-Dihydrothien-2-yl, 2,5-Dihydrothien-3-yl, 4,5-Dihydrothien-2-yl, 4,5-Dihydrothien-3-yl, 2,3-Dihydro-1H-pyrrol-2-yl, 2,3-Dihydro-1H-pyrrol-3-yl, 2,5-Dihydro-1H-pyrrol-2-yl, 2,5-Dihydro-1H-pyrrol-3-yl, 4,5-Dihydro-1H-pyrrol-2-yl, 4,5-Dihydro-1H-pyrrol-3-yl, 3,4-Dihydro-2H-pyrrol-2-yl, 3,4-Dihydro-2H-pyrrol-3-yl, 3,4-Dihydro-5H-pyrrol-2-yl, 3,4-Dihydro-5H-pyrrol-3-yl, 2-Furyl, 3-Furyl, 2-Thienyl, 3-Thienyl, Pyrrol-2-yl oder Pyrrol-3-yl;
   5-gliedrige Ringe mit zwei Heteroatomen wie:
      Tetrahydropyrazol-3-yl, Tetrahydropyrazol-4-yl, Tetrahydroisoxazol-3-yl, Tetrahydroisoxazol-4-yl, Tetrahydroisoxazol-5-yl, 1,2-Oxathiolan-3-yl, 1,2-Oxathiolan-4-yl, 1,2-Oxathiolan-5-yl, Tetrahydroisothiazol-3-yl, Tetrahydroisothiazol-4-yl, Tetrahydroisothiazol-5-yl, 1,2-Dithiolan-3-yl, 1,2-Dithiolan-4-yl, Tetrahydroimidazol-2-yl, Tetrahydroimidazol-4-yl, Tetrahydrooxazol-2-yl, Tetrahydrooxazol-4-yl,
      Tetrahydrooxazol-5-yl, Tetrahydrothiazol-2-yl, Tetrahydrothiazol-4-yl, Tetrahydrothiazol-5-yl, 1,3-Dioxolan-2-yl, 1,3-Dioxolan-4-yl, 1,3-Oxathiolan-2-yl, 1,3-Oxathiolan-4-yl, 1,3-Oxathioian-5-yl, 1,3-Dithiolan-2-yl, 1,3-Dithiolan-4-yl, 4,5-Dihydro-1H-pyrazol-3-yl, 4,5-Dihydro-1H-pyrazol-4-yl, 4,5-Dihydro-1H-pyrazol-5-yl, 2,5-Dihydro-1H-pyrazol-3-yl, 2,5-Dihydro-1H-pyrazol-4-yl, 2,5-Dihydro-1H-pyrazol-5-yl, 4,5-Dihydroisoxazol-3-yl, 4,5-Dihydroisoxazol-4-yl, 4,5-Dihydroisoxazol-5-yl, 2,5-Dihydroisoxazol-3-yl, 2,5-Dihydroisoxazol-4-yl, 2,5-Dihydroisoxazol-5-yl, 2,3-Dihydroisoxazol-3-yl, 2,3-Dihydroisoxazol-4-yl, 2,3-Dihydroisoxazol-5-yl, 4,5-Dihydroisothiazol-3-yl, 4,5-Dihydroisothiazol-4-yl, 4,5-Dihydroisothiazol-5-yl, 2,5-Dihydroisothiazol-3-yl, 2,5-Dihydroisothiazol-4-yl, 2,5-Dihydroisothiazol-5-yl, 2,3-Dihydroisothiazol-3-yl, 2,3-Dihydroisothiazol-4-yl, 2,3-Dihydroisothiazol-5-yl, Δ³-1,2-Dithiol-3-yl, Δ³-1,2-Dithiol-4-yl, Δ³-1,2-Dithiol-5-yl, 4,5-Dihydro-1H-imidazol-2-yl, 4,5-Dihydro-1H-imidazol-4-yl, 4,5-Dihydro-1H-imidazol-5-yl, 2,5-Dihydro-1H-imidazol-2-yl, 2,5-Dihydro-1H-imidazol-4-yl, 2,5-Dihydro-1H-imidazol-5-yl, 2,3-Dihydro-1H-imidazol-2-yl, 2,3-Dihydro-1H-imidazol-4-yl, 4,5-Dihydrooxazol-2-yl, 4,5-Dihydrooxazol-4-yl, 4,5-Dihydrooxazol-5-yl, 2,5-Dihydrooxazol-2-yl, 2,5-Dihydrooxazol-4-yl, 2,5-Dihydrooxazol-5-yl, 2,3-Dihydrooxazol-2-yl, 2,3-Dihydrooxazol-4-yl, 2,3-Dihydrooxazol-5-yl, 4,5-Dihydrothiazol-2-yl, 4,5-Dihydrothiazol-4-yl, 4,5-Dihydrothiazol-5-yl, 2,5-Dihydrothiazol-2-yl, 2,5-Dihydrothiazol-4-yl, 2,5-Dihydrothiazol-5-yl, 2,3-Dihydrothiazol-2-yl, 2,3-Dihydrothiazol-4-yl, 2,3-Dihydrothiazol-5-yl, 1,3-Dioxol-2-yl, 1,3-Dioxol-4-yl, 1,3-Dithiol-2-yl, 1,3-Dithiol-4-yl, 1,3-Oxathiol-2-yl, 1,3-Oxathiol-4-yl, 1,3-Oxathiol-5-yl, Pyrazol-3-yl, Pyrazol-4-yl, Isoxazol-3-yl, Isoxazol-4-yl, Isoxazol-5-yl, Isothiazol-3-yl, Isothiazol-4-yl, Isothiazol-5-yl, Imidazol-2-yl, Imidazol-4-yl, Oxazol-2-yl, Oxazol-4-yl, Oxazol-5-yl, Thiazol-2-yl, Thiazol-4-yl oder Thiazol-5-yl;
   5-gliedrige Ringe mit drei Heteroatomen wie:
      1,2,3-Δ²-Oxadiazolin-4-yl, 1,2,3-Δ²-Oxadiazolin-5-yl, 1,2,4-Δ⁴-Oxadiazolin-3-yl, 1,2,4-Δ⁴-Oxadiazolin-5-yl, 1,2,4-Δ²-Oxadiazolin-3-yl, 2,2,4-Δ²-Oxadiazolin-5-yl, 1,2,4-Δ³-Oxadiazolin-3-yl, 1,2,4-Δ³-Oxadiazolin-5-yl, 1,3,4-Δ²-Oxadiazolin-2-yl, 1,3,4-Δ²-Oxadiazolin-5-yl, 1,3,4-Δ³-Oxadiazolin-2-yl, 1,3,4-Oxadiazolin-2-yl, 1,2,3-Δ²-Thiadiazolin-4-yl, 1,2,3-Δ²-Thiadiazolin-5-yl, 1,2,4-Δ⁴-Thiadiazolin-3-yl, 1,2,4-Δ⁴-Thiadiazolin-5-yl, 1,2,4-Δ³-Thiadiazolin-3-yl, 1,2,4-Δ³-Thiadiazolin-5-yl, 1,2,4-Δ²-Thiadiazolin-3-yl, 1,2,4-Δ²-Thiadiazolin-5-yl, 1,3,4-Δ²-Thiadiazolin-2-yl, 1,3,4-Δ²-Thiadiazolin-5-yl, 1,3,4-Δ³-Thiadiazolin-2-yl, 1,3,4-Thiadiazolin-2-yl, 1,3,2-Dioxathiolan-4-yl, 1,2,3-Δ²-Triazolin-4-yl, 1,2,3-Δ²-Triazolin-5-yl, 1,2,4-Δ²-Triazolin-3-yl, 1,2,4-Δ²-Triazolin-5-yl, 1,2,4-Δ³-Triazolin-3-yl, 1,2,4-Δ³-Triazolin-5-yl, 1,2,4-Δ¹-Triazolin-2-yl, 1,2,4-Triazolin-3-yl, 3H-1,2,4-Dithiazol-5-yl, 2H-1,3,4-Dithiazol-5-yl, 2H-1,3,4-Oxathiazol-5-yl, 1,2,3-Oxadiazol-4-yl, 1,2,3-Oxadiazol-5-yl, 1,2,4-Oxadiazol-3-yl, 1,2,4,-Oxadiazol-5-yl, 1,3,4-Oxadiazol-2-yl, 1,2,3-Thiadiazol-4-yl, 1,2,3-Thiadiazol-5-yl, 1,2,4-Thiadiazol-3-yl, 1,2,4-Thiadiazol-5-yl, 1,3,4-Thiadiazolyl-2-yl, 1,2,3-Triazol-4-yl oder 1,2,4-Triazol-3-yl;
   5-gliedrige Ringe mit vier Heteroatomen wie:
      Tetrazol-5-yl;

Alle Phenylringe bzw. Heterocyclylreste sowie alle Phenylkomponenten in Phenyl-C₁-C₆-alkyl, Phenylcarbonyl-C₁-C₆-alkyl, Phenylcarbonyl, Phenylalkenylcarbonyl, Phenoxycarbonyl, Phenyloxythiocarbonyl, Phenylaminocarbonyl und N-(C₁-C₆-Alkyl)-N-phenylaminocarbonyl bzw. Heterocyclylkomponenten in Heterocyclyl-C₁-C₆-alkyl, Heterocyclylcarbonyl-C₁-C₆-alkyl, Heterocyclylcarbonyl, Heterocyclyloxythiocarbonyl, Heterocyclylalkenylcarbonyl, Heterocyclyloxycarbonyl, Heterocyclylaminocarbonyl und N(C₁-C₆-Alkyl)-N-heterocyclylaminocarbonyl sind, soweit nicht anders angegeben, vorzugsweise unsubstituiert oder tragen ein bis drei Halogenatome und/oder eine Nitrogruppe, einen Cyanorest und/oder einen oder zwei Methyl-, Trifluormethyl-, Methoxy- oder Trifluormethoxysubstituenten.

Weiterhin steht der Ausdruck "Y bildet gemeinsam mit den beiden Kohlenstoffen, an die es gebunden ist, einen gesättigten, partiell gesättigten oder ungesättigten Heterocyclus, der ein bis drei gleiche oder verschiedene Heteroatome, ausgewählt aus folgender Gruppe: Sauerstoff, Schwefel oder Stickstoff, enthält" beispielsweise für 5-gliedrige Ringe mit einem Heteroatom, wie:
Tetrahydrofurandiyl, Tetrahydrothiendiyl, Tetrahydropyrroldiyl, Dihydrofurandiyl, Dihydrothiendiyl, Dihydropyrroldiyl, Furandiyl, Thiendiyl oder Pyrroldiyl;
oder 5-gliedrige Ringe mit zwei Heteroatomen wie:
Tetrahydropyrazoldiyl, Tetrahydroisoxazoldiyl, 1,2-Oxathiolandiyl, Tetrahydroisothiazoldiyl, 1,2-Dithiolandiyl, Tetrahydroimidazoldiyl, Tetrahydrooxazoldiyl, Tetrahydrothiazoldiyl, 1,3-Dioxolandiyl, 1,3-Oxathiolandiyl, Dihydropyrazoldiyl, Dihydroisoxazoldiyl, Dihydroisothiazoldiyl, 1,2-Dithioldiyl, Dihydroimidazoldiyl, Dihydrooxazoldiyl, Dihydrothiazoldiyl, Dioxoldiyl, Oxathioldiyl, Pyrazoldiyl, Isoxazoldiyl, Isothiazoldiyl, Imidazoldiyl, Oxazoldiyl oder Thiazoldiyl;
oder 5-gliedrige Ringe mit drei Heteroatomen wie:
   1,2,3-Oxadiazolindiyl, 1,2,3-Thiadiazolindiyl, 1,2,3-Triazolindiyl, 1,2,3-Oxadiazoldiyl, 1,2,3-Thiadiazoldiyl oder 1,2,3-Triazoldiyl;
wobei ggf. der Schwefel der genannten Heteroyclen zu S=O oder S(=O)₂ oxidiert sein kann;
und wobei die Anellierung mit dem Grundkörper über zwei benachbarte Kohlenstoffatome erfolgt.

Die erfindungsgemäßen Verbindungen der Formel I mit R⁹ = IIa werden als Verbindungen der Formel Ia sowie Verbindungen der Formel I mit R⁹ = IIb als Ib bezeichnet.

In Hinblick auf die Verwendung der erfindungsgemäßen Verbindungen der Formel I als Herbizide haben die Variablen vorzugsweise folgende Bedeutungen, und zwar jeweils für sich allein oder in Kombination:
- X: Sauerstoff, Schwefel, S=O oder S(=O)₂;
- Y: bildet gemeinsam mit den beiden Kohlenstoffen, and das es gebunden ist, einen gesättigten, partiell gesättigten oder ungesättigten 5-gliedrigen Heterocyclus, der ein bis zwei gleiche oder verschiedene Heteroatome, ausgewählt aus folgender Gruppe: Sauerstoff, Schwefel oder Stickstoff, enthält;
- R¹,R²: Wasserstoff oder C₁-C₆-Alkyl;
- R³: Halogen, C₁-C₆-Alkyl oder C₁-C₆-Alkoxy;
- R⁴: Nitro, Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, Aminosulfonyl, N-(C₁-C₆-alkyl)-aminosulfonyl, N,N-Di-(C₁-C₆-alkyl)-aminosulfonyl, N-((C₁-C₆-Alkylsulfonyl)-amino, N-(C₁-C₆-Halogenalkylsulfony)-amino, N- (C₁-C₆-Alkyl) -N-(C₁-C₆-alkylsulfonyl) -amino oder N-(C₁-C₆-alkyl) -N- (C₁-C₆-halogenalkylsulfonyl)-amino; insbesondere Nitro, Halogen, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfonyl oder C₁-C₆-Halogenalkylsulfonyl;
- R⁵: Wasserstoff;
- m: 0, 1 oder 2;
- R⁹: ein Rest IIa 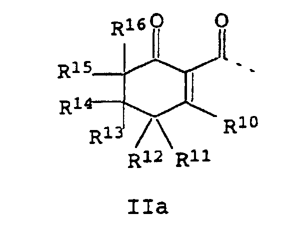
wobei
- R¹⁰: Hydroxy;
- R¹¹, R¹⁵: Wasserstoff oder C₁-C₄-Alkyl, wie Methyl, Ethyl oder Propyl;
bevorzugt Wasserstoff oder Methyl;
- R¹², R¹⁴, R¹⁶: Wasserstoff oder C₁-C₄-Alkyl, wie Methyl, Ethyl oder Propyl;
bevorzugt Wasserstoff oder Methyl;
- R¹³: Wasserstoff, Hydroxy, C₁-C₆-Alkyl, Di-(C₁-C₆-alkoxy)-methyl, (C₁-C₆-Alkoxy)- (C₁-C₆-alkylthio)-methyl, Di-(C₁-C₆-alkylthio)methyl, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl oder C₁-C₆-Halogenalkylsulfonyl; 1,3-Dioxolan-2-yl, 1,3-Dioxan-2-yl, 1,3-Oxathiolan-2-yl, 1,3-Oxathian-2-yl, 1,3-Dithiolan-2-yl oder 1,3-Dithian-2-yl, wobei die sechs letztgenannten Reste durch einen bis drei C₁-C₄-Alkylreste substituiert sein können;
bevorzugt Wasserstoff, Hydroxy oder C₁-C₄-Alkyl, wie Methyl, Ethyl oder Propyl;
oder
R¹² und R¹³ oder R¹³ und R¹⁶ bilden gemeinsam eine π-Bindung oder eine C₃-C₅-Alkylkette, die einen bis drei Reste aus folgender Gruppe tragen kan: Halogen, Cyano, C₁-C₄-Alkyl, C₁-C₄-Halogenalkyl oder C₁-C₄-Alkoxycarbonyl, aus;
oder
R¹¹ und R¹⁶ bilden gemeinsam eine C₁-C₄-Alkylkette, die eine bis drei Reste aus folgender Gruppe tragen kann: Halogen, Cyano, C₁-C₄-Alkyl, C₁-C₄-Halogenalkyl oder C₁-C₄-Alkoxycarbonyl, aus;
oder
R¹³ und R¹⁴ bilden gemeinsam eine -O-(CH₂)ₚ-O-, -O-(CH₂)ₚ-S- oder -S-(CH₂)ₚ-S-Kette, die durch einen bis drei Reste aus folgender Gruppe substituiert sein kann: Halogen, Cyano, C₁-C₄-Alkyl, C₁-C₄-Halogenalkyl oder C₁-C₄-Alkoxycarbonyl;
bevorzugt bilden R¹³ und R¹⁴ gemeinsam eine -O-(CH₂)ₚ-O-, -O-(CH₂)ₚ-S- oder -S-(CH₂)ₚ-S-Kette, die durch einen bis drei der Reste aus folgender Gruppen substituiert sein kann: C₁-C₄-Alkyl oder C₁-C₄-Alkoxycarbonyl;
oder
R¹³ und R¹⁴ bilden gemeinsam mit dem Kohlenstoff, an dem sie gebunden sind, eine Carbonylgruppe aus;
p 2, 3 oder 4;

Besonders bevorzugt sind Verbindungen der Formel I, wobei die Variablen folgende Bedeutungen haben, und zwar für sich allein oder in Kombination:
- X: Sauerstoff, Schwefel, S=O oder S(=O)₂,
- Y: bildet gemeinsam mit den beiden Kohlenstoffen, an die es gebunden ist, nachfolgende Heterocyclen aus:
(Bei den nachfolgenden Ausführungen der Heterocyclen stellt jeweils die obere Wellenlinie die Verknüpfung zum Kohlenwasserstoff, der die Reste R¹ und R² trägt, und die untere Wellenlinie die Verknüpfung zum meta-Kohlenstoff des Benzoylteils dar). 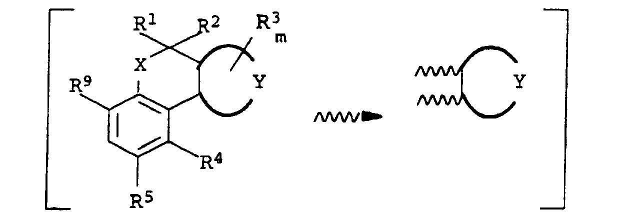 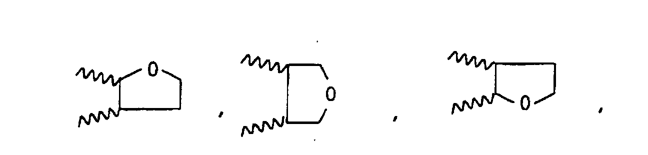 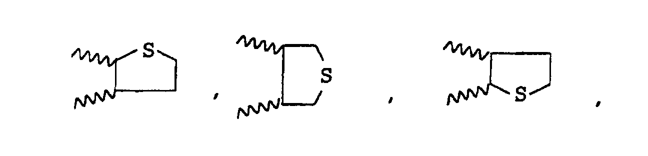 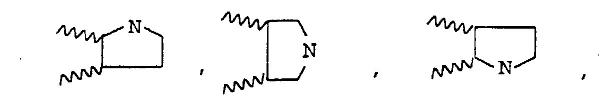 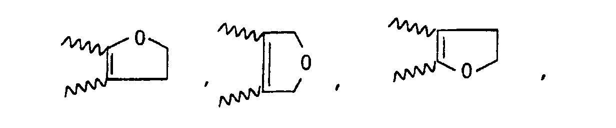 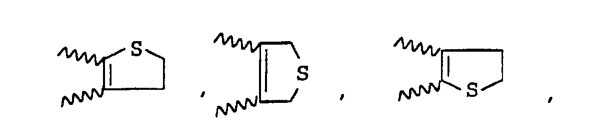 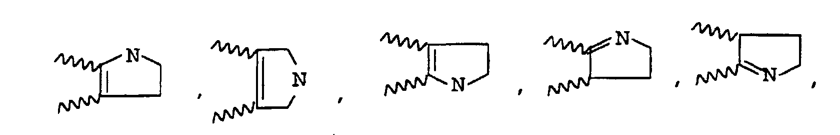 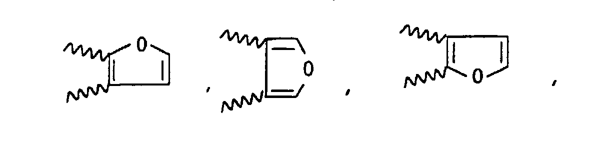 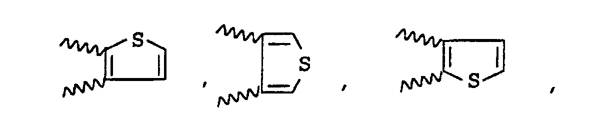 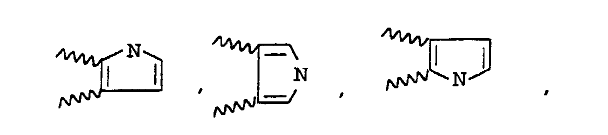 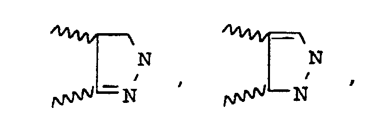 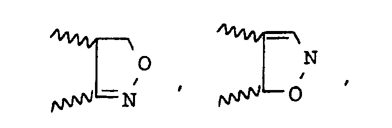 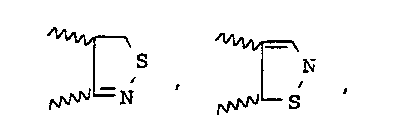 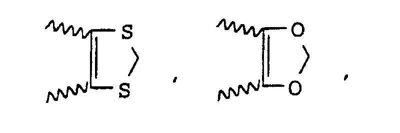 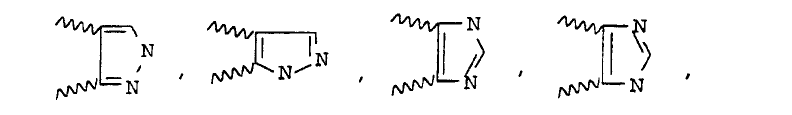 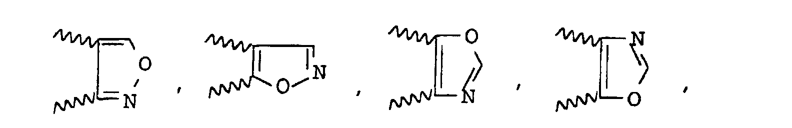 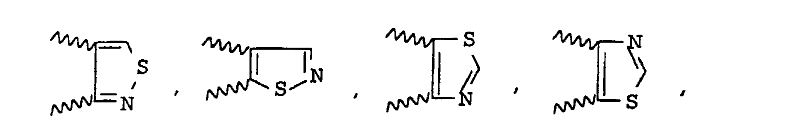 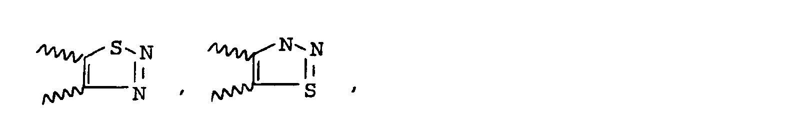 wobei der Schwefel der genannten Heterocyclen zu S=O oder S(=O)₂ oxidiert sein kann;
insbesondere bildet Y gemeinsam mit den beiden Kohlenstoffen, an die es gebunden ist, nachfolgende Heterocyclen aus: 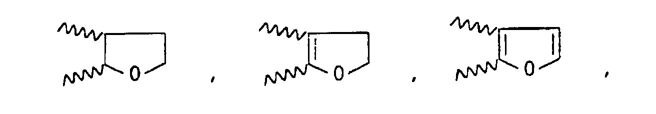 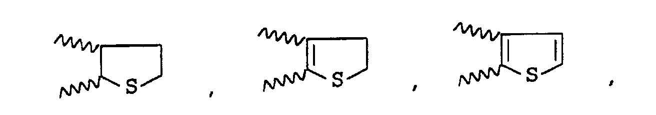 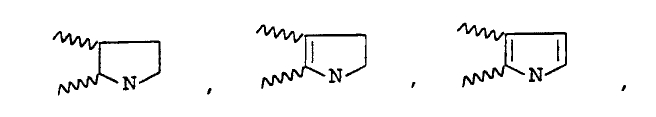 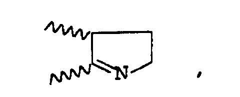 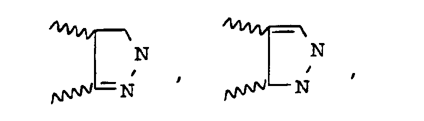 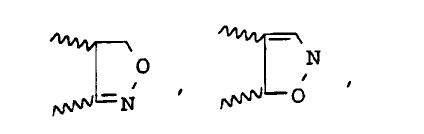 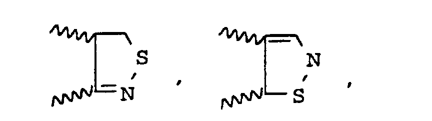 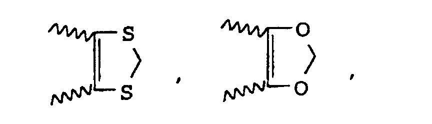 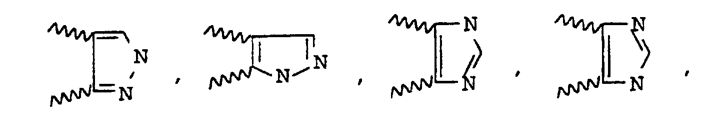 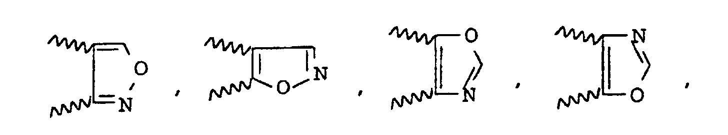 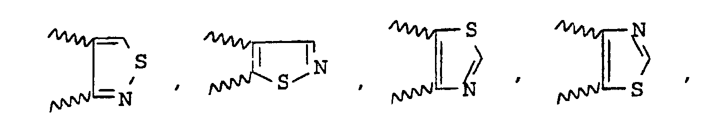 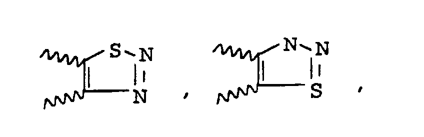
- R¹,R²: Wasserstoff;
- R³: C₁-C₆-Alkyl, wie Methyl, Ethyl oder n-Propyl; insbesondere Methyl;
- R⁴: Nitro, Halogen, C₁-C₆-Alkyl, C₁-C₆-Halogenalkyl, C₁-C₆-Alkoxy, C₁-C₆-Alkylthio oder C₁-C₆-Alkylsulfonyl; insbesondere Nitro, Halogen wie Fluor, Chlor oder Brom, C₁-C₆-Halogenalkyl wie Trifluormethyl, C₁-C₆-Alkylthio wie Methylthio oder Ethylthio oder C₁-C₆-Alkylsulfonyl, wie Methylsulfonyl oder Ethylsulfonyl; besonders bevorzugt Nitro, Chlor, Trifluormethyl, Methylthio oder Methylsulfonyl;
- R⁵: Wasserstoff;
- m: 0, 1 oder 2;
insbesondere 0 oder 1;
- R⁹: ein Rest IIa
- R¹⁰: Hydroxy,

Insbesondere bevorzugt sind die Verbindungen Ia, wobei
- X: Sauerstoff, Schwefel, S(=O)₂;
- Y: gemeinsam mit den beiden Kohlenstoffen, an die es gebunden ist, folgende Heterocyclen ausbildet: 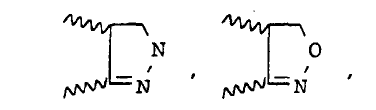 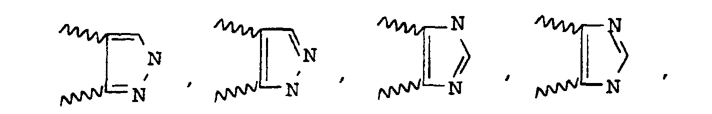 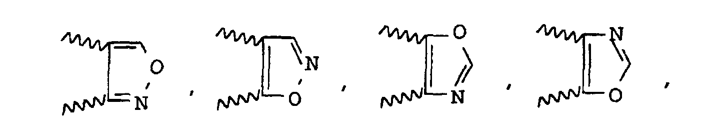 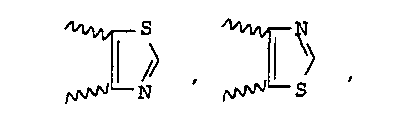
- R¹, R²: Wasserstoff;
- R³: C₁-C₄-Alkyl;
- R⁴: Nitro, Halogen, C₁-C₆-Alkyl, C₁-C₆-Halogenalkyl, C₁-C₆-Alkoxy, C₁-C₆-Alkylthio oder C₁-C₆-Alkylsulfonyl;
- R⁵: Wasserstoff oder C₁-C₆-Alkyl;
- m: 0, 1 oder 2;

Außerordentlich bevorzugt sind die Verbindungen der Formel Ia, mit
- R¹⁰: Hydroxy;

Ebenso außerordentlich bevorzugt sind die Verbindungen der Formel Ia, mit
- R¹¹, R¹⁵: Wasserstoff oder C₁-C₄-Alkyl;
- R¹², R¹⁴, R¹⁶: Wasserstoff oder C₁-C₄-Alkyl;
- R¹³: Wasserstoff, Hydroxy, C₁-C₆-Alkyl, Di-(C₁-C₆-alkoxy)-methyl, (C₁-C₆-Alkoxy)-(C₁-C₆-alkylthio)-methyl, Di-(C₁-C₆-alkylthio)-methyl, C₁-C₆-Alkylthio, C₁-C₆-Halogenalkylthio, C₁-C₆-Alkylsulfonyl oder C₁-C₆-Halogenalkylsulfonyl;
insbesondere Wasserstoff, Hydroxy oder C₁-C₆-Alkyl;
oder
R¹² und R¹⁶ bilden gemeinsam eine C₁-C₄-Alkylkette, die einen bis drei Reste aus folgender Gruppe tragen kann: Halogen, C₁-C₄-Alkyl oder C₁-C₄-Halogenalkyl;
insbesondere bilden R¹² und R¹⁶ gemeinsam eine Methylenbrücke aus, die einen oder zwei Reste aus folgender Gruppe tragen kann: Halogen, C₁-C₂-Alkyl oder C₁-C₂-Halogenalkyl;
oder
R¹³ und R¹⁴ bilden gemeinsam mit dem Kohlenstoff, an dem sie gebunden sind, eine Carbonylgruppe aus;
Ebenso außerordentlich bevorzugt sind die Verbindungen der Formel I, mit
- R¹¹, R¹⁵: Wasserstoff oder C₁-C₄-Alkyl;
- R¹², R¹⁴, R¹⁶: Wasserstoff oder C₁-C₄-Alkyl;
- R¹³: Wasserstoff, Hydroxy, C₁-C₆-Alkyl, Di-(C₁-C₆-alkoxy)-methyl, (C₁-C₆-Alkoxy)-(C₁-C₆-alkylthio)-methyl, Di-(C₁-C₆-alkylthio)methyl, C₁-C₆-Alkylthio, C₁-C₆-Halogenalkylthio, C₁-C₆-Alkylsulfonyl oder C₁-C₆-Halogenalkylsulfonyl;
insbesondere Wasserstoff, Hydroxy oder C₁-C₆-Alkyl;
oder
R¹³ und R¹⁴ bilden gemeinsam mit dem Kohlenstoff, an den sie gebunden sind eine Carbonylgruppe aus;

Ebenso insbesondere bevorzugt sind die Verbindungen der Formel I, wobei R⁹ folgende Bedeutung hat:

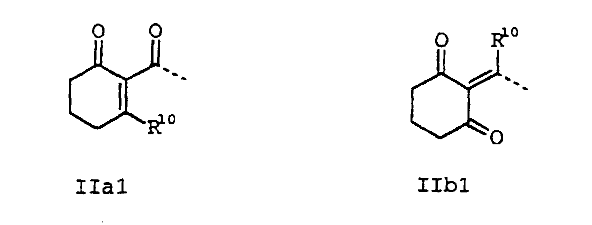

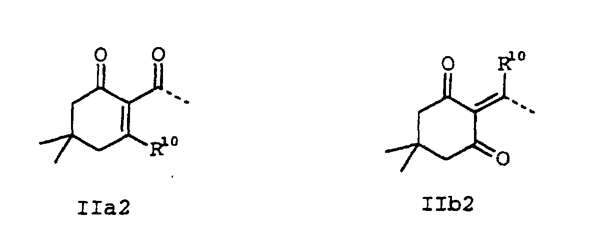

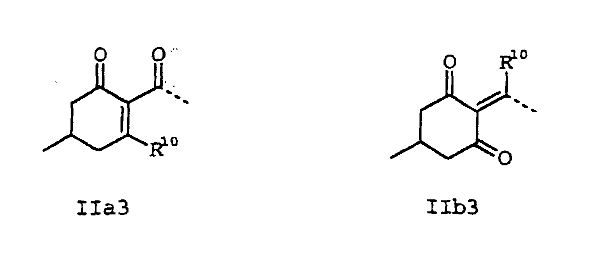

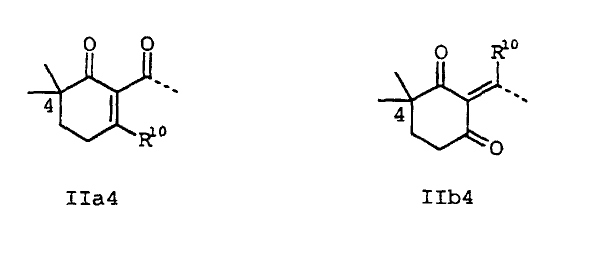

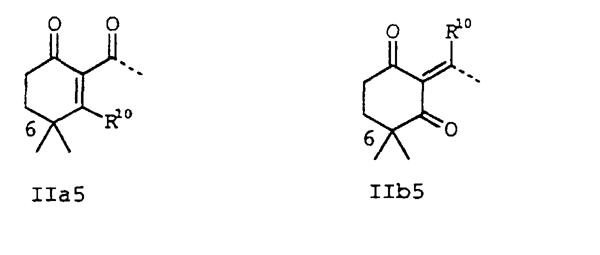

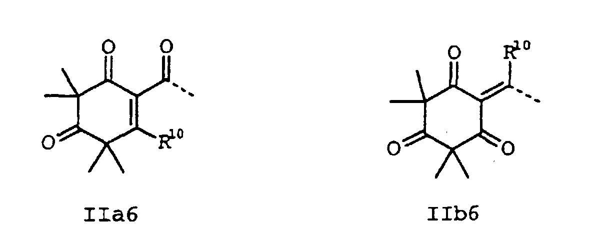

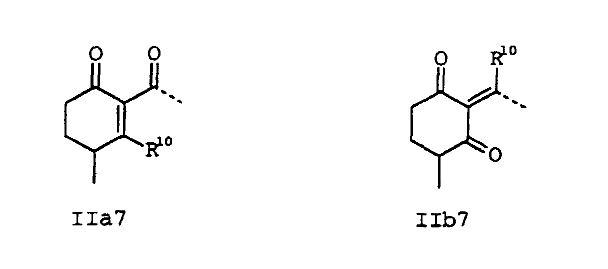

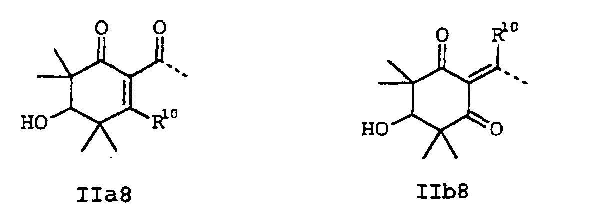

Ebenso insbesondere bevorzugt sind die Verbindungen der Formel Ia1 (≡ Ia mit R¹, R², R⁵ und R¹¹ bis R¹⁶ = H, m = O, Bedeutung des Heterocyclus laut Strukturformel), insbesonderst die Verbindungen Ia1.n, wobei die Variablen X, R⁴ und R¹⁰ die in Tabelle 1 genannte Bedeutung haben.
Die gegebenen Restedefinitionen R¹ bis R¹⁶, X, Y und m sowie die Bedeutung des anellierten Heterocyclus sind nicht nur in Kombination miteinander, sondern auch für sich allein betrachtet für die erfindungsgemäßen Verbindungen von besonderer Bedeutung.
(Aus Gründen der klareren Darstellung gilt jeweils in den Formeln Ia1, Ia2 ... die Bedeutung des anellierten Heterocyclus wie jeweils in der zugehörigen Strukturformel angegeben.)

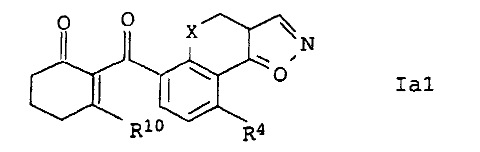

**Tabelle 1:**

| n | X | R⁴ | R¹⁰ |
|---|---|---|---|
| 21. | O | F | OH |
| 22. | O | Cl | OH |
| 23. | O | Br | OH |
| 24. | O | NO₂ | OH |
| 25. | O | SCH₃ | OH |
| 26. | O | SO₂CH₃ | OH |
| 27. | O | SO₂CH₂CH₃ | OH |
| 28. | O | CH₃ | OH |
| 29. | O | CF₃ | OH |
| 30. | O | OCHF₂ | OH |
| 31. | S | F | OH |
| 32. | S | Cl | OH |
| 33. | S | Br | OH |
| 34. | S | NO₂ | OH |
| 35. | S | SCH₃ | OH |
| 36. | S | SO₂CH₃ | OH |
| 37. | S | SO₂CH₂CH₃ | OH |
| 38. | S | CH₃ | OH |
| 39. | S | CF₃ | OH |
| 40. | S | OCHF₂ | OH |
| 41. | SO₂ | F | OH |
| 42. | SO₂ | Cl | OH |
| 43. | SO₂ | Br | OH |
| 44. | SO₂ | NO₂ | OH |
| 45. | SO₂ | SCH₃ | OH |
| 46. | SO₂ | SO₂CH₃ | OH |
| 47. | SO₂ | SO₂CH₂CH₃ | OH |
| 48. | SO₂ | CH₃ | OH |
| 49. | SO₂ | CF₃ | OH |
| 50. | SO₂ | OCHF₂ | OH |

Ebenso insbesondere bevorzugt sind die Verbindungen der Formel Ia2 (≡ Ia mit R¹, R², R⁵ und R¹¹ bis R¹⁶ = H, R³ = CH₃, m = 1), insbesondere die Verbindungen Ia2.n, wobei die variablen X, R⁴ und R¹⁰ die in Tabelle 1 genannte Bedeutung haben.

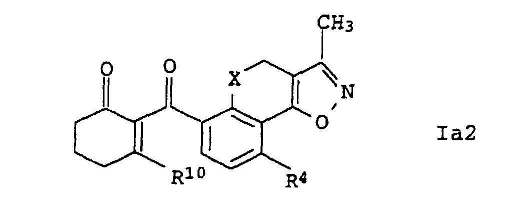

Ebenso insbesondere bevorzugt sind die Verbindungen der Formel Ia3 (≡ Ia mit R¹, R², R⁵ und R¹¹ bis R¹⁶ = H, R³ = CH₃, m = 1) , insbesondere die Verbindungen Ia3.n, wobei die Variablen X, R⁴ und R¹⁰ die in Tabelle 1 genannte Bedeutung haben.

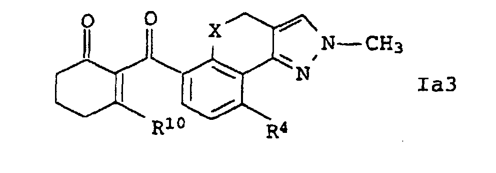

Ebenso insbesondere bevorzugt sind die Verbindungen der Formel Ia4 (≡ Ia mit R¹, R², R⁵ und bis R¹⁶ = H, R³ = CH₃, m = 2), insbesondere die Verbindungen Ia4.n, wobei die variablen X, R⁴ und R¹⁰ die in Tabelle 1 genannte Bedeutung haben.

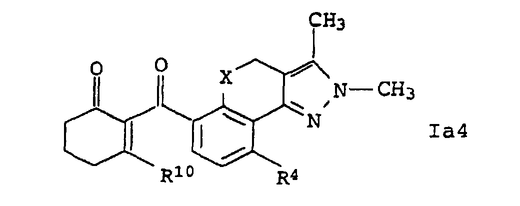

Ebenso insbesondere bevorzugt sind die Verbindungen der Formel Ia5 (≡ Ia mit R¹, R², R⁵ und R¹¹ bis R¹⁶ = H, R³ = CH₃, m = 1), insbesondere die Verbindungen Ia5.n, wobei die Variablen X, R⁴ und R¹⁰ die in Tabelle 1 genannte Bedeutung haben.

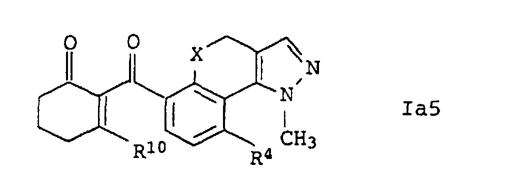

Ebenso insbesondere bevorzugt sind die Verbindungen der Formel Ia6 (≡ Ia mit R¹, R², R⁵ und R¹¹ bis R¹⁶ = H, R³ = CH₃, m = 2), insbesondere die Verbindungen Ia6.n, wobei die Variablen X, R⁴ und R¹⁰ die in Tabelle 1 genannte Bedeutung haben.

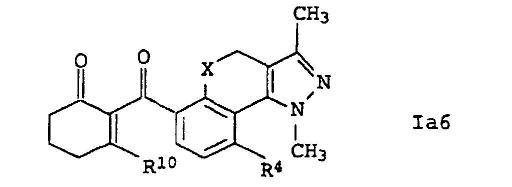

Ebenso insbesondere bevorzugt sind die Verbindungen der Formel Ia7 (≡ Ia mit R¹, R², R⁵ und R¹¹ bis R¹⁶ = H, m = 0), insbesondere die Verbindungen Ia7.n, wobei die Variablen X, R⁴ und R¹⁰ die in Tabelle 1 genannte Bedeutung haben.

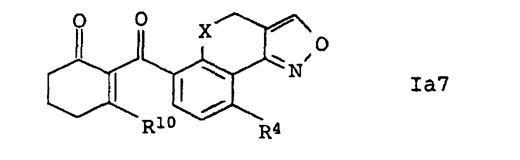

Ebenso insbesondere bevorzugt sind die Verbindungen der Formel Ia8 (≡ Ia mit R¹, R², R⁵ und R¹¹ bis R¹⁶ = H, R³ = CH³, m = 1), insbesondere die Verbindungen Ia8.n, wobei die Variablen X, R⁴ und R¹⁰ die in Tabelle 1 genannte Bedeutung haben.

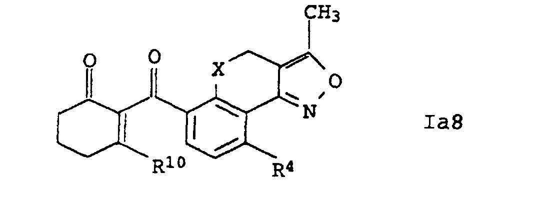

Ebenso insbesondere bevorzugt sind die Verbindungen der Formel Ia9 (≡ Ia mit R¹, R², R⁵ und R¹¹ bis R¹⁶ = H, m = 0), insbesondere die Verbindungen Ia9.n, wobei die Variablen X, R⁴ und R¹⁰ die in Tabelle 1 genannte Bedeutung haben.

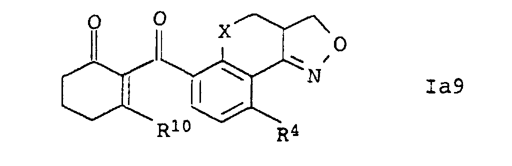

Ebenso insbesondere bevorzugt sind die Verbindungen der Formel Ia10 (≡ Ia mit R¹, R², R⁵ und R¹¹ bis R¹⁶ = H, m = 0), insbesondere die Verbindungen Ia10.n, wobei die Variablen X, R⁴ und R¹⁰ die in Tabelle 1 genannte Bedeutung haben.

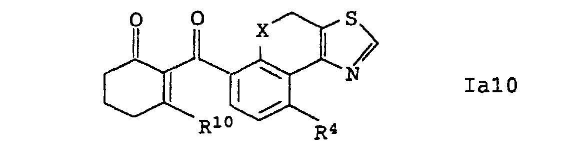

Ebenso insbesondere bevorzugt sind die Verbindungen der Formel Ia11 (≡ Ia mit R¹, R², R⁵ und R¹¹ bis R¹⁶ = H, m = 0), insbesondere die Verbindungen Iall.n, wobei die Variablen X, R⁴ bis R¹⁰ die in Tabelle 1 genannte Bedeutung haben.

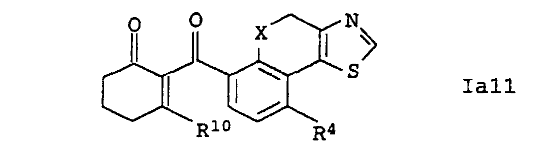

Ebenso insbesondere bevorzugt sind die Verbindungen der Formel Ia12 (≡ Ia mit R¹, R², R⁵ und R¹¹ bis R¹⁶ = H, m = 0), insbesondere die Verbindungen Ia12.n, wobei die Variablen X, R⁴ und R¹⁰ die in Tabelle 1 genannte Bedeutung haben.

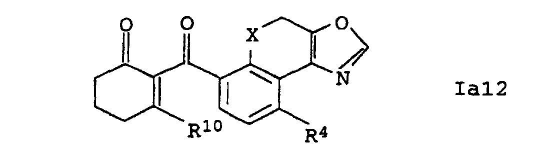

Ebenso insbesondere bevorzugt sind die Verbindungen der Formel Ia13 (≡ Ia mit R¹, R², R⁵ und R¹¹ bis R¹⁶ = H, m = 0), insbesondere die Verbindungen Ia13.n, wobei die Variablen X, R⁴ und R¹⁰ die in Tabelle 1 genannte Bedeutung haben.

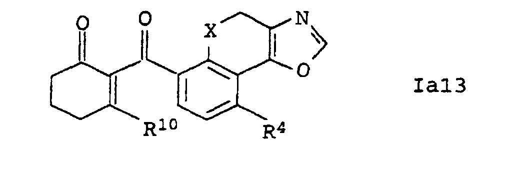

Ebenso insbesondere bevorzugt sind die Verbindungen der Formel Ia14 (≡ Ia mit R¹, R², R⁵ und R¹¹ bis R¹⁶ = H, R³ = CH₃, m = 1), insbesondere die Verbindungen Ia14.n, wobei die Variablen X, R⁴ und R¹⁰ die in Tabelle 1 genannte Bedeutung haben.

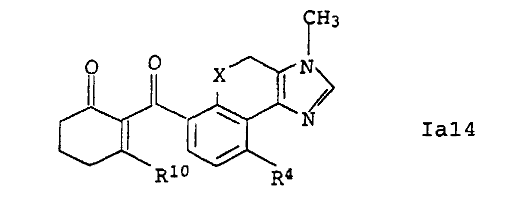

Ebenso insbesondere bevorzugt sind die Verbindungen der Formel Ia15 (≡ Ia mit R¹, R², R⁵ und R¹¹ bis R¹⁶ = H, R³ = CH₃, m = 1), insbesondere die Verbindungen Ia15.n, wobei die Variablen X, R4 und R¹⁰ die in Tabelle 1 genannte Bedeutung haben. 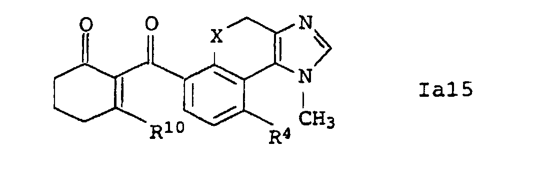 Ebenso insbesondere bevorzugt sind die Verbindungen der Formel Ia22 (≡ Ia mit R¹, R², R⁵ und R¹¹ bis R¹⁶ = H, R³ = CH₃, m = 1), insbesondere die Verbindungen Ia22.n, wobei die Variablen X, R4 und R¹⁰ die in Tabelle 1 genannte Bedeutung haben.

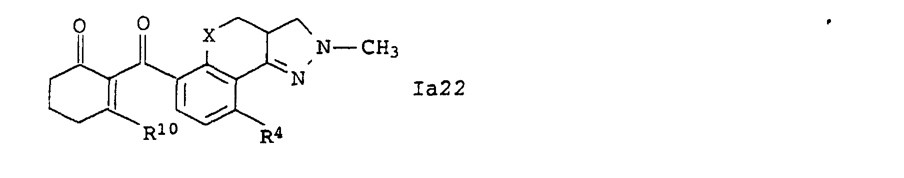

Ebenso insbesondere bevorzugt sind die Verbindungen der Formel Ia23 (=Ia mit R¹, R², R⁵ und R¹¹, R¹², R¹⁴ bis R¹⁶ = H, R¹³ = H, m = O), insbesondere die Verbindungen Ia23.n, wobei die Variablen X, R⁴ und R¹⁰ die in Tabelle I genannte Bedeutung haben.

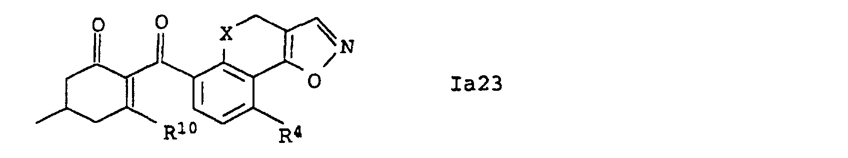

Ebenso insbesondere bevorzugt sind die Verbindungen der Formel Ia24 (≡ Ia mit R¹, R², R⁵ und R¹¹, R¹², R¹⁴ bis R¹⁶ = H, R³ und R¹³ = CH₃, m = 1), insbesondere die Verbindungen Ia24.n, wobei die Variablen X, R⁴ und R¹⁰ die in Tabelle 1 genannte Bedeutung haben.

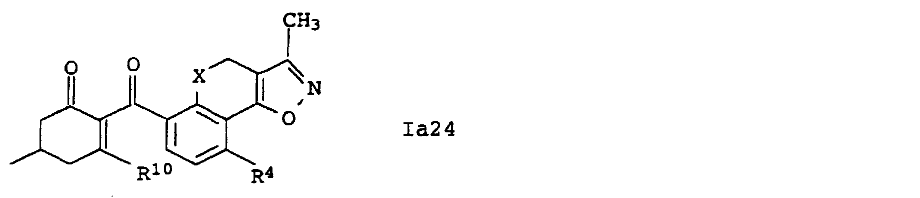

Ebenso insbesondere bevorzugt sind die Verbindungen der Formel Ia25 (≡ Ia mit R¹, R², R⁵ und R¹¹, R¹², R¹⁴ bis R¹⁶ = H, R³ und R¹³ = CH₃, m = 1), insbesondere die Verbindungen Ia25.n, wobei die Variablen X, R⁴ und R¹⁰ die in Tabelle 1 genannte Bedeutung haben.

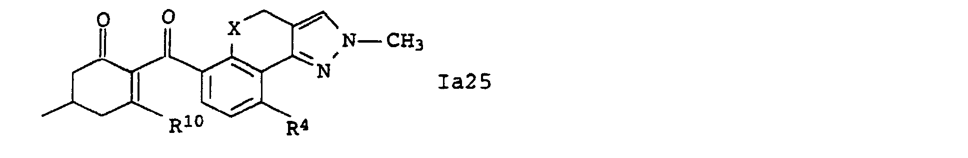

Ebenso insbesondere bevorzugt sind die Verbindungen der Formel Ia26 (≡ Ia mit R¹, R², R⁵ und R¹¹, R¹², R¹⁴ bis R¹⁶ = H, R³ und R¹³ = CH₃, m = 2), insbesondere die Verbindungen Ia26.n, wobei die Variablen X, R⁴ und R¹⁰ die in Tabelle 1 genannte Bedeutung haben.

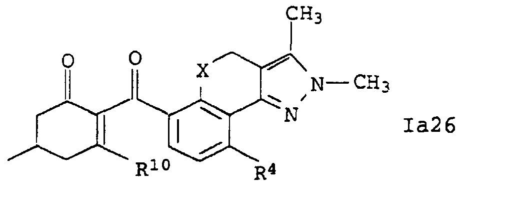

Ebenso insbesondere bevorzugt sind die Verbindungen der Formel Ia27 (≡ Ia mit R¹, R², R⁵ und R¹¹, R¹², R¹⁴ bis R¹⁶ = H, R³ und R¹³ = CH₃, m = 1), insbesondere die Verbindungen Ia27.n, wobei die Variablen X, R⁴ und R¹⁰ die in Tabelle 1 genannte Bedeutung haben.

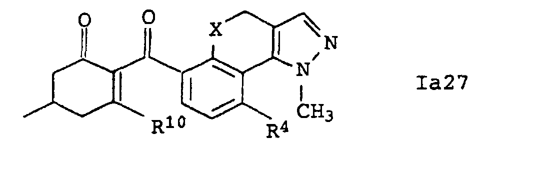

Ebenso insbesondere bevorzugt sind die Verbindungen der Formel Ia28 (≡ Ia mit R¹, R², R⁵ und R¹¹, R¹², R¹⁴ bis R¹⁶ = H, R³ und R¹³ = CH₃, m = 2), insbesondere die Verbindungen Ia28.n, wobei die Variablen X, R⁴ und R¹⁰ die in Tabelle 1 genannte Bedeutung haben.

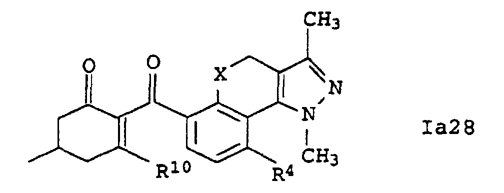

Ebenso insbesondere bevorzugt sind die Verbindungen der Formel Ia29 (≡ Ia mit R¹, R², R⁵ und R¹¹, R¹², R¹⁴ bis R¹⁶ = H, R¹³ = CH₃, m = 0), insbesondere die Verbindungen Ia29.n, wobei die Variablen X, R⁴ und R¹⁰ die in Tabelle 1 genannte Bedeutung haben.

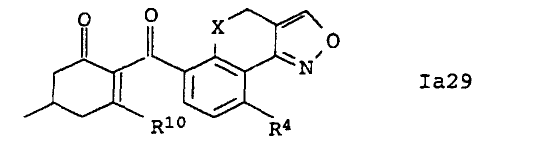

Ebenso insbesondere bevorzugt sind die Verbindungen der Formel Ia30 (≡ Ia mit R¹, R², R⁵ und R¹¹, R¹², R¹⁴ bis R¹⁶ = H, R³ und R¹³ = CH³, m = 1), insbesondere die Verbindungen Ia30.n, wobei die Variablen X, R⁴ und R¹⁰ die in Tabelle 1 genannte Bedeutung haben. 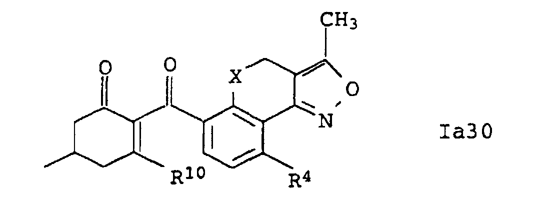 Ebenso insbesondere bevorzugt sind die Verbindungen der Formel Ia31 (≡ Ia mit R¹, R², R⁵ und R¹¹, R¹², R¹⁴ bis R¹⁶ = H, R¹³ = CH₃, m = 0), insbesondere die verbindungen Ia31.n, wobei die Variablen X, R⁴ und R¹⁰ die in Tabelle 1 genannte Bedeutung haben. 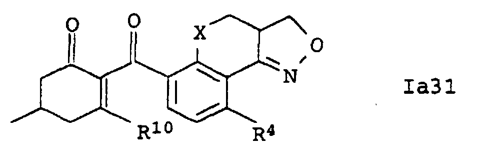 Ebenso insbesondere bevorzugt sind die Verbindungen der Formel Ia32 (≡ Ia mit R¹, R², R⁵ und R¹¹, R¹², R¹⁴ bis R¹⁶ = H, R¹³ = CH₃, m = 0), insbesondere die Verbindungen Ia32.n, wobei die Variablen X, R⁴ und R¹⁰ die in Tabelle 1 genannte Bedeutung haben. 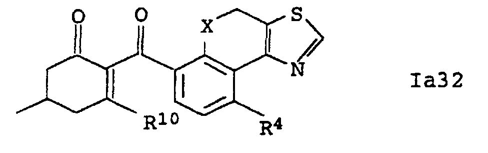 Ebenso insbesondere bevorzugt sind die Verbindungen der Formel Ia33 (≡ Ia mit R¹, R², R⁵ und R¹¹, R¹², R¹⁴ bis R¹⁶ = H, R¹³ = CH₃, m = 0), insbesondere die Verbindungen Ia33.n, wobei die Variablen X, R⁴ und R¹⁰ die in Tabelle 1 genannte Bedeutung haben. 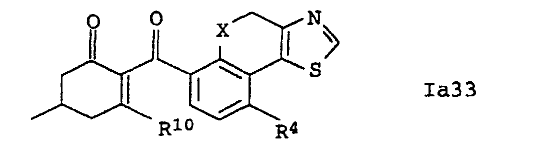 Ebenso insbesondere bevorzugt sind die Verbindungen der Formel Ia34 (≡ Ia mit R¹, R², R⁵ und R¹¹, R¹², R¹⁴ bis R¹⁶ = H, R¹³ = CH₃, m = 0), insbesondere die Verbindungen Ia34.n, wobei die Variablen X, R⁴ und R¹⁰ die in Tabelle 1 genannte Bedeutung haben. 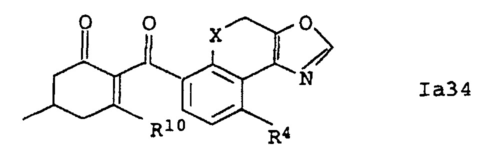 Ebenso insbesondere bevorzugt sind die Verbindungen der Formel Ia35 (≡ Ia mit R¹, R², R⁵ und R¹¹, R¹², R¹⁴ bis R¹⁶ = H, R¹³ = CH₃, m = 0), insbesondere die Verbindungen Ia35.n, wobei die Variablen X, R⁴ und R¹⁰ die in Tabelle 1 genannte Bedeutung haben.

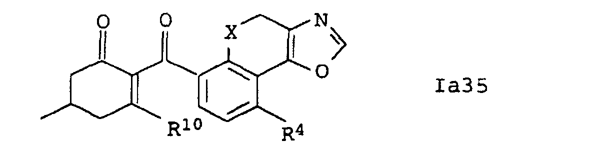

Ebenso insbesondere bevorzugt sind die Verbindungen der Formel Ia36 (≡ Ia mit R¹, R², R⁵ und R¹¹, R¹², R¹⁴ bis R¹⁶ = H, R³ und R¹³ = CH₃, m = 1), insbesondere die Verbindungen Ia36.n, wobei die Variablen X, R⁴ und R¹⁰ die in Tabelle 1 genannte Bedeutung haben.

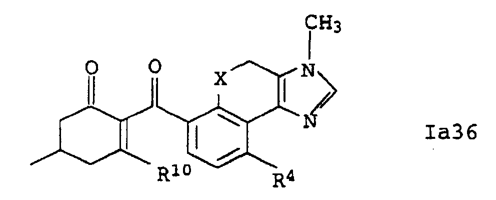

Ebenso insbesondere bevorzugt sind die Verbindungen der Formel Ia37 (≡ Ia mit R¹, R², R⁵ und R¹¹, R¹², R¹⁴ bis R¹⁶ = H, R³ und R¹³ = CH₃, m = 1), insbesondere die Verbindungen Ia37.n, wobei die Variablen X, R⁴ und R¹⁰ die in Tabelle 1 genannte Bedeutung haben.

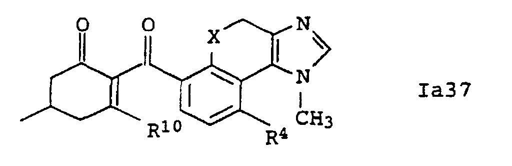

Ebenso insbesondere bevorzugt sind die Verbindungen der Formel Ia44 (≡ Ia mit R¹, R², R⁵ und R¹¹, R¹², R¹⁴ bis R¹⁶ = H, R³ und R¹³ = CH₃, m = 1), insbesondere die Verbindungen Ia44.n, wobei die Variablen X, R⁴ und R¹⁰ die in Tabelle 1 genannte Bedeutung haben.

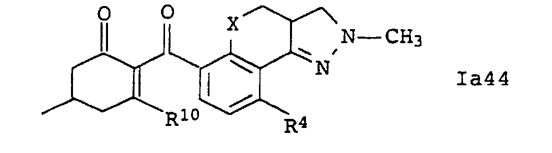

Ebenso insbesondere bevorzugt sind die Verbindungen der Formel Ia45 (≡ Ia mit R¹, R², R⁵ und R¹¹, R¹², R¹⁵ und R¹⁶ = H, R¹³ und R¹⁴ = CH₃, m = 0), insbesondere die Verbindungen Ia45.n, wobei die Variablen X, R⁴ und R¹⁰ die in Tabelle I genannte Bedeutung haben.

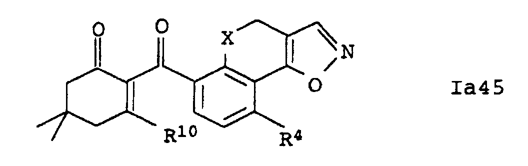

Ebenso insbesondere bevorzugt sind die Verbindungen der Formel Ia46 (≡ Ia mit R¹, R², R⁵ und R¹¹, R¹², R¹⁵, R¹⁶ = H, R³, R¹³ und R¹⁴ = CH₃, m = 1), insbesondere die Verbindungen Ia46.n, wobei die Variablen X, R⁴ und R¹⁰ die in Tabelle 1 genannte Bedeutung haben.

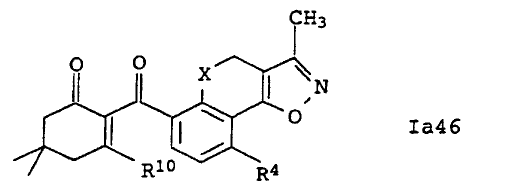

Ebenso insbesondere bevorzugt sind die Verbindungen der Formel Ia47 (≡ Ia mit R¹, R², R⁵ und R¹¹, R¹², R¹⁵, R¹⁶ = H, R³, R¹³ und R¹⁴ = CH₃, m = 1), insbesondere die Verbindungen Ia47.n, wobei die Variablen X, R⁴ und R¹⁰ die in Tabelle 1 genannte Bedeutung haben.

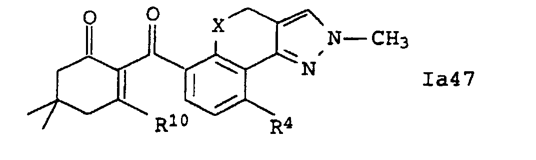

Ebenso insbesondere bevorzugt sind die Verbindungen der Formel Ia48 (≡ Ia mit R¹, R², R⁵ und R¹¹, R¹², R¹⁵, R¹⁶ = H, R³, R¹³ und R¹⁴ = CH₃, m = 2), insbesondere die Verbindungen Ia48.n, wobei die Variablen X, R⁴ und R¹⁰ die in Tabelle 1 genannte Bedeutung haben.

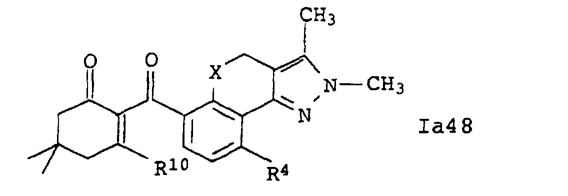

Ebenso insbesondere bevorzugt sind die Verbindungen der Formel Ia49 (≡ Ia mit R¹, R², R⁵ und R¹¹, R¹², R¹⁵, R¹⁶ = H, R³, R¹³ und R¹⁴ = CH₃, m = 1), insbesondere die Verbindungen Ia49.n, wobei die Variablen X, R⁴ und R¹⁰ die in Tabelle 1 genannte Bedeutung haben.

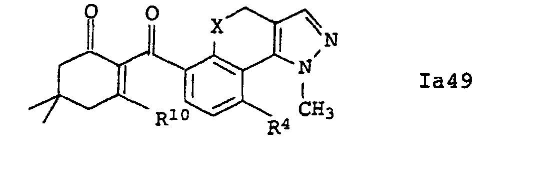

Ebenso insbesondere bevorzugt sind die Verbindungen der Formel Ia50 (≡ Ia mit R¹, R², R⁵ und R¹¹, R¹², R¹⁵, R¹⁶ = H, R³, R¹³ und R¹⁴ = CH₃, m = 2), insbesondere die Verbindungen Ia50.n, wobei die Variablen X, R⁴ und R¹⁰ die in Tabelle 1 genannte Bedeutung haben.

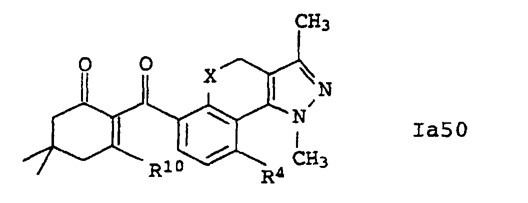

Ebenso insbesondere bevorzugt sind die Verbindungen der Formel Ia51 (≡ Ia mit R¹, R², R⁵ und R¹¹, R¹², R¹⁵, R¹⁶ = H, R¹³ und R¹⁴ = CH₃, m = 0), insbesondere die Verbindungen Ia51.n, wobei die Variablen X, R⁴ und R¹⁰ die in Tabelle 1 genannte Bedeutung haben.

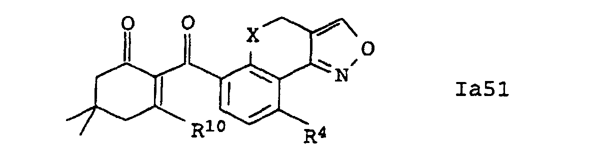

Ebenso insbesondere bevorzugt sind die Verbindungen der Formel Ia52 (≡ Ia mit R¹, R², R⁵ und R¹¹, R¹², R¹⁵, R¹⁶ = H, R³, R¹³ und R¹⁴ = CH³, m = 1), insbesondere die Verbindungen Ia52.n, wobei die Variablen X, R⁴ und R¹⁰ die in Tabelle 1 genannte Bedeutung haben.

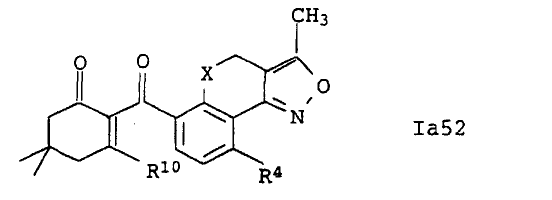

Ebenso insbesondere bevorzugt sind die Verbindungen der Formel Ia53 (≡ Ia mit R¹, R², R⁵ und R¹¹, R¹², R¹⁵, R¹⁶ = H, R¹³ und R¹⁴ = CH₃, m = 0), insbesondere die Verbindungen Ia53.n, wobei die Variablen X, R⁴ und R¹⁰ die in Tabelle 1 genannte Bedeutung haben.

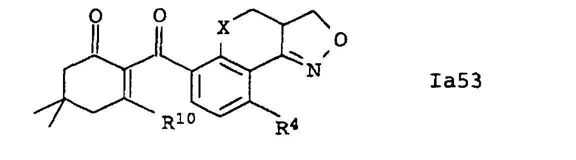

Ebenso insbesondere bevorzugt sind die Verbindungen der Formel Ia54 (≡ Ia mit R¹, R², R⁵ und R¹¹, R¹², R¹⁵, R¹⁶ = H, R¹³ und R¹⁴ = CH₃, m = 0), insbesondere die Verbindungen Ia54.n, wobei die Variablen X. R⁴ und R¹⁰ die in Tabelle 1 genannte Bedeutung haben.

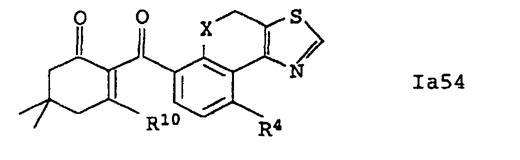

Ebenso insbesondere bevorzugt sind die Verbindungen der Formel Ia55 (≡ Ia mit R¹, R², R⁵ und R¹¹, R¹², R¹⁵, R¹⁶ = H, R¹³ und R¹⁴ = CH₃, m = 0), insbesondere die Verbindungen Ia55.n, wobei die Variablen X, R⁴ und R¹⁰ die in Tabelle 1 genannte Bedeutung haben.

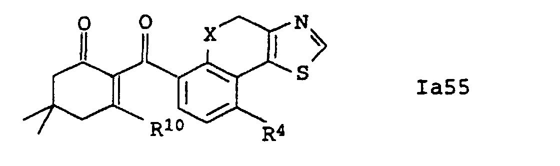

Ebenso insbesondere bevorzugt sind die Verbindungen der Formel Ia56 (≡ Ia mit R¹, R², R⁵ und R¹¹, R¹², R¹⁵, R¹⁶ = H, R¹³ und R¹⁴ = CH₃, m = 0), insbesondere die Verbindungen Ia56.n, wobei die Variablen X, R⁴ und R¹⁰ die in Tabelle 1 genannte Bedeutung haben.

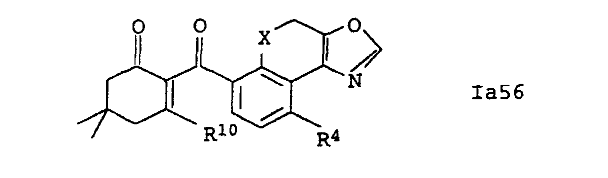

Ebenso insbesondere bevorzugt sind die Verbindungen der Formel Ia57 (≡ Ia mit R¹, R², R⁵ und R¹¹, R¹², R¹⁵, R¹⁶ = H, R¹³ und R¹⁴ = CH₃, m = 0), insbesondere die Verbindungen Ia57.n, wobei die Variablen X, R⁴ und R¹⁰ die in Tabelle 1 genannte Bedeutung haben.

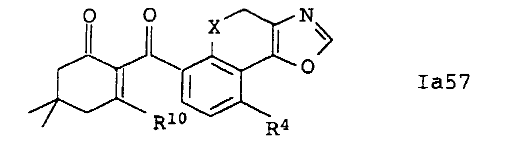

Ebenso insbesondere bevorzugt sind die Verbindungen der Formel Ia58 (≡ Ia mit R¹, R², R⁵ und R¹¹, R¹², R¹⁵, R¹⁶ = H, R³, R¹³ und R¹⁴ = CH₃, m = 1), insbesondere die Verbindungen Ia58.n, wobei die Variablen X, R⁴ und R¹⁰ die in Tabelle 1 genannte Bedeutung haben.

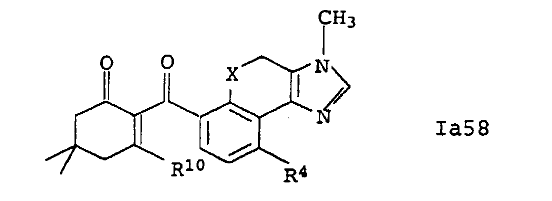

Ebenso insbesondere bevorzugt sind die Verbindungen der Formel Ia59 (≡ Ia mit R¹, R², R⁵ und R¹¹, R¹², R¹⁵, R¹⁶ = H, R³, R¹³ und R¹⁴ = CH₃, m = 1), insbesondere die Verbindungen Ia59.n, wobei die Variablen X, R⁴ und R¹⁰ die in Tabelle 1 genannte Bedeutung haben.

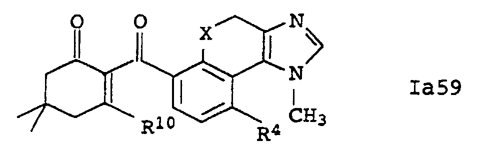

Ebenso insbesondere bevorzugt sind die Verbindungen der Formel Ia66 (≡ Ia mit R¹, R², R⁵ und R¹¹, R¹², R¹⁵, R¹⁶ = H, R³, R¹³ und R¹⁴ = CH₃, m = 1), insbesondere die Verbindungen Ia66.n, wobei die Variablen X, R⁴ und R¹⁰ die in Tabelle 1 genannte Bedeutung haben.

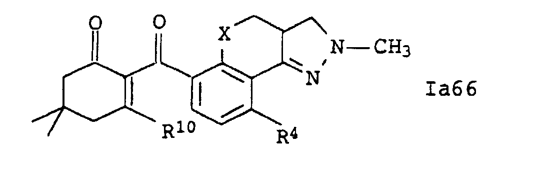

Ebenso insbesondere bevorzugt sind die Verbindungen der Formel Ia67 (≡ Ia mit R¹, R², R⁵ und R¹² bis R¹⁶ = H, R¹¹ = CH₃, m = 1), insbesondere die Verbindungen Ia67.n, wobei die Variablen X, R⁴ und R¹⁰ die in Tabelle 1 genannte Bedeutung haben.

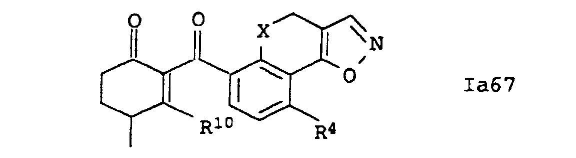

Ebenso insbesondere bevorzugt sind die Verbindungen der Formel Ia68 (≡ Ia mit R¹, R², R⁵ und R¹² bis R¹⁶ = H, R³ und R¹¹ = CH₃, m = 1), insbesondere die Verbindungen Ia68.n, wobei die Variablen X, R⁴ und R¹⁰ die in Tabelle 1 genannte Bedeutung haben.

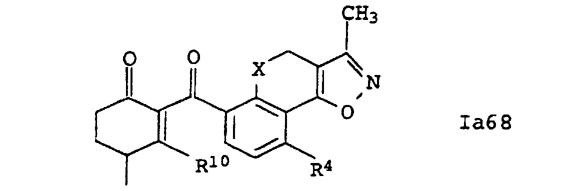

Ebenso insbesondere bevorzugt sind die Verbindungen der Formel Ia69 (≡ Ia mit R¹, R², R⁵ und R¹² bis R¹⁶ = H, R³ und R¹¹ = CH₃, m = 1), insbesondere die Verbindungen Ia69.n, wobei die Variablen X, R⁴ und R¹⁰ die in Tabelle 1 genannte Bedeutung haben.

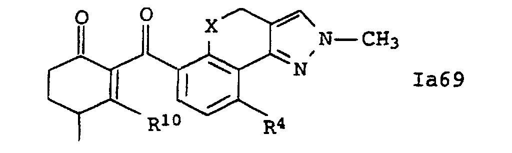

Ebenso insbesondere bevorzugt sind die Verbindungen der Formel Ia70 (≡ Ia mit R¹, R², R⁵ und R¹² bis R¹⁶ = H, R³ und R¹¹ = CH₃, m = 2), insbesondere die Verbindungen Ia70.n, wobei die Variablen X, R⁴ und R¹⁰ die in Tabelle 1 genannte Bedeutung haben.

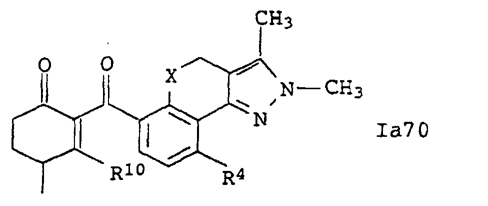

Ebenso insbesondere bevorzugt sind die Verbindungen der Formel Ia71 (≡ Ia mit R¹, R², R⁵ und R¹² bis R¹⁶ = H, R³ und R¹¹ = CH₃, m = 1), insbesondere die Verbindungen Ia71.n, wobei die Variablen X, R⁴ und R¹⁰ die in Tabelle 1 genannte Bedeutung haben.

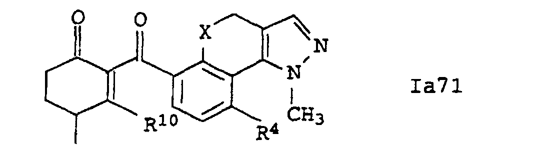

Ebenso insbesondere bevorzugt sind die Verbindungen der Formel Ia72 (≡ Ia mit R¹, R², R⁵ und R¹² bis R¹⁶ = H, R³ und R¹¹ = CH₃, m = 2), insbesondere die Verbindungen Ia72.n, wobei die Variablen X, R⁴ und R¹⁰ die in Tabelle 1 genannte Bedeutung haben.

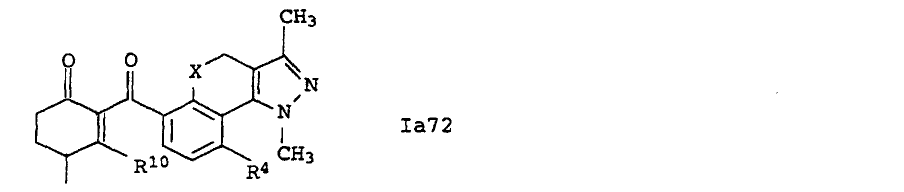

Ebenso insbesondere bevorzugt sind die Verbindungen der Formel Ia73 (≡ Ia mit R¹, R², R⁵ und R¹² bis R¹⁶ = H, R¹¹ = CH₃, m = 0), insbesondere die Verbindungen Ia73.n, wobei die Variablen X, R⁴ und R¹⁰ die in Tabelle 1 genannte Bedeutung haben.

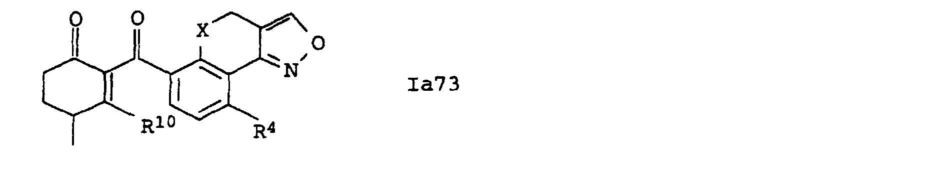

Ebenso insbesondere bevorzugt sind die Verbindungen der Formel Ia74 (≡ Ia mit R¹, R², R⁵ und R¹² bis R¹⁶ = H, R³ und R¹¹ = CH₃, m = 1), insbesondere die Verbindungen Ia74.n, wobei die Variablen X, R⁴ und R¹⁰ die in Tabelle 1 genannte Bedeutung haben.

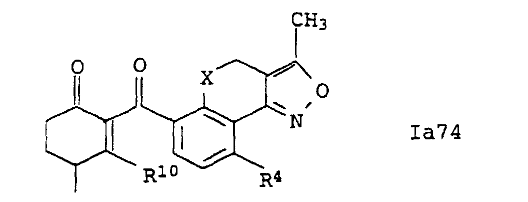

Ebenso insbesondere bevorzugt sind die Verbindungen der Formel Ia75 (≡ Ia mit R¹, R², R⁵ und R¹² bis R¹⁶ = H, R¹¹ = CH₃, m = 0), insbesondere die Verbindungen Ia75.n, wobei die Variablen X, R⁴ und R¹⁰ die in Tabelle 1 genannte Bedeutung haben.

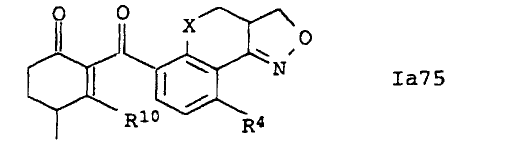

Ebenso insbesondere bevorzugt sind die Verbindungen der Formel Ia76 (≡ Ia mit R¹, R², R⁵ und R¹² bis R¹⁶ = H,R¹¹ = CH₃, m = 0), insbesondere die Verbindungen Ia76.n, wobei die Variablen X, R⁴ und R¹⁰ die in Tabelle 1 genannte Bedeutung haben.

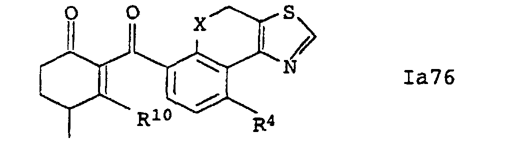

Ebenso insbesondere bevorzugt sind die Verbindungen der Formel Ia77 (≡ Ia mit R¹, R², R⁵ und R¹² bis R¹⁶ = H, R¹¹ = CH₃, m = 0), insbesondere die Verbindungen Ia77.n, wobei die Variablen X, R⁴ und R¹⁰ die in Tabelle 1 genannte Bedeutung haben.

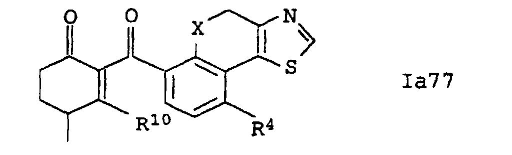

Ebenso insbesondere bevorzugt sind die Verbindungen der Formel Ia78 (≡ Ia mit R¹, R², R⁵ und R¹² bis R¹⁶ = H, R¹¹ = CH₃, m = 0), insbesondere die Verbindungen Ia78.n, wobei die Variablen X, R⁴ und R¹⁰ die in Tabelle 1 genannte Bedeutung haben.

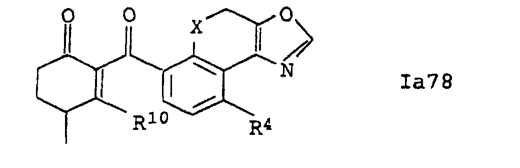

Ebenso insbesondere bevorzugt sind die Verbindungen der Formel Ia79 (≡ Ia mit R¹, R², R⁵ und R¹² bis R¹⁶ = H, R¹¹ = CH₃, m = 0), insbesondere die Verbindungen Ia79.n, wobei die variablen X, R⁴ und R¹⁰ die in Tabelle 1 genannte Bedeutung haben.

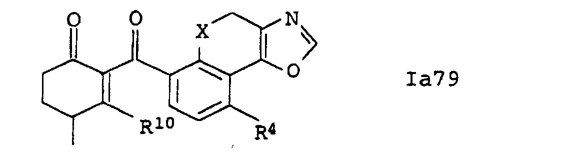

Ebenso insbesondere bevorzugt sind die Verbindungen der Formel Ia80 (≡ Ia mit R¹, R², R⁵ und R¹² bis R¹⁶ = H, R³ und R¹¹ = CH₃, m = 1), insbesondere die Verbindungen Ia80.n, wobei die Variablen X, R⁴ und R¹⁰ die in Tabelle 1 genannte Bedeutung haben.

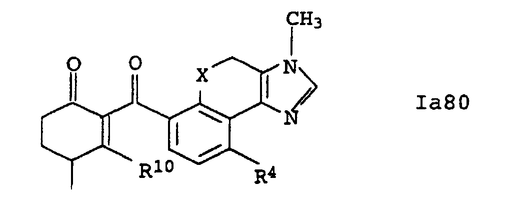

Ebenso insbesondere bevorzugt sind die Verbindungen der Formel Ia81 (≡ Ia mit R¹, R², R⁵ und R¹² bis R¹⁶ = H, R³ und R¹¹ = CH₃, m = 1), insbesondere die Verbindungen Ia81.n, wobei die Variablen X, R⁴ und R¹⁰ die in Tabelle 1 genannte Bedeutung haben.

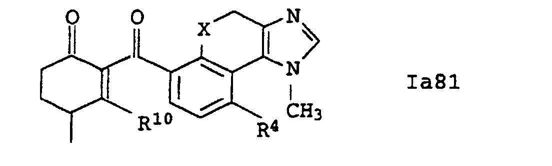

Ebenso insbesondere bevorzugt sind die Verbindungen der Formel Ia88 (≡ Ia mit R¹, R², R⁵ und R¹² bis R¹⁶ = H, R³ und R¹¹ = CH₃, m = 1), insbesondere die Verbindungen Ia88.n, wobei die Variablen X, R⁴ und R¹⁰ die in Tabelle 1 genannte Bedeutung haben.

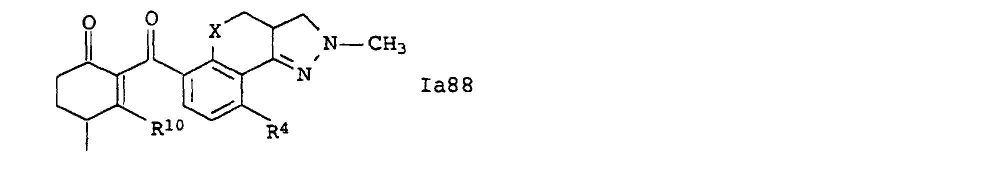

Ebenso insbesondere bevorzugt sind die Verbindungen der Formel Ia89 (≡ Ia mit R¹, R², R⁵ und R¹³ bis R¹⁶ = H, R¹¹ und R¹² = CH₃, m = 0), insbesondere die Verbindungen Ia89.n, wobei die Variablen X, R⁴ und R¹⁰ die in Tabelle 1 genannte Bedeutung haben.

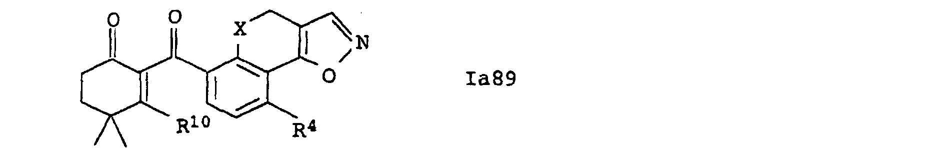

Ebenso insbesondere bevorzugt sind die Verbindungen der Formel Ia90 (≡ Ia mit R¹, R², R⁵ und R¹³ bis R¹⁶ = H, R³, R¹¹ und R¹² = CH₃, m = 1), insbesondere die Verbindungen Ia90.n, wobei die Variablen X, R⁴ und R¹⁰ die in Tabelle 1 genannte Bedeutung haben.

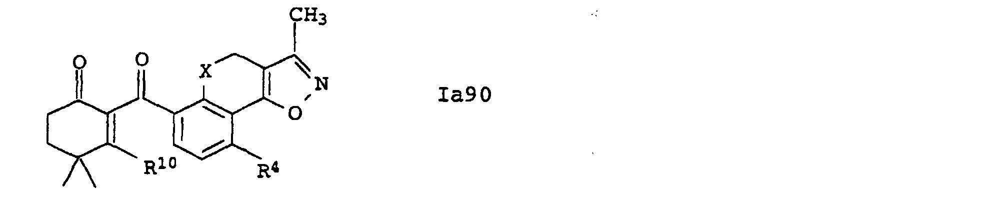

Ebenso insbesondere bevorzugt sind die Verbindungen der Formel Ia91 (≡ Ia mit R¹, R², R⁵, R¹³ bis R¹⁶ = H, R³, R¹¹ und R¹² = CH₃, m = 1), insbesondere die Verbindungen Ia91.n, wobei die Variablen X, R⁴ und R¹⁰ die in Tabelle 1 genannte Bedeutung haben.

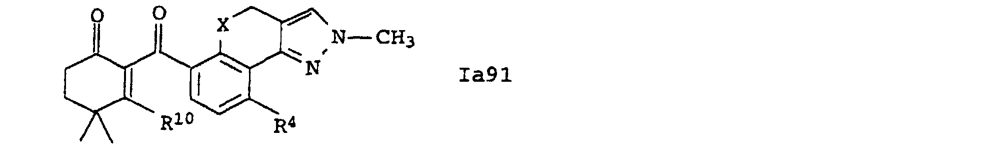

Ebenso insbesondere bevorzugt sind die Verbindungen der Formel Ia92 (≡ Ia mit R¹, R², R⁵, R¹³ bis R¹⁶ = H, R³, R¹¹ und R¹² = CH₃, m = 2), insbesondere die Verbindungen Ia92.n, wobei die Variablen X, R⁴ und R¹⁰ die in Tabelle 1 genannte Bedeutung haben.

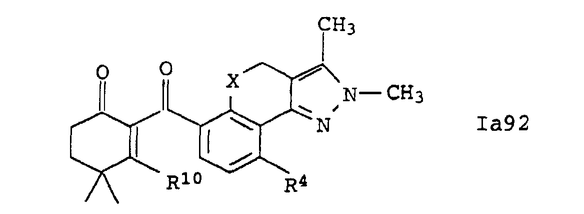

Ebenso insbesondere bevorzugt sind die Verbindungen der Formel Ia93 (≡ Ia mit R¹, R², R⁵ und R¹³ bis R¹⁶ = H, R³, R¹¹ und R¹² = CH₃, m = 1), insbesondere die Verbindungen Ia93.n, wobei die Variablen X, R⁴ und R¹⁰ die in Tabelle 1 genannte Bedeutung haben.

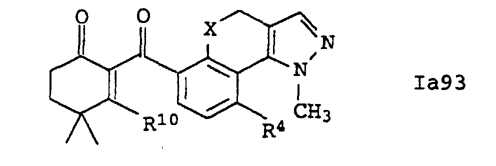

Ebenso insbesondere bevorzugt sind die Verbindungen der Formel Ia94 (≡ Ia mit R¹, R², R⁵ und R¹³ bis R¹⁶ = H, R³, R¹¹ und R¹² = CH₃, m = 2), insbesondere die Verbindungen Ia94.n, wobei die Variablen X, R⁴ und R¹⁰ die in Tabelle 1 genannte Bedeutung haben.

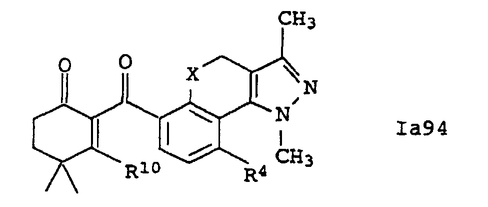

Ebenso insbesondere bevorzugt sind die Verbindungen der Formel Ia95 (≡ Ia mit R¹, R², R⁵ und R¹² bis R¹⁶ = H, R¹¹ und R¹² = CH₃, m = 0), insbesondere die Verbindungen Ia95.n, wobei die Variablen X, R⁴ und R¹⁰ die in Tabelle 1 genannte Bedeutung haben.

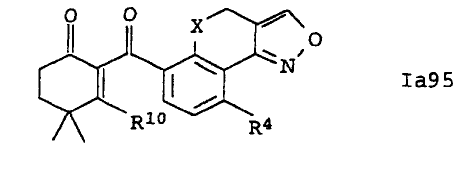

Ebenso insbesondere bevorzugt sind die Verbindungen der Formel Ia96 (≡ Ia mit R¹, R², R⁵ und R¹³ bis R¹⁶ = H, R³, R¹¹ und R¹² = CH₃, m = 1), insbesondere die Verbindungen Ia96.n, wobei die Variablen X, R⁴ und R¹⁰ die in Tabelle 1 genannte Bedeutung haben.

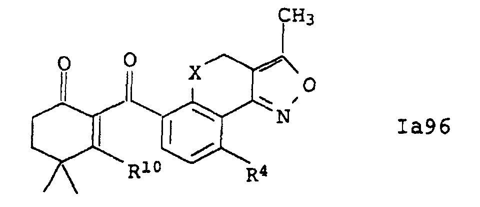

Ebenso insbesondere bevorzugt sind die Verbindungen der Formel Ia97 (≡ Ia mit R¹, R², R⁵ und R¹³ bis R¹⁶ = H, R¹¹ und R¹² = CH₃, m = 0), insbesondere die Verbindungen Ia97.n, wobei die Variablen X, R⁴ und R¹⁰ die in Tabelle 1 genannte Bedeutung haben.

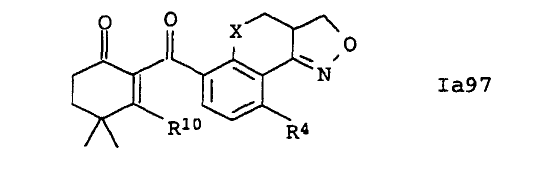

Ebenso insbesondere bevorzugt sind die Verbindungen der Formel Ia98 (≡ Ia mit R¹, R², R⁵ und R¹³ bis R¹⁶ = H, R¹¹ und R¹² = CH₃, m = 0), insbesondere die Verbindungen Ia98.n, wobei die Variablen X, R⁴ und R¹⁰ die in Tabelle 1 genannte Bedeutung haben.

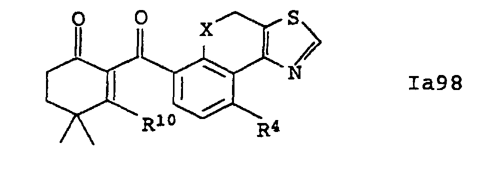

Ebenso insbesondere bevorzugt sind die Verbindungen der Formel Ia99 (≡ Ia mit R¹, R², R⁵ und R¹³ bis R¹⁶ = H, R¹¹ und R¹² = CH₃, m = 0), insbesondere die Verbindungen Ia99.n, wobei die Variablen X, R⁴ bis R¹⁰ die in Tabelle 1 genannte Bedeutung haben.

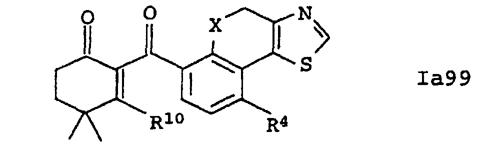

Ebenso insbesondere bevorzugt sind die Verbindungen der Formel Ia100 (≡ Ia mit R¹, R², R⁵ und R¹³ bis R¹⁶ = H, R¹¹ und R¹² = CH₃, m = 0), insbesondere die Verbindungen Ia100.n, wobei die Variablen X, R⁴ und R¹⁰ die in Tabelle 1 genannte Bedeutung haben.

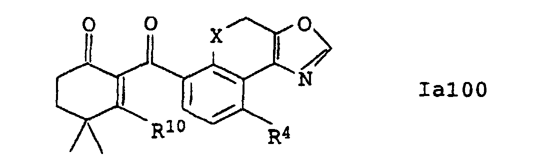

Ebenso insbesondere bevorzugt sind die Verbindungen der Formel Ia101 (≡ Ia mit R¹, R², R⁵ und R¹³ bis R¹⁶ = H, R¹¹ und R¹² = CH₃, m = 0), insbesondere die Verbindungen Ia101.n, wobei die Variablen X, R⁴ und R¹⁰ die in Tabelle 1 genannte Bedeutung haben.

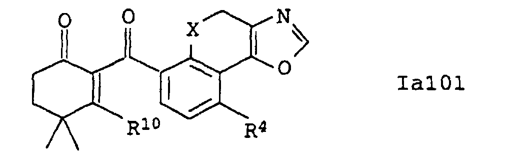

Ebenso insbesondere bevorzugt sind die Verbindungen der Formel Ia102 (≡ Ia mit R¹, R², R⁵ und R¹³ bis R¹⁶ = H, R³, R¹¹ und R¹² = CH₃, m = 1), insbesondere die Verbindungen Ia102.n, wobei die Variablen X, R⁴ und R¹⁰ die in Tabelle 1 genannte Bedeutung haben.

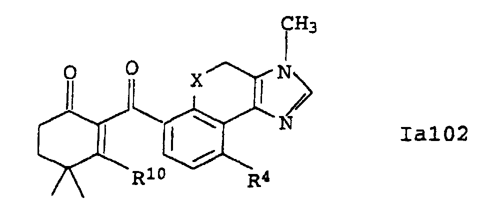

Ebenso insbesondere bevorzugt sind die Verbindungen der Formel Ia103 (≡ Ia mit R¹, R², R⁵ und R¹³ bis R¹⁶ = H, R³, R¹¹ und R¹² = CH₃, m = 1), insbesondere die Verbindungen Ia103.n, wobei die Variablen X, R⁴ und R¹⁰ die in Tabelle 1 genannte Bedeutung haben.

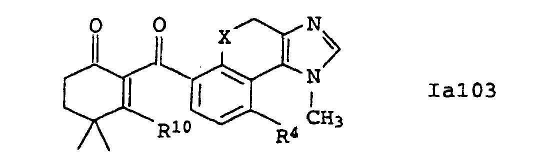

Ebenso insbesondere bevorzugt sind die Verbindungen der Formel Ia110 (≡ Ia mit R¹, R², R⁵ und R¹³ bis R¹⁶ = H, R³, R¹¹ und R¹² = CH₃, m = 1), insbesondere die Verbindungen Ia110.n, wobei die Variablen X, R⁴ und R¹⁰ die in Tabelle 1 genannte Bedeutung haben.

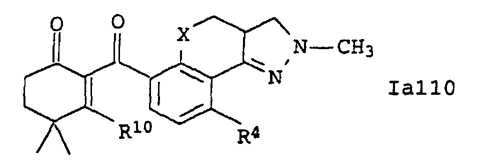

Ebenso insbesondere bevorzugt sind die Verbindungen der Formel Ia111 (≡ Ia mit R¹, R², R⁵ und R¹¹ bis R¹⁴ = H, R¹⁵ und R¹⁶ = CH₃, m = 0), insbesondere die Verbindungen Ia111.n, wobei die Variablen X, R⁴ und R¹⁰ die in Tabelle 1 genannte Bedeutung haben.

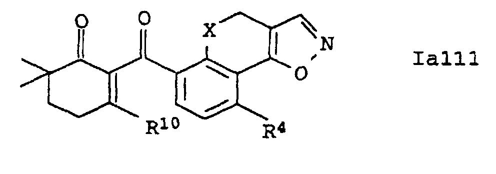

Ebenso insbesondere bevorzugt sind die Verbindungen der Formel Ia112 (≡ Ia mit R¹, R², R⁵ und R¹¹ bis R¹⁴ = H, R³, R¹⁵ und R¹⁶ = CH₃, m = 1), insbesondere die Verbindungen Ia111.n, wobei die variablen X, R⁴ und R¹⁰ die in Tabelle 1 genannte Bedeutung haben.

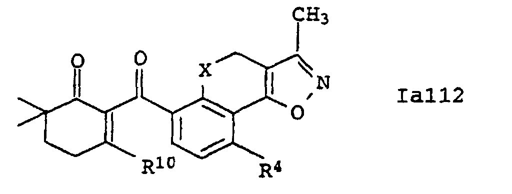

Ebenso insbesondere bevorzugt sind die Verbindungen der Formel Ia113 (≡ Ia mit R¹, R², R⁵ und R¹¹ bis R¹⁴ = H, R³, R¹⁵ und R¹⁶ = CH₃, m = 1), insbesondere die Verbindungen Ia113.n, wobei die Variablen X, R⁴ und R¹⁰ die in Tabelle 1 genannte Bedeutung haben.

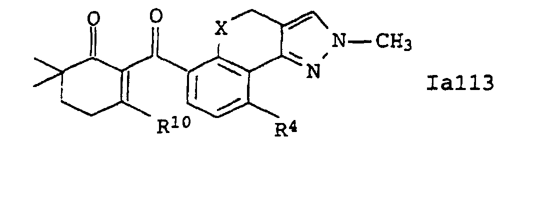

Ebenso insbesondere bevorzugt sind die Verbindungen der Formel Ia114 (≡ Ia mit R¹, R², R⁵ und R¹¹ bis R¹⁴ = H, R³, R¹⁵ und R¹⁶ = CH₃, m = 2), insbesondere die Verbindungen Ia114.n, wobei die Variablen X, R⁴ und R¹⁰ die in Tabelle 1 genannte Bedeutung haben.

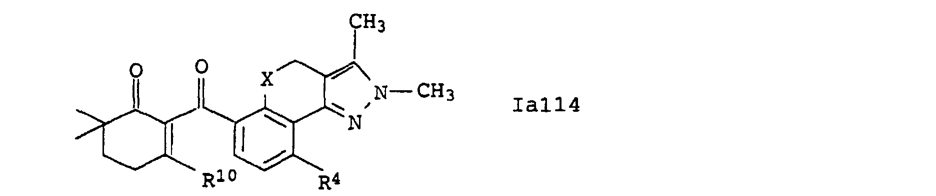

Ebenso insbesondere bevorzugt sind die Verbindungen der Formel Ia115 (≡ Ia mit R¹, R², R⁵ und R¹¹ bis R¹⁴ = H, R³, R¹⁵ und R¹⁶ = CH₃, m = 1), insbesondere die Verbindungen Ia115.n, wobei die Variablen X, R⁴ und R¹⁰ die in Tabelle 1 genannte Bedeutung haben.

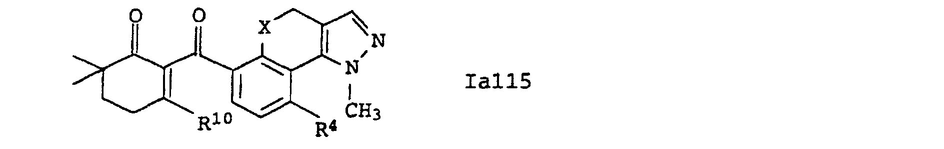

Ebenso insbesondere bevorzugt sind die Verbindungen der Formel Ia116 (≡ Ia mit R¹, R², R⁵ und R¹¹ bis R¹⁴ = H, R³, R¹⁵ und R¹⁶ = CH₃, m = 2), insbesondere die Verbindungen Ia116.n, wobei die variablen X, R⁴ und R¹⁰ die in Tabelle 1 genannte Bedeutung haben.

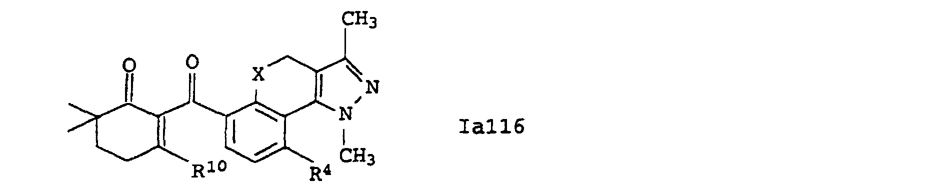

Ebenso insbesondere bevorzugt sind die Verbindungen der Formel Ia117 (≡ Ia mit R¹, R², R⁵ und R¹¹ bis R¹⁴ = H, R¹⁵ und R¹⁶ = CH₃, m = 0), insbesondere die Verbindungen Ia117.n, wobei die Variablen X, R⁴ und R¹⁰ die in Tabelle 1 genannte Bedeutung haben.

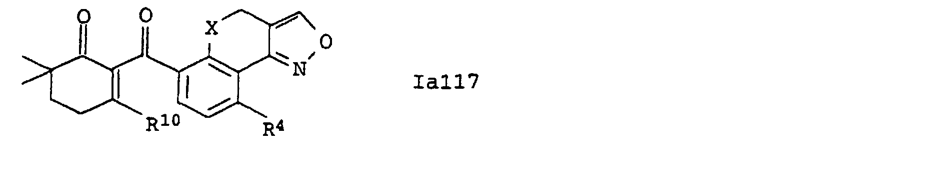

Ebenso insbesondere bevorzugt sind die Verbindungen der Formel Ia118 (≡ Ia mit R¹, R², R⁵ und R¹¹ bis R¹⁴ = H, R³, R¹⁵ und R¹⁶ = CH₃, m = 1), insbesondere die Verbindungen Ia118.n, wobei die Variablen X, R⁴ und R¹⁰ die in Tabelle 1 genannte Bedeutung haben.

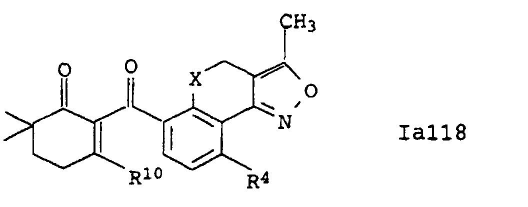

Ebenso insbesondere bevorzugt sind die Verbindungen der Formel Ia119 (≡ Ia mit R¹, R², R⁵ und R¹¹ bis R¹⁴ = H, R¹⁵ und R¹⁶ = CH₃, m = 0), insbesondere die Verbindungen Ia119.n, wobei die Variablen X, R⁴ und R¹⁰ die in Tabelle 1 genannte Bedeutung haben.

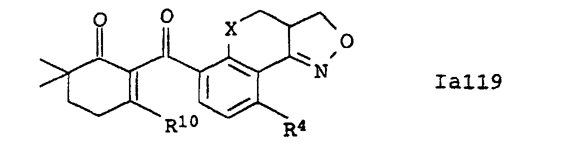

Ebenso insbesondere bevorzugt sind die Verbindungen der Formel Ia120 (≡ Ia mit R¹, R², R⁵ und R¹¹ bis R¹⁴ = H, R¹⁵ und R¹⁶ = CH₃, m = 0), insbesondere die Verbindungen Ia120.n, wobei die Variablen X, R⁴ und R¹⁰ die in Tabelle 1 genannte Bedeutung haben.

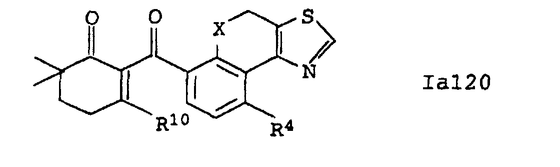

Ebenso insbesondere bevorzugt sind die Verbindungen der Formel Ia121 (≡ Ia mit R¹, R², R⁵ und R¹¹ bis R¹⁴ = H, R¹⁵ und R¹⁶ = CH₃, m = 0), insbesondere die Verbindungen Ia121.n, wobei die Variablen X, R⁴ bis R¹⁰ die in Tabelle 1 genannte Bedeutung haben.

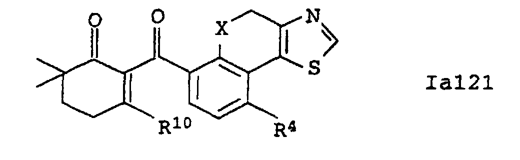

Ebenso insbesondere bevorzugt sind die Verbindungen der Formel Ia122 (≡ Ia mit R¹, R², R⁵ und R¹¹ bis R¹⁴ = H, R¹⁵ und R¹⁶ = CH₃, m = 0), insbesondere die Verbindungen Ia122.n, wobei die Variablen X, R⁴ und R¹⁰ die in Tabelle 1 genannte Bedeutung haben.

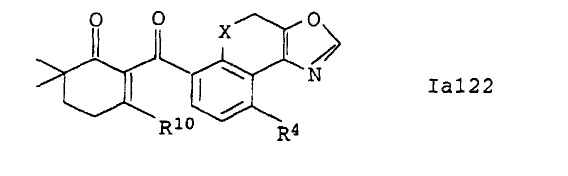

Ebenso insbesondere bevorzugt sind die Verbindungen der Formel Ia123 (≡ Ia mit R¹, R², R⁵ und R¹¹ bis R¹⁴ = H, R¹⁵ und R¹⁶ = CH₃, m = 0), insbesondere die Verbindungen Ia123.n, wobei die Variablen X, R⁴ und R¹⁰ die in Tabelle 1 genannte Bedeutung haben.

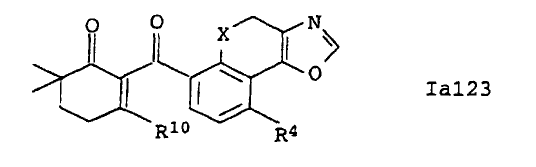

Ebenso insbesondere bevorzugt sind die Verbindungen der Formel Ia124 (≡ Ia mit R¹, R², R⁵ und R¹¹ bis R¹⁴ = H, R³, R¹⁵ und R¹⁶ = CH₃, m = 1), insbesondere die Verbindungen Ia124.n, wobei die Variablen X, R⁴ und R¹⁰ die in Tabelle 1 genannte Bedeutung haben.

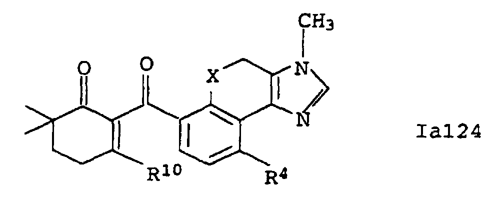

Ebenso insbesondere bevorzugt sind die Verbindungen der Formel Ia125 (≡ Ia mit R¹, R², R⁵ und R¹¹ bis R¹⁴ = H, R³, R¹⁵ und R¹⁶ = CH₃, m = 1), insbesondere die Verbindungen Ia125.n, wobei die Variablen X, R⁴ und R¹⁰ die in Tabelle 1 genannte Bedeutung haben.

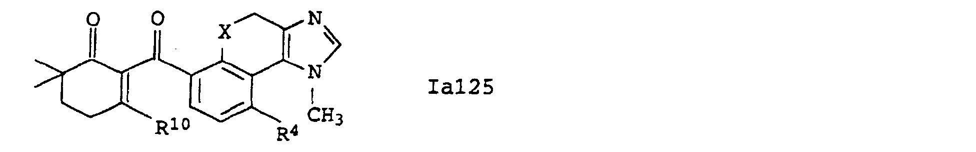

Ebenso insbesondere bevorzugt sind die Verbindungen der Formel Ia132 (≡ Ia mit R¹, R², R⁵ und R¹¹ bis R¹⁴ = H, R³, R¹⁵ und R¹⁶ = CH₃, m = 1), insbesondere die Verbindungen Ia132.n, wobei die Variablen X, R⁴ und R¹⁰ die in Tabelle 1 genannte Bedeutung haben.

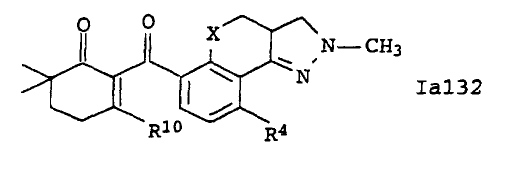

Ebenso insbesondere bevorzugt sind die Verbindungen der Formel Ia133 (≡ Ia mit R¹, R² und R⁵ = H, R¹¹, R¹², R¹⁵ und R¹⁶ = CH₃, R¹³ und R¹⁴ bilden gemeinsam mit dem Kohlenstoff an den sie gebunden sind eine Carbonylgruppe aus, m = 0), insbesondere die Verbindungen Ia133.n, wobei die Variablen X, R⁴ und R¹⁰ die in Tabelle I genannte Bedeutung haben.

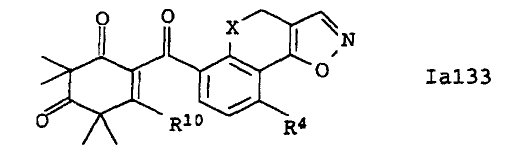

Ebenso insbesondere bevorzugt sind die Verbindungen der Formel Ia134 (≡ Ia mit R¹, R² und R⁵ = H, R³, R¹¹, R¹², R¹⁵ und R¹⁶ = CH₃, R¹³ und R¹⁴ bilden gemeinsam mit dem Kohlenstoff an den sie gebunden sind eine Carbonylgruppe aus, m = 1), insbesondere die Verbindungen Ia134.n, wobei die Variablen X, R⁴ und R¹⁰ die in Tabelle 1 genannte Bedeutung haben.

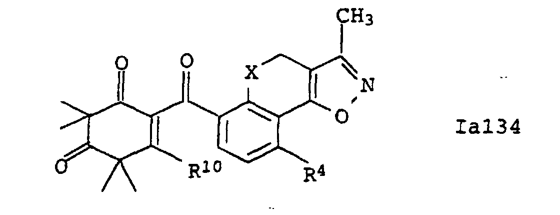

Ebenso insbesondere bevorzugt sind die Verbindungen der Formel Ia135 (≡ Ia mit R¹, R² und R⁵ = H, R³, R¹¹, R¹², R¹⁵ und R¹⁶ = CH₃, R¹³ und R¹⁴ bilden gemeinsam mit dem Kohlenstoff an den sie gebunden sind eine Carbonylgruppe aus, m = 1), insbesondere die Verbindungen Ia135.n, wobei die Variablen X, R⁴ und R¹⁰ die in Tabelle 1 genannte Bedeutung haben.

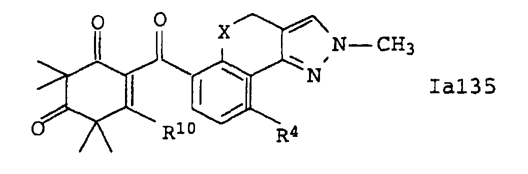

Ebenso insbesondere bevorzugt sind die Verbindungen der Formel Ia136 (≡ Ia mit R¹, R² und R⁵ = H, R³, R¹¹, R¹², R¹⁵ und R¹⁶ = CH₃, R¹³ und R¹⁴ bilden gemeinsam mit dem Kohlenstoff an den sie gebunden sind eine Carbonylgruppe aus, m = 2), insbesondere die Verbindungen Ia136.n, wobei die Variablen X, R⁴ und R¹⁰ die in Tabelle 1 genannte Bedeutung haben.

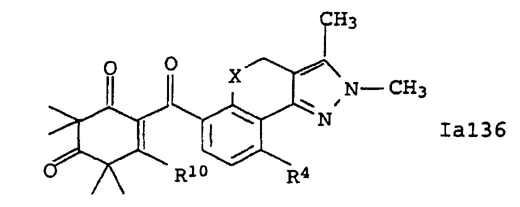

Ebenso insbesondere bevorzugt sind die Verbindungen der Formel Ia137 (≡ Ia mit R¹, R² und R⁵ = H, R³, R¹¹, R¹², R¹⁵ und R¹⁶ = CH₃, R¹³ und R¹⁴ bilden gemeinsam mit dem Kohlenstoff an den sie gebunden sind eine Carbonylgruppe aus, m = 1), insbesondere die Verbindungen Ia137.n, wobei die Variablen X, R⁴ und R¹⁰ die in Tabelle 1 genannte Bedeutung haben. 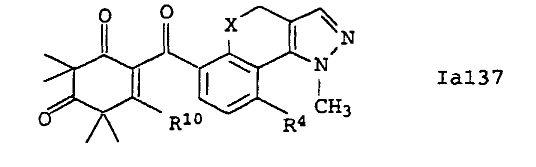 Ebenso insbesondere bevorzugt sind die Verbindungen der Formel Ia138 (≡ Ia mit R¹, R² und R⁵ = H, R³, R¹¹, R¹², R¹⁵ und R¹⁶ = CH₃, R¹³ und R¹⁴ bilden gemeinsam mit dem Kohlenstoff an den sie gebunden sind eine Carbonylgruppe aus, m = 2), insbesondere die Verbindungen Ia138.n, wobei die Variablen X, R⁴ und R¹⁰ die in Tabelle 1 genannte Bedeutung haben.

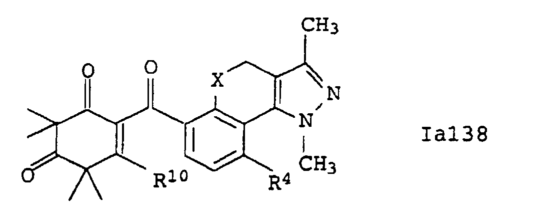

Ebenso insbesondere bevorzugt sind die Verbindungen der Formel Ia139 (≡ Ia mit R¹, R² und R⁵ = H, R³, R¹¹, R¹², R¹⁵ und R¹⁶ = CH₃, R¹³ und R¹⁴ bilden gemeinsam mit dem Kohlenstoff an den sie gebunden sind eine Carbonylgruppe aus, m = 0), insbesondere die Verbindungen Ia139.n, wobei die Variablen X, R⁴ und R¹⁰ die in Tabelle 1 genannte Bedeutung haben.

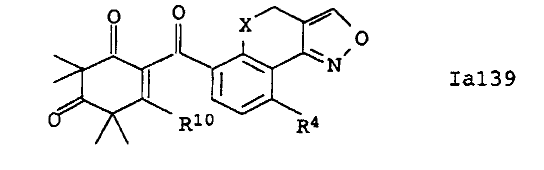

Ebenso insbesondere bevorzugt sind die Verbindungen der Formel Ia140 (≡ Ia mit R¹, R² und R⁵ = H, R³, R¹¹, R¹², R¹⁵ und R¹⁶ = CH₃, R¹³ und R¹⁴ bilden gemeinsam mit dem Kohlenstoff an den sie gebunden sind eine Carbonylgruppe aus, m = 1), insbesondere die Verbindungen Ia140.n, wobei die Variablen X, R⁴ und R¹⁰ die in Tabelle 1 genannte Bedeutung haben.

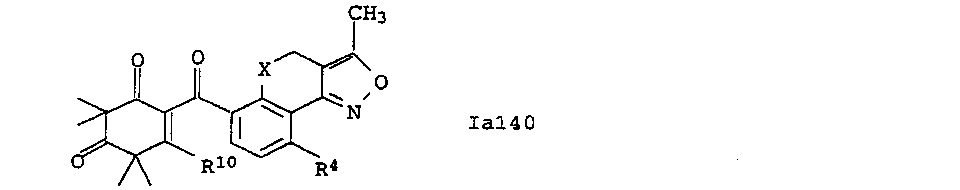

Ebenso insbesondere bevorzugt sind die Verbindungen der Formel Ia141 (≡ Ia mit R¹, R² und R⁵ = H, R³, R¹¹, R¹², R¹⁵ und R¹⁶ = CH₃, R¹³ und R¹⁴ bilden gemeinsam mit dem Kohlenstoff an den sie gebunden sind eine Carbonylgruppe aus, m = 0), insbesondere die Verbindungen Ia141.n, wobei die Variablen X, R⁴ und R¹⁰ die in Tabelle 1 genannte Bedeutung haben.

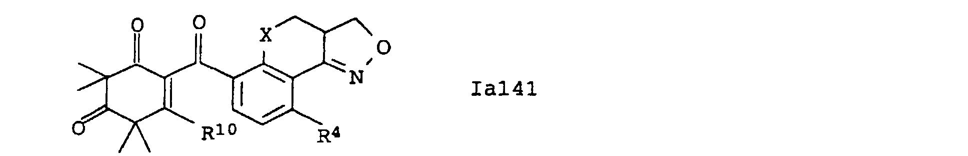

Ebenso insbesondere bevorzugt sind die Verbindungen der Formel Ia142 (≡ Ia mit R¹, R² und R⁵ = H, R³, R¹¹, R¹², R¹⁵ und R¹⁶ = CH₃, R¹³ und R¹⁴ bilden gemeinsam mit dem Kohlenstoff an den sie gebunden sind eine Carbonylgruppe aus, m = 0), insbesondere die Verbindungen Ia142.n, wobei die Variablen X, R⁴ und R¹⁰ die in Tabelle 1 genannte Bedeutung haben.

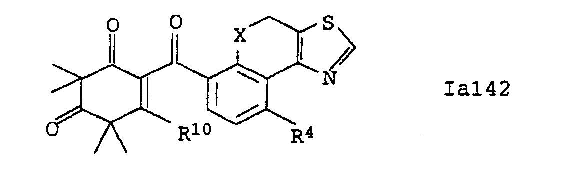

Ebenso insbesondere bevorzugt sind die Verbindungen der Formel Ia143 (≡ Ia mit R¹, R² und R⁵ = H, R³, R¹¹, R¹², R¹⁵ und R¹⁶ = CH₃, R¹³ und R¹⁴ bilden gemeinsam mit dem Kohlenstoff an den sie gebunden sind eine Carbonylgruppe aus, m = 0), insbesondere die Verbindungen Ia143.n, wobei die Variablen X, R⁴ und R¹⁰ die in Tabelle 1 genannte Bedeutung haben.

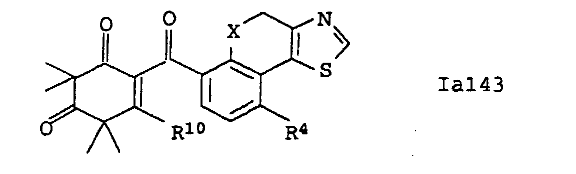

Ebenso insbesondere bevorzugt sind die Verbindungen der Formel Ia144 (≡ Ia mit R¹, R² und R⁵ = H, R³, R¹¹, R¹², R¹⁵ und R¹⁶ = CH₃, R¹³ und R¹⁴ bilden gemeinsam mit dem Kohlenstoff an den sie gebunden sind eine Carbonylgruppe aus, m = 0), insbesondere die Verbindungen Ia144.n, wobei die Variablen X, R⁴ und R¹⁰ die in Tabelle 1 genannte Bedeutung haben.

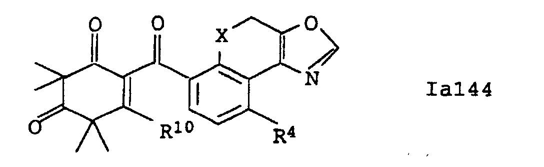

Ebenso insbesondere bevorzugt sind die Verbindungen der Formel Ia145 (≡ Ia mit R¹, R² und R⁵ = H, R³, R¹¹, R¹², R¹⁵ und R¹⁶ = CH₃, R¹³ und R¹⁴ bilden gemeinsam mit dem Kohlenstoff an den sie gebunden sind eine Carbonylgruppe aus, m = 0), insbesondere die Verbindungen Ia145.n, wobei die Variablen X, R⁴ und R¹⁰ die in Tabelle 1 genannte Bedeutung haben.

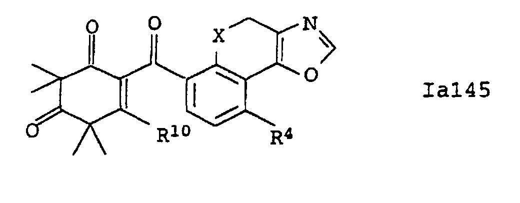

Ebenso insbesondere bevorzugt sind die Verbindungen der Formel Ia146 (≡ Ia mit R¹, R² und R⁵ = H, R³, R¹¹, R¹², R¹⁵ und R¹⁶ = CH₃, R¹³ und R¹⁴ bilden gemeinsam mit dem Kohlenstoff an den sie gebunden sind eine Carbonylgruppe aus, m = 1), insbesondere die Verbindungen Ia146.n, wobei die Variablen X, R⁴ und R¹⁰ die in Tabelle 1 genannte Bedeutung haben.

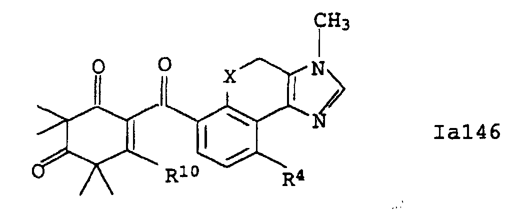

Ebenso insbesondere bevorzugt sind die Verbindungen der Formel Ia147 (≡ Ia mit R¹, R² und R⁵ = H, R³, R¹¹, R¹², R¹⁵ und R¹⁶ = CH₃, R¹³ und R¹⁴ bilden gemeinsam mit dem Kohlenstoff an den sie gebunden sind eine Carbonylgruppe aus, m = 1), insbesondere die Verbindungen Ia147.n, wobei die Variablen X, R⁴ und R¹⁰ die in Tabelle 1 genannte Bedeutung haben.

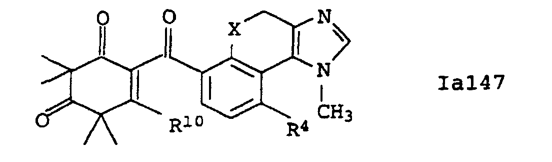

Ebenso insbesondere bevorzugt sind die Verbindungen der Formel Ia154 (≡ Ia mit R¹, R² und R⁵ = H, R³, R¹¹, R¹², R¹⁵ und R¹⁶ = CH₃, R¹³ und R¹⁴ bilden gemeinsam mit dem Kohlenstoff an den sie gebunden sind eine Carbonylgruppe aus, m = 1), insbesondere die Verbindungen Ia154.n, wobei die Variablen X, R⁴ und R¹⁰ die in Tabelle 1 genannte Bedeutung haben.

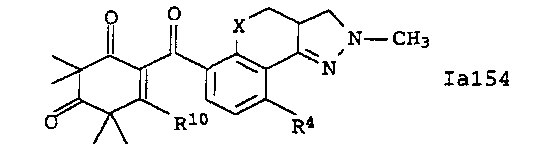

Ebenso insbesondere bevorzugt sind die Verbindungen der Formel Ia155 (≡ Ia mit R¹, R², R⁵ und R¹¹, R¹³ bis R¹⁵ = H, R¹² und R¹⁶ bilden gemeinsam eine -(CH₂)₂-Kette aus, m = 0), insbesondere die Verbindungen Ia 155.n, wobei die variablen X, R⁴ und R¹⁰ die in Tabelle I genannte Bedeutung haben.

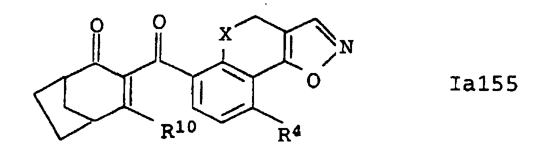

Ebenso insbesondere bevorzugt sind die Verbindungen der Formel Ia156 (≡ Ia mit R¹, R², R⁵ und R¹¹, R¹³ bis R¹⁵ = H, R¹² und R¹⁶ bilden gemeinsam eine -(CH₂)₂-Kette aus, m = 1), insbesondere die Verbindungen Ia 156.n, wobei die Variablen X, R⁴ und R¹⁰ die in Tabelle I genannte Bedeutung haben.

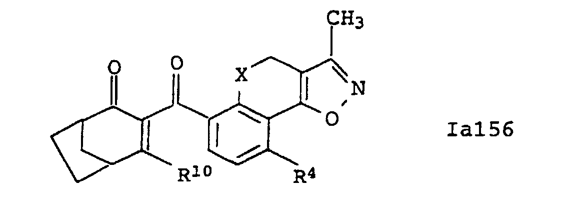

Ebenso insbesondere bevorzugt sind die Verbindungen der Formel Ia157 (≡ Ia mit R¹, R², R⁵ und R¹¹, R¹³ bis R¹⁵ = H, R¹² und R¹⁶ bilden gemeinsam eine -(CH₂)₂-Kette aus, m = 1), insbesondere die Verbindungen Ia 157.n, wobei die Variablen X, R⁴ und R¹⁰ die in Tabelle I genannte Bedeutung haben.

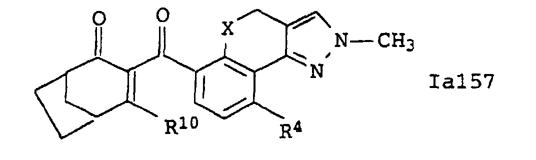

Ebenso insbesondere bevorzugt sind die Verbindungen der Formel Ia158 (≡ Ia mit R¹, R², R⁵ und R¹¹, R¹³ bis R¹⁵ = H, R¹² und R¹⁶ bilden gemeinsam eine -(CH₂)₂-Kette aus, m = 2), insbesondere die Verbindungen Ia 158.n, wobei die Variablen X, R⁴ und R¹⁰ die in Tabelle I genannte Bedeutung haben.

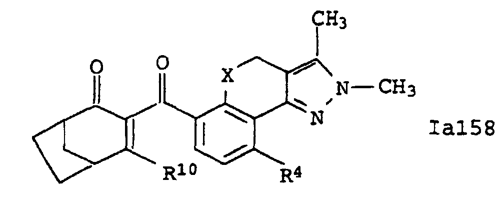

Ebenso insbesondere bevorzugt sind die Verbindungen der Formel Ia159 (≡ Ia mit R¹, R², R⁵ und R¹¹, R¹³ bis R¹⁵ = H, R¹² und R¹⁶ bilden gemeinsam eine -(CH₂)₂-Kette aus, m = 1), insbesondere die Verbindungen Ia 159.n, wobei die Variablen X, R⁴ und R¹⁰ die in Tabelle I genannte Bedeutung haben.

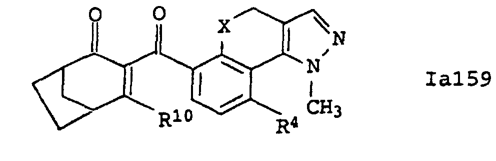

Ebenso insbesondere bevorzugt sind die Verbindungen der Formel Ia160 (≡ Ia mit R¹, R², R⁵ und R¹¹, R¹³ bis R¹⁵ = H, R¹² und R¹⁶ bilden gemeinsam eine -(CH₂)₂-Kette aus, m = 2), insbesondere die Verbindungen Ia 160.n, wobei die Variablen X, R⁴ und R¹⁰ die in Tabelle I genannte Bedeutung haben.

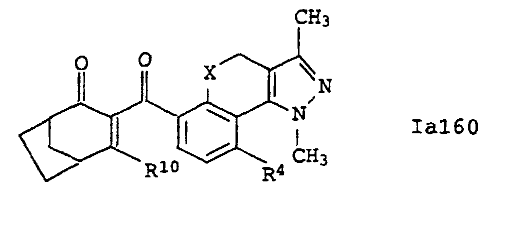

Ebenso insbesondere bevorzugt sind die Verbindungen der Formel Ia161 (≡ Ia mit R¹, R², R⁵ und R¹¹, R¹³ bis R¹⁵ = H, R¹² und R¹⁶ bilden gemeinsam eine - (CH₂)₂-Kette aus, m = 0), insbesondere die Verbindungen Ia 161.n, wobei die Variablen X, R⁴ und R¹⁰ die in Tabelle I genannte Bedeutung haben.

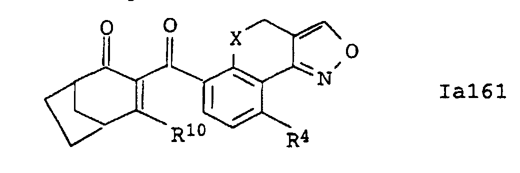

Ebenso insbesondere bevorzugt sind die Verbindungen der Formel Ia162 (≡ Ia mit R¹, R², R⁵ und R¹¹, R¹³ bis R¹⁵ = H, R¹² und R¹⁶ bilden gemeinsam eine - (CH2)2-Kette aus, m = 1), insbesondere die Verbindungen Ia 162.n, wobei die Variablen X, R⁴ und R¹⁰ die in Tabelle I genannte Bedeutung haben.

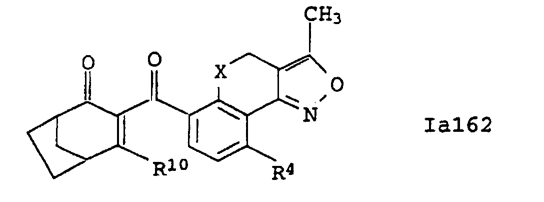

Ebenso insbesondere bevorzugt sind die Verbindungen der Formel Ia163 (≡ Ia mit R¹, R², R⁵ und R¹¹, R¹³ bis R¹⁵ = H, R¹² und R¹⁶ bilden gemeinsam eine - (CH2)2-Kette aus, m = 0), insbesondere die Verbindungen Ia 163.n, wobei die Variablen X, R⁴ und R¹⁰ die in Tabelle I genannte Bedeutung haben.

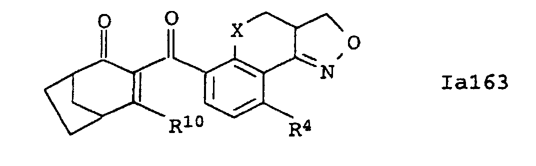

Ebenso insbesondere bevorzugt sind die Verbindungen der Formel Ia164 (≡ Ia mit R¹, R², R⁵ und R¹², R¹³ bis R¹⁵ = H, R¹² und R¹⁶ bilden gemeinsam eine -(CH₂)₂-Kette aus, m = 0), insbesondere die Verbindungen Ia 164.n, wobei die Variablen X, R⁴ und R¹⁰ die in Tabelle I genannte Bedeutung haben.

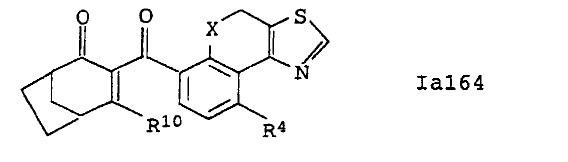

Ebenso insbesondere bevorzugt sind die Verbindungen der Formel Ia165 (≡ Ia mit R¹, R², R⁵ und R¹¹, R¹³ bis R¹⁵ = H, R¹² und R¹⁶ bilden gemeinsam eine -(CH₂)₂-Kette aus, m = 0), insbesondere die Verbindungen Ia 165.n, wobei die Variablen X, R⁴ und R¹⁰ die in Tabelle I genannte Bedeutung haben.

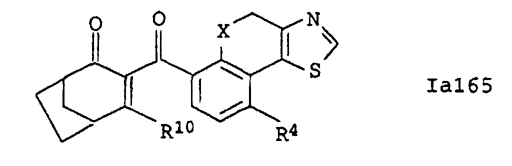

Ebenso insbesondere bevorzugt sind die Verbindungen der Formel Ia166 (≡ Ia mit R¹, R², R⁵ und R¹¹, R¹³ bis R¹⁵ = H, R¹² und R¹⁶ bilden gemeinsam eine -(CH₂)₂-Kette aus, m = 0), insbesondere die Verbindungen Ia 166.n, wobei die Variablen X, R⁴ und R¹⁰ die in Tabelle I genannte Bedeutung haben.

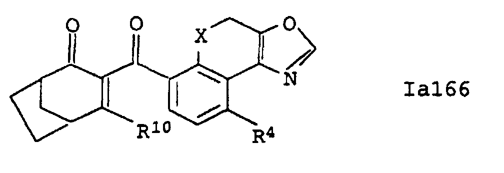

Ebenso insbesondere bevorzugt sind die Verbindungen der Formel Ia167 (≡ Ia mit R¹, R², R⁵ und R¹¹, R¹³ bis R¹⁵ = H, R¹² und R¹⁶ bilden gemeinsam eine -(CH₂)₂-Kette aus, m = 0), insbesondere die Verbindungen Ia 167.n, wobei die Variablen X, R⁴ und R¹⁰ die in Tabelle I genannte Bedeutung haben.

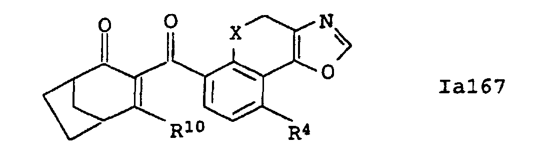

Ebenso insbesondere bevorzugt sind die Verbindungen der Formel Ia168 (≡ Ia mit R¹, R², R⁵ und R¹¹, R¹³ bis R¹⁵ = H, R¹² und R¹⁶ bilden gemeinsam eine -(CH₂)₂-Kette aus, m = 1), insbesondere die Verbindungen Ia 168.n, wobei die Variablen X, R⁴ und R¹⁰ die in Tabelle I genannte Bedeutung haben.

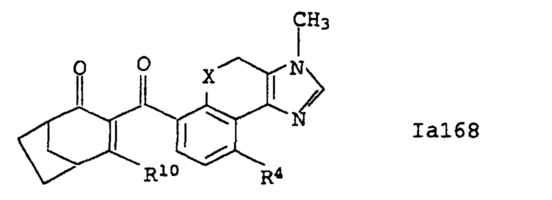

Ebenso insbesondere bevorzugt sind die Verbindungen der Formel Ia169 (≡ Ia mit R¹, R², R⁵ und R¹¹, R¹³ bis R¹⁵ = H, R¹² und R¹⁶ bilden gemeinsam eine - (CH₂)₂-Kette aus, m = 1), insbesondere die Verbindungen Ia 169.n, wobei die Variablen X, R⁴ und R¹⁰ die in Tabelle I genannte Bedeutung haben.

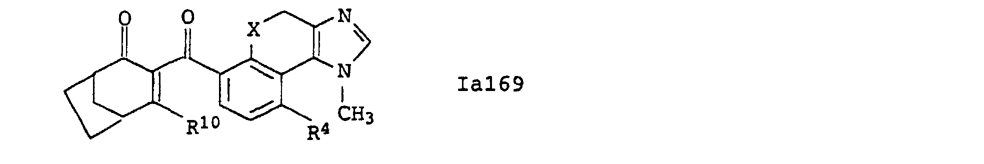

Ebenso insbesondere bevorzugt sind die Verbindungen der Formel Ia176 (≡ Ia mit R¹, R², R⁵ und R¹¹, R¹³ bis R¹⁵ = H, R¹² und R¹⁶ bilden gemeinsam eine -(CH₂)₂-Kette aus, m = 1), insbesondere die Verbindungen Ia 176.n, wobei die Variablen X, R⁴ und R¹⁰ die in Tabelle I genannte Bedeutung haben.

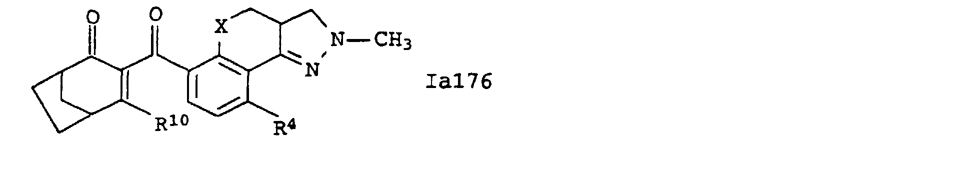

Die tricyclischen Benzoylcyclohexandion-Derivate der Formel I sind auf verschiedene Art und Weise erhältlich, beispielsweise nach folgenden Verfahren:

E. Darstellung von Verbindungen der Formel Iα (≡ I mit R¹⁰ = Hydroxy) durch Umsetzung einer aktivierten tricyclischen Benzoesäure der Formel VIα oder einer tricyclischen Benzoesäure VIβ, die vorzugsweise in situ aktiviert wird, mit einem Cyclohexandion der Formel V zu den Acylierungsprodukt VII und anschließende Umlagerung.

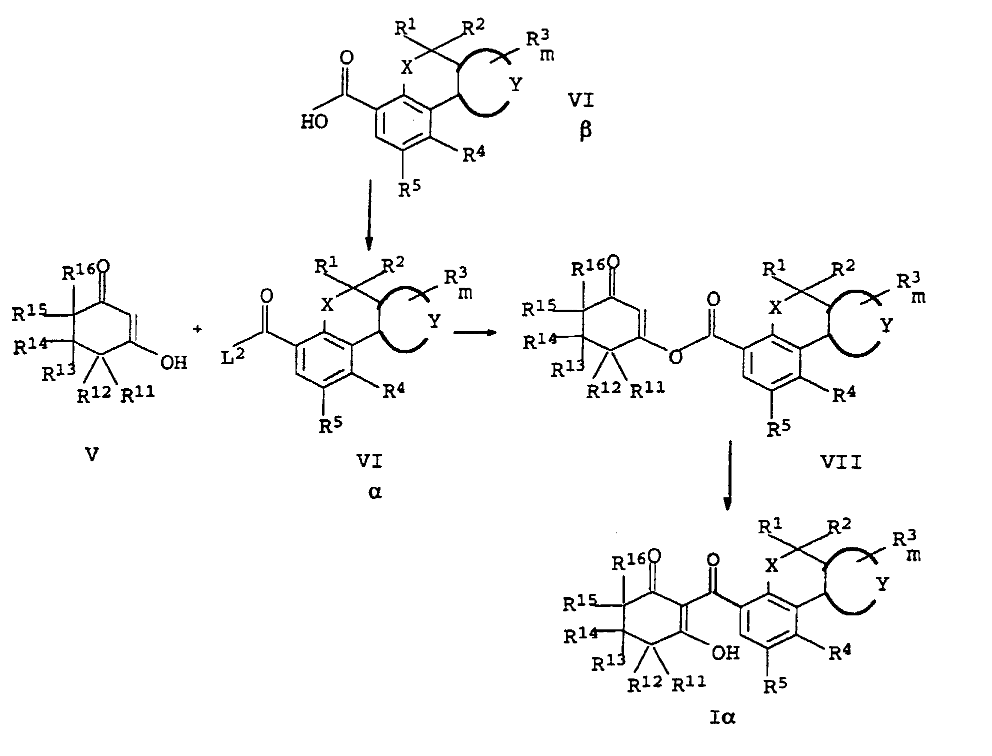

L² steht für eine nucleophil verdrängbare Abgangsgruppe, wie Halogen z.B. Brom oder Chlor, Hetaryl, z.B. Imidazolyl oder Pyridyl, Carboxylat, z.B. Acetat oder Trifluoracetat etc.

Die aktivierte tricyclische Benzoesäure VIα kann direkt eingesetzt werden, wie im Fall der tricyclischen Benzoylhalogenide oder in situ erzeugt werden, z.B. mit Dicyclohexylcarbodiimid, Triphenylphosphin/Azodicarbonsäureester, 2-Pyridindisulfid/Triphenylphosphin, Carbonyldiimidazol etc.

Gegebenenfalls kann es von Vorteil sein, die Acylierungsreaktion in Gegenwart einer Base auszuführen. Die Reaktanden und die Hilfsbase werden dabei zweckmäßigerweise in äquimolaren Mengen eingesetzt. Ein geringer Überschuß der Hilfsbase z.B. 1,2 bis 1,5 Moläquivalente, bezogen auf VI, kann unter Umständen vorteilhaft sein.

Als Hilfsbasen eignen sich tertiäre Alkylamine, Pyridin oder Alkalimetallcarbonate. Als Lösungsmittel können z.B. chlorierte Kohlenwasserstoffe, wie Methylenchlorid oder 1,2-Dichlorethan, aromatische Kohlenwasserstoffe, wie Toluol, Xylol oder Chlorbenzol, Ether, wie Diethylether, Methyl-tert.-butylether, Tetrahydrofuran oder Dioxan, polare aprotische Lösungsmittel, wie Acetonitril, Dimethylformamid oder Dimethylsulfoxid oder Ester wie Essigsäureethylester oder Gemische hiervon verwendet werden.

Werden tricyclische Benzoylhalogenide als aktivierte Carbonsäurekomponente eingesetzt, so kann es zweckmäßig sein, bei Zugabe dieses Reaktionspartners die Reaktionsmischung auf 0-10°C abzukühlen. Anschließend rührt man bei 20 - 100°C, vorzugsweise bei 25 - 50°C, bis die Umsetzung vollständig ist. Die Aufarbeitung erfolgt in üblicher Weise, z.B. wird das Reaktionsgemisch auf Wasser gegossen, das Wertprodukt extrahiert. Als Lösungsmittel eignen sich hierfür besonders Methylenchlorid, Diethylether und Essigsäureethylester. Nach Trocknen der organischen Phase und Entfernen des Lösungsmittels kann der rohe Ester ohne weitere Reinigung zur Umlagerung eingesetzt werden.

Die Umlagerung der Ester VII zu den Verbindungen der Formel Iα erfolgt zweckmäßigerweise bei Temperaturen von 20 bis 100°C in einem Lösungsmittel und in Gegenwart einer Base sowie gegebenenfalls mit Hilfe einer Cyanoverbindung als Katalysator.

Als Lösungsmittel können z.B. Acetonitril, Methylenchlorid, 1,2-Dichlorethan, Dioxan, Essigsäureethylester, Toluol oder Gemische hiervon verwendet werden. Bevorzugte Lösungsmittel sind Acetonitril und Dioxan.

Geeignete Basen sind tertiäre Amine wie Triethylamin, aromatische Amine wie Pyridin oder Alkalicarbonate, wie Natriumcarbonat oder Kaliumcarbonat, die vorzugsweise in äquimolarer Menge oder bis zu einem vierfachen Überschuß, bezogen auf den Ester, eingesetzt werden. Bevorzugt werden Triethylamin oder Alkalicarbonat verwendet, vorzugsweise in doppelt äquimolarem Verhältnis in Bezug auf den Ester.

Als Cyanoverbindungen kommen anorganische Cyanide, wie Natriumcyanid oder Kaliumcyanid und organische Cyanoverbindungen, wie Acetoncyanhydrin oder Trimethylsilylcyanid in Betracht. Sie werden in einer Menge von 1 bis 50 Molprozent, bezogen auf den Ester, eingesetzt. Vorzugsweise werden Acetoncyanhydrin oder Trimethylsilylcyanid, z.B. in einer Menge von 5 bis 15, vorzugsweise 10 Molprozent, bezogen auf den Ester, eingesetzt.

Die Aufarbeitung kann in an sich bekannter weise erfolgen. Das Reaktionsgemisch wird z.B. mit verdünnter Mineralsäure, wie 5 %ige Salzsäure oder Schwefelsäure, angesäuert, mit einem organischen Lösungsmittel, z.B. Methylenchlorid oder Essigsäureethylester extrahiert. Der organische Extrakt kann mit 5-10%iger Alkalicarbonatlösung, z.B. Natriumcarbonat- oder Kaliumcarbonatlösung extrahiert werden. Die wäßrige Phase wird angesäuert und der sich bildende Niederschlag abgesaugt und/oder mit Methylenchlorid oder Essigsäureethylester extrahiert, getrocknet und eingeengt.

Die tricyclischen Benzoylhalogenide der Formel VIα mit L² = Cl, Br können auf an sich bekannte Art und Weise durch Umsetzung der tricyclischen Benzoesäuren der Formel VIß (≡ VIb) mit Halogenierungsreagentien wie Thionylchlorid, Thionylbromid, Phosgen, Diphosgen, Triphosgen, Oxalylchlorid, Oxalylbromid hergestellt werden.

Die tricyclischen Benzoesäuren der Formel VIß (≡VIb) können in bekannter Weise durch saure oder basische Hydrolyse aus den entsprechenden Estern VIc hergestellt werden.

Tricyclische Benzoesäurederivate der Formel VI, die ebenfalls Gegenstand der Erfindung sind, sind neu, 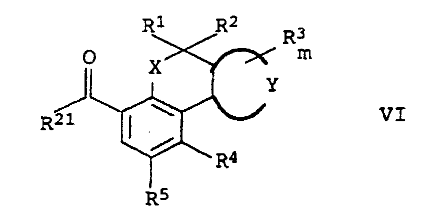 wobei die variablen folgende Bedeutung haben:
- X: Sauerstoff, Schwefel, S=O oder S(=O)₂;
- Y: bildet gemeinsam mit den beiden Kohlenstoffen, an die es gebunden ist, einen gesättigten, partiell gesättigten oder ungesättigten 5-gliedrigen Heterocyclus, der ein bis drei gleiche oder verschiedene Heteroatome, ausgewählt aus folgender Gruppe: Sauerstoff, Schwefel oder Stickstoff, enthält;
- R¹, R²: Wasserstoff, C₁-C₆-Alkyl, C₁-C₆-Halogenalkyl, C₁-C₆-Alkoxy oder C₁-C₆-Halogenalkoxy;
- R³: Halogen, C₁-C₆-Alkyl, C₁-C₆-Halogenalkyl, C₁-C₆-Alkoxy oder C₁-C₆-Halogenalkoxy;
- R⁴: Nitro, Halogen, Cyano, C₁-C₆-Alkyl, C₁-C₆-Halogenalkyl, C₁-C₆-Alkoxy, C₁-C₆-Halogenalkoxy,. C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, Aminosulfonyl, N-(C₁-C₆-Alkyl)-aminosulfonyl, N,N-Di-(C₁-C₆-alkyl)-aminosulfonyl, N-(C₁-C₆-Alkylsulfonyl)-amino, N-(C₁-C₆-Halogenalkylsulfonyl)-amino, N-(C₁-C₆-Alkyl)-N-(C₁-C₆-alkylsulfonyl)-amino oder N-(C₁-C₆-Alky)-N-(C₁-C₆-halogenalkylsulfonyl)-amino;
- R⁵: Wasserstoff, C₁-C₆-Alkyl oder Halogen;
- m: 0, 1 oder 2;
- R²¹: Hydroxy oder ein abhydrolysierbarer Rest;

Beispiele für abhydrolysierbare Reste sind Alkoxy-, Phenoxy-, Alkylthio-, Phenylthio-Reste, die ggf. substituiert sein können, Halogenide, Heteroaryl-Reste, die über Stickstoff gebunden sind, Amino-, Imino-Reste, die ggf. substituiert sein können etc.

Bevorzugt sind tricyclische Benzoesäurehalogenide VIa (VI mit R²¹ = Halogen) 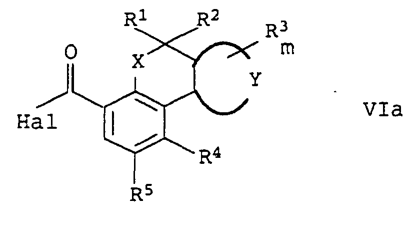 wobei die Variablen X, Y, R¹ bis R⁵ und m die unter Formel VI genannte Bedeutung haben und
- Hal: Halogen, insbesondere Chlorid oder Bromid, bedeutet.

Ebenso bevorzugt sind tricyclische Benzosäuren der Formel VIb (VI mit R¹⁷ = Hydroxy; ≡ VIγ), 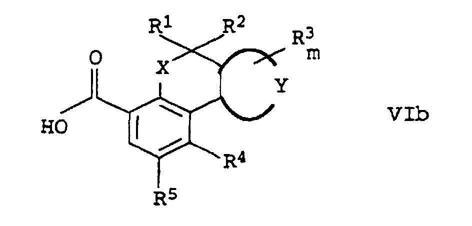 wobei die Variablen X, Y, R¹ bis R⁵ und m die unter Formel VI genannte Bedeutung haben.

Ebenso bevorzugt sind tricyclische Benzosäureester der Formel VIc (VI mit R²¹ = T = C₁-C₆-Alkoxy), 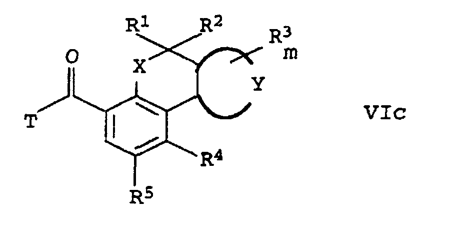 wobei die Variablen X, Y, R¹ bis R⁵ und m die unter Formel VI genannte Bedeutung haben und
- T: C₁-C₆-Alkoxy bedeutet.

Die besonders bevorzugten Ausführungsformen der tricyclischen Benzoesäure-Derivate der Formel VI, VIa, VIb und VIc in Bezug auf die variablen X, Y, R¹ bis R⁵ und m entsprechen denen der tricyclischen Benzoylcyclohexandion-Derivate der Formel I.

Insbesondere bevorzugt sind die Verbindungen VI, VIa, VIb und VIc, wobei Y gemeinsam mit den beiden Kohlenstoffen, an die es gebunden ist, folgende Heterocyclen ausbildet:

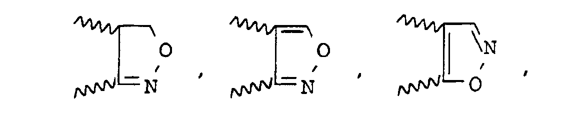

Hierbei sind Verbindungen VI, VIa, VIb und VIc außerordentlich bevorzugt, wobei
- R⁴: Nitro, Halogen, C₁-C₆-Alkyl, C₁-C₆-Halogenalkyl, C₁-C₆-Alkoxy, C₁-C₆-Alkythio oder C₁-C₆-Alkylsulfonyl; insbesondere C₁-C₆-Alkylksulfonyl;
bedeutet.

Die tricyclischen Benzoesäureester VIc sind auf verschiedene Art und Weise erhältlich.

Beispielsweise können Benzosäureester der Formel VIII, die auf an sich bekannte Art und Weise hergestellt werden (vgl. z.B. Chem. Pharm. Bull. 1985, 33 (8), 3336; Helv. Chim. Acta 1987, 70, 1326; J. Chem. Soc. Perkin Trans. 1972, 2019; J. Chem. Soc. Perkin Trans. 1991, 2763; Tetrahydron Asymmetry 1998, 9, 1137), zu cyclischen Ketonen der Formel IX cyclisiert werden (vgl. z.B. Chem. Ber. 1923, 56, 1819; J. Chem. Soc. Perkin I 1991, 2763; J. Med. Chem. 1988, 31, 230; Tetrahedron 1987, 43, 4549; Synlett 1991, 6, 443; Chem. Pharm. Bull. 1985, 33 (8), 3336). Diese lassen sich in Analogie zu bekannten Verfahren (vgl. z.B. J. Heterocyclic Chem. 1976, 13, 545; J. Heterocyclic Chem. 1972, 9, 1341; J. Org. Chem. 1978, 43, 3015; J. Chem. Soc. Perkin Trans. I 1978, 86; J. Org. Chem. 1986, 51, 2021) in die tricyclischen Benzoesäureester der Formel VIc überführen.

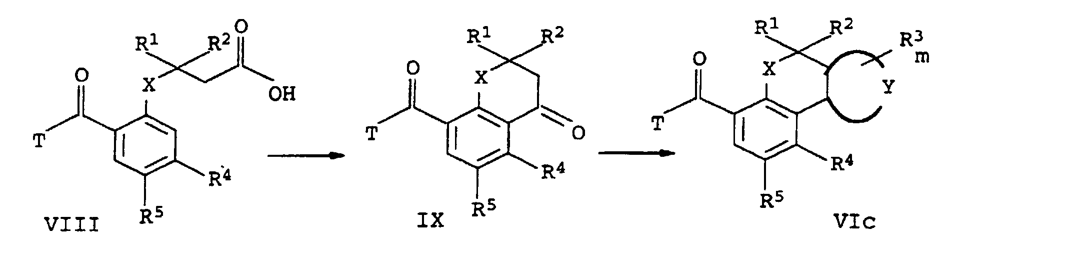

Weiterhin kann es in Betracht kommen das cyclische Keton der Formel IX auf an sich bekannte Weise zu acetylieren (X), z.B. mit einem Anhydrid oder Säureanhydrid ggf. in Gegenwart von katalytischen Mengen einer Lewissäure, wie Bortrifluorid (vgl. z.B. Can. J. Chem. 1979, 57, 3292; J. Am. Chem. Soc. 1953, 75, 626) und anschließend mit einem Hydrazin umzusetzen (vgl. A.R. Katritzky et al., Comprehensive Heterocyclic Chemistry, Vol. 5, p. 121, 277 - 280 (1984), Pergamon Press; J. Org. Chem. 1961, 26, 451; Org. Synth. 1949, 29, 54), wobei der resultierende Pyrazolrest nach üblichen Verfahren weiter modifiziert werden kann.

Weiterhin kann das Diketon X mit Hydroxylamin oder Äquivalenten hiervon umgesetzt werden (vgl. A.R. Katritzky et al., Comprehensive Heterocyclic Chemistry, Vol. 6, p. 61 - 64, 118 (1984), Pergamon Press; Chem. Ber. 1967, 100, 3326). Man erhält entsprechende Isoxazolderivate, die nach üblichen Verfahren weiter modifiziert werden können.

Ebenso ist es möglich, das Diketon X mit Amidinen umzusetzen (vgl. z.B. A.R. Katritzky et al., Comprehensive Heterocyclic Chemistry, Vol. 3, p. 112 - 114 (1924), Pergamon Press; J. Chem. Soc. C 1967, 1922; Org. Synth. 1963, IV, 182). Die so erhaltenen Pyrimidinderivate können bei Bedarf nach den üblichen verfahren weiter modifiziert werden.

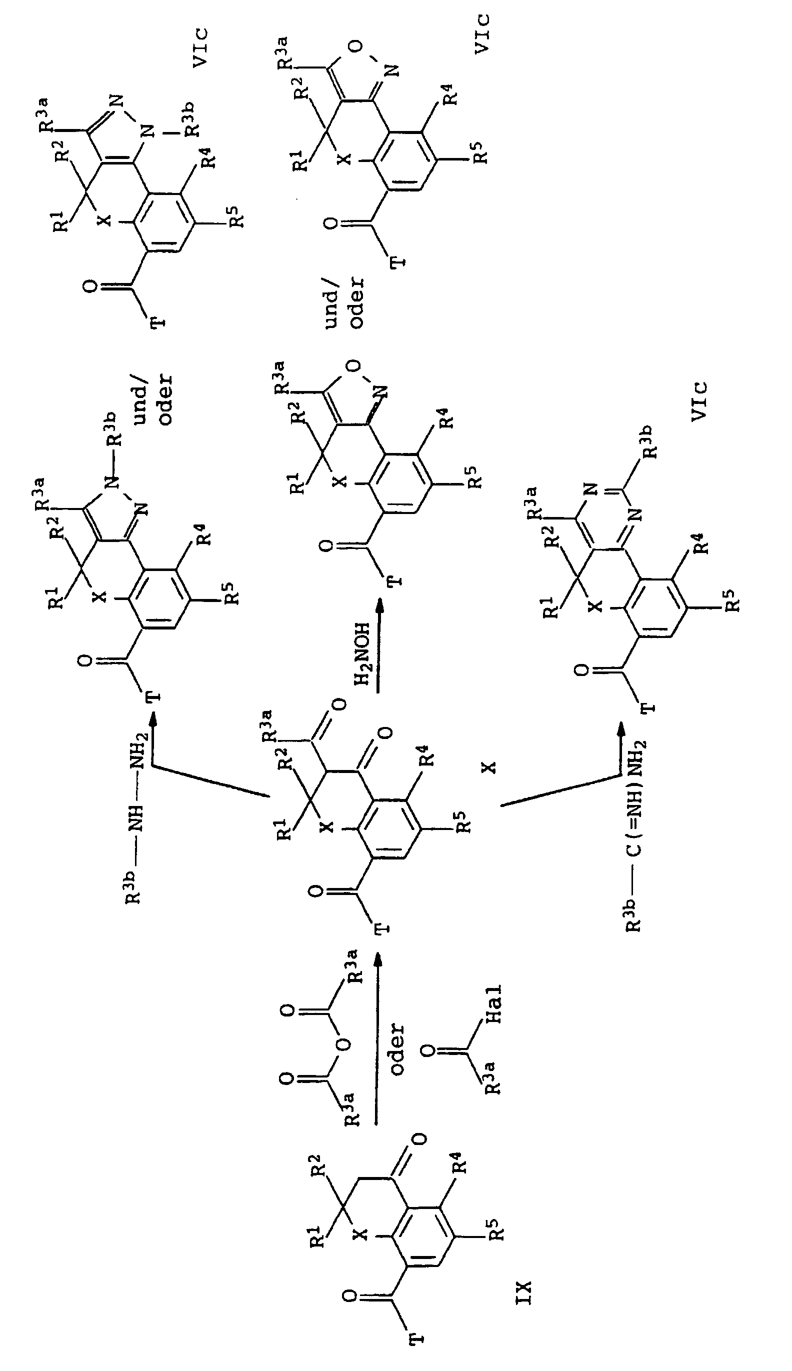

Es ist auch möglich bei den voranstehend genannten Reaktionen anstelle des Diketons X Äquivalente hiervon, wie Enolether oder Enamine, die in Analogie zu bekannten Verfahren dargestellt werden können, einzusetzen.

Es kann auch in Betracht kommen das cyclische Keton der Formel IX in Analogie zu bekannten Verfahren mit einen Aldehyd oder Keton umzusetzen (XI) (vgl. z.B. Tetrahedron Lett. 1978, 2111; Tetrahedron Lett. 1981, 5251; Chem. Ber. 1960, 2294; J. Chem. Soc. Perkin Trans. 1, 1991, 1467; Tetrahedron Lett. 1992, 8091). Das resultierende ungesättigte cyclische Keton der Formel XI kann auf an sich bekannte Weise (vgl. z.B. A.R. Katritzky et al. Comprehensive Heterocyclic Chemistry, Vol. 2, 6 (1984), Pergamon Press; J. Heterocyclic Chem. 1969, 533; J. Heterocyclic Chem. 1968, 853) mit einem Hydrazin umgesetzt werden, wobei das resultierende Pyrazolin nach üblichen Verfahren weiter modifiziert werden kann.

Weiterhin kann das ungesättigte cyclische Keton der Formel XI mit Hydroxylamin oder Äquivalenten hiervon umgesetzt werden (Z. Chem. 1980, 20, 19). Man erhält entsprechende Isoxazolinderivate, die nach üblichen Verfahren weiter modifiziert werden können.

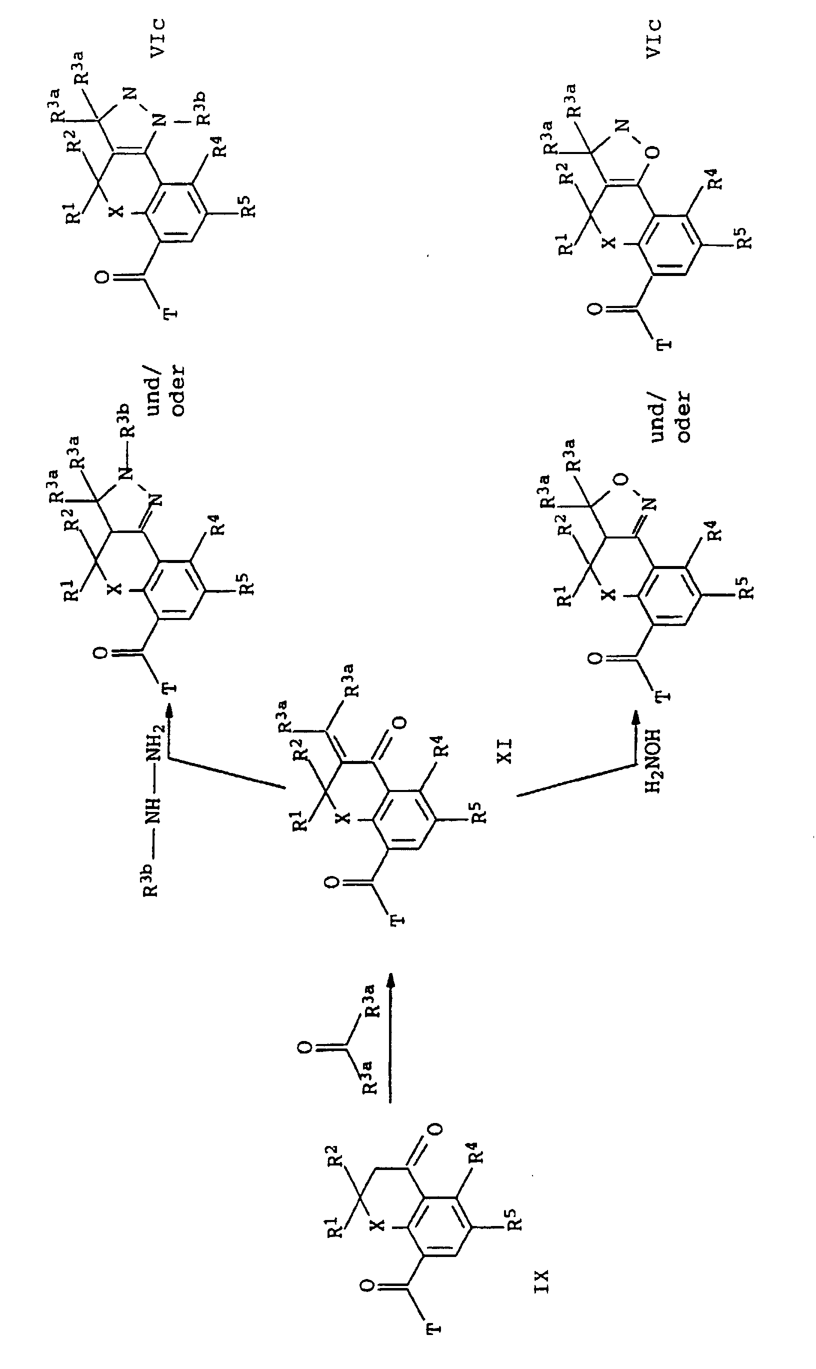

Weiterhin können Aldehyde der Formel XII, die auf an sich bekannte Art und Weise hergestellt werden können, in Analogie zu literaturbekannten Verfahren durch Umsetzung mit einem Hydrazin oder Hydroxylamin (oder Äquivalenten hiervon) in entsprechende Hydrazone oder Oxime überführt werden (vgl. z.B. Synth. Commun. 1990, 20, 1373; J. Org. Chem. 1980, 45, 3756). Diese können wiederum auf an sich bekannte Art und Weise in die entsprechenden 1,3-Dipole überführt werden, die dann im Rahmen einer [3 + 2]-Cycloaddition zu den Verbindungen VIc abreagieren (vgl. z.B. Synth. Commun. 1990, 20, 1373; EP-A 386 892; J. Org. Chem. 1980, 45, 3756; Tetrahedron Lett. 1981, 22, 1333.)

Die so erhaltenen Pyrazole bzw. Pyrazoline sowie Isoxazole bzw. Isoxazoline können nach üblichen Verfahren weiter modifiziert werden.

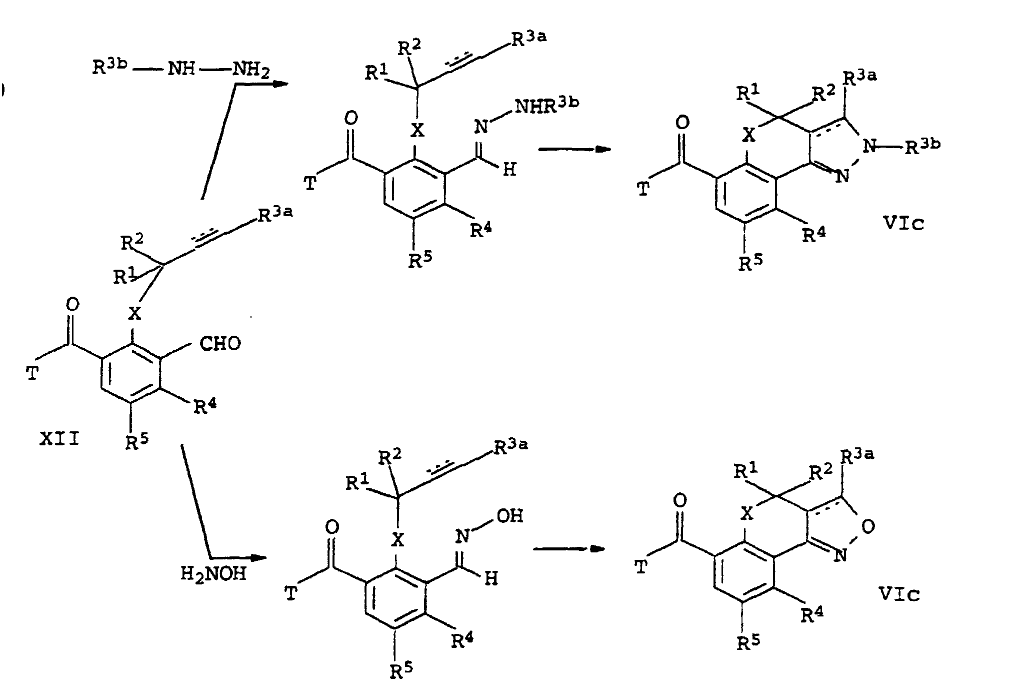

Ebenso ist es möglich das cyclische Keton der Formel IX mit einen Dithiol oder einem "gemischten Alkohol" in Analogie zu literaturbekannten Verfahren (vgl. z.B. T.W. Greene et al., Protective Groups in Organic Synthesis, John Wiley & Sons, 133-140) umzusetzen und anschließend einer Umlagerung in Gegenwart von Brom oder einer geeigneten Lewisssäure wie z.B. Tellurtetrachlorid zu unterwerfen (vgl. Tetrahedron 1991, 47, 4187; Synthesis 1991, 223; J. Chem. Am. Soc. Chem. Commun. 1985, 1645).

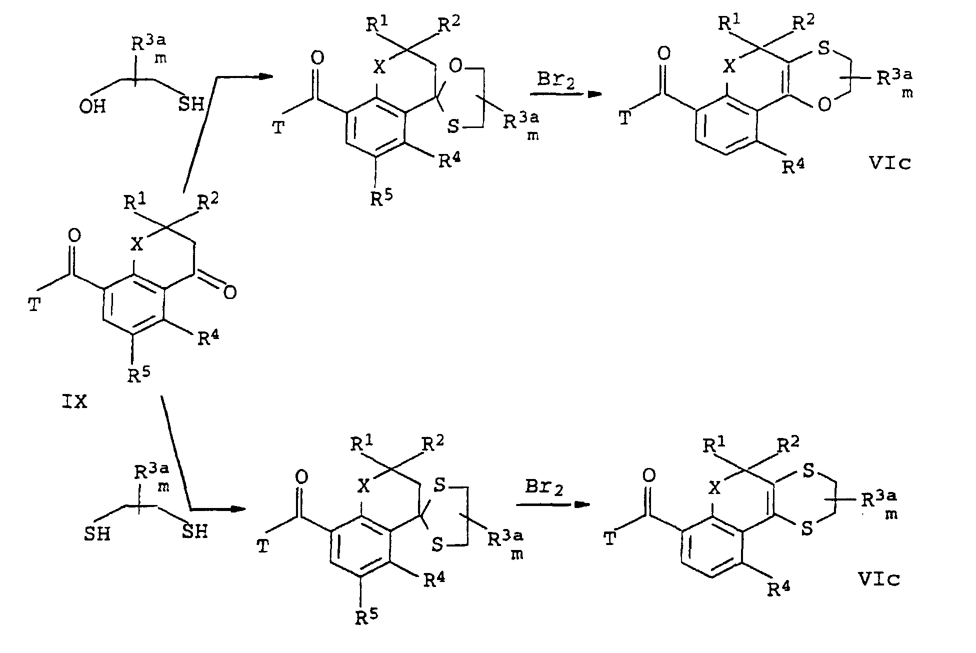

Die so erhaltenen Heterocyclen können nach an sich bekannten Verfahren ggf. weiter modifiziert werden.

Die vorstehend genannten Substituenten R^{3a} stehen für Wasserstoff, C₁-C₆-Alkyl, C₁-C₆-Halogenalkyl, Hydroxy, C₁-C₆-Alkoxy oder C₁-C₆-Halogenalkoxy; weiterhin stehen die voranstehend genannten Reste R^{3b} für Wasserstoff, C₁-C₆-Alkyl oder C₁-C₆-Halogenalkyl.

Die tricyclischen Benzoesäureester der Formel VIc bzw. die tricyclischen Benzoesäuren der Formel VIb können durch Umsetzung eines tricyclischen Benzolderivats der Formel XIII mit einem C₁-C₆-Alkohol bzw. Wasser in Gegenwart von Kohlenmonoxid, eines Katalysators sowie eine Base erhalten werden.

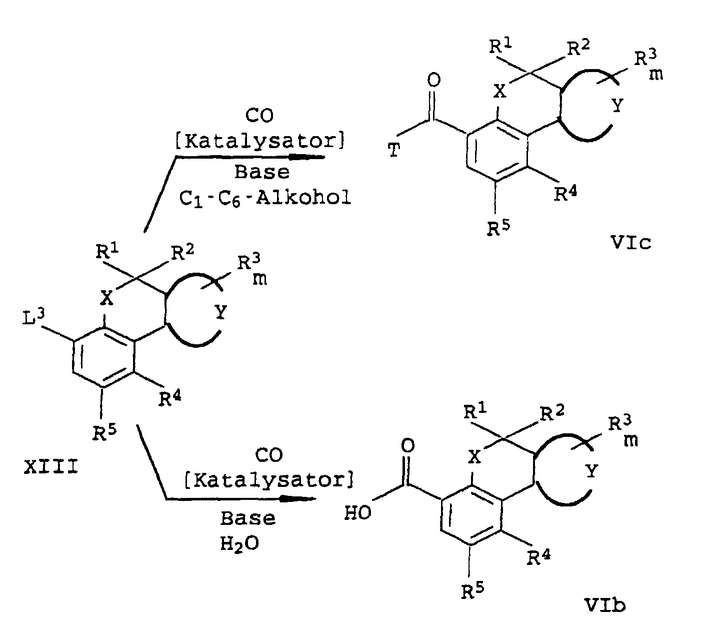

L³ steht hierbei für eine Abgangsgruppe wie Halogen, z.B. Chlor, Brom oder Iod, oder Sulfat wie Mesylat oder Triflat; bevorzugt sind Brom oder Triflat.

Als Katalysatoren eignen sich Palladiumligandkomplexe, in denen das Palladium in der Oxidationsstufe 0 vorliegt, metallisches Palladium, das gegebenenfalls auf einen Träger aufgezogen wurde, und vorzugsweise Palladium(II)salze. Die Umsetzung mit Palladium(II)salzen und metallischem Palladium wird vorzugsweise in Gegenwart von Komplexliganden durchgeführt.

Als Palladium(0)ligandkomplex kommt beispielsweise Tetrakis(triphenylphosphan)palladium in Frage.

Metallisches Palladium ist vorzugsweise auf einen inerten Träger wie beispielsweise Aktivkohle, Siliciumdioxid, Aluminiumoxid, Bariumsulfat oder Calciumcarbonat aufgezogen. Die Reaktion wird vorzugsweise in Gegenwart von Komplexliganden wie beispielsweise Triphenylphosphan durchgeführt.

Als Palladium(II)salze eigenen sich beispielsweise Palladiumacetat und Palladiumchlorid. Bevorzugt wird in Gegenwart von Komplexliganden wie beispielsweise Triphenylphospha- gearbeitet.

Geeignete Komplexliganden für die Palladiumligandkomplexe, bzw. in deren Gegenwart die Umsetzung mit metallischem Palladium oder Palladium(II)salzen vorzugsweise ausgeführt wird, sind tertiäre Phosphane, deren Struktur durch folgende Formeln wiedergegeben wird: 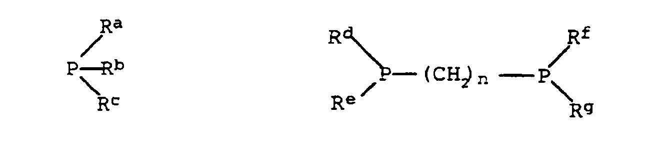 wobei n die Zahlen 1 bis 4 bedeutet und die Reste R^{a} bis R^{g} für C₁-C₆-Alkyl, Aryl-C₁-C₂-alkyl oder vorzugsweise Aryl stehen. Aryl steht beispielsweise für Naphthyl und gegebenenfalls substituiertes Phenyl wie beispielsweise 2-Tolyl und insbesondere für unsubstituiertes Phenyl.

Die Herstellung der komplexen Palladiumsalze kann in an sich bekannter Weise ausgehend von kommerziell erhältlichen Palladiumsalzen wie Palladiumchlorid oder Palladiumacetat und den entsprechenden Phosphanen wie z.B. Triphenylphosphan oder 1,2-Bis(diphenylphosphano)ethan erfolgen. Ein Großteil der komplexierten Palladiumsalze ist auch kommerziell erhältlich. Bevorzugte Palladiumsalze sind [(R)(+)2,2'-Bis(diphenylphosphano)-1,1'-binaphthyl]palladium(II)chlorid, Bis(triphenylphosphan)palladium(II)acetat und insbesondere Bis(triphenylphosphan)palladium(II)chlorid.

Der Palladiumkatalysator wird in der Regel in einer Konzentration von 0,05 bis 5 Mol%, und bevorzugt 1-3 Mol% eingesetzt.

Als Basen kommen tertiäre Amine wie beispielsweise N-Methylpiperidin, Ethyldiisopropylamin, 1,8-Bisdimethylaminonaphthalin oder insbesondere Triethylamin in Betracht. Ebenso eigenen sich Alkalicarbonat, wie Natriumcarbonat oder Kaliumcarbonat. Aber auch Gemische von Kaliumcarbonat und Triethylamin sind geeignet.

In der Regel werden 2 bis 4 Moläquivalente, insbesondere 2 Moläquivalente, des Alkalicarbonats, sowie 1 bis 4 Moläquivalente, insbesondere 2 Moläquivalente des tertiären Amins bezogen auf das tricyclische Benzolderivat der Formel IX eingesetzt.

Als Lösungsmittel können Nitrile wie Benzonitril und Acetonitril, Amide wie Dimethylformamid, Dimethylacetamid, Tetra-C₁-C₄-alkylharnstoffe oder N-Methylpyrrolidon und vorzugsweise Ether wie Tetrahydrofuran, Methyl-tert.-butylether dienen. Insbesondere werden Ether wie 1,4-Dioxan und Dimethoxyethan als Lösungsmittel bevorzugt.

Weiterhin können die tricyclischen Benzoesäuren der Formel VIb erhalten werden, indem man ein tricyclisches Benzolderivat der Formel XIII mit L³ Halogen wie Chlor oder Brom, insbesondere Brom, durch Umsetzung mit beispielsweise n-Butyllithium oder Magnesium in das metallierte Derivat überführt und anschließend mit Kohlendioxid quencht (vgl. z.B. J. Org. Chem. 1990, 55, 773; Angew. Chem. Int. Ed. 1969, 8, 68).

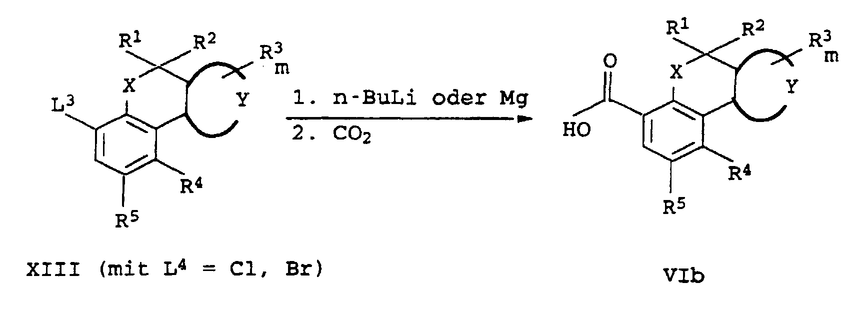

Ebenso können die tricyclischen Benzoesäuren VIb durch Verseifung der entsprechenden Nitrile in Analogie zu literaturbekannten Verfahren erhalten werden. Die Nitrile wiederum können durch Halogen/Nitril-Austausch erhalten werden oder durch Sandmeyer-Reaktion aus den entsprechenden Anilinen XIV.

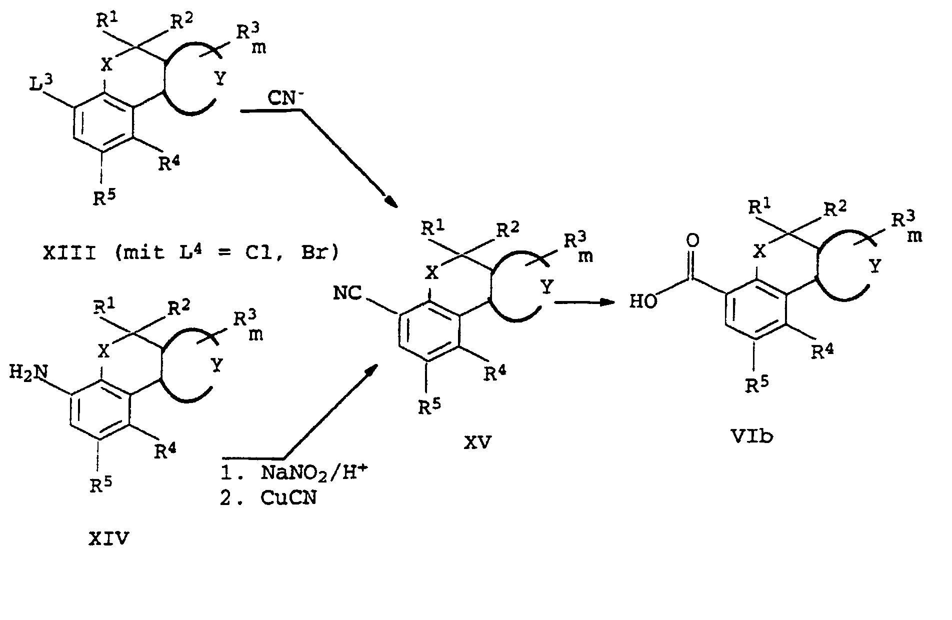

Die Verbindungen der Formel XIII, die ebenfalls Gegenstand der vorliegenden Erfindung sind, sind neu,
wobei die Variablen folgende Bedeutung haben:
- X: Sauerstoff, Schwefel, S=O oder S(=O)₂
- Y: bildet gemeinsam mit den beiden Kohlenstoffen, an die es gebunden ist, einen gesättigten, partiell gesättigten oder ungesättigten 5-gliedrigen Heterocyclus, der ein bis drei gleiche oder verschiedene Heteroatome, ausgewählt aus folgender Gruppe: Sauerstoff, Schwefel oder Stickstoff, enthält;
- R¹,R²: Wasserstoff, C₁-C₆-Alkyl, C₁-C₆-Halogenalkyl, C₁-C₆-Alkoxy oder C₁-C₆-Halogenalkoxy;
- R³: Halogen, C₁-C₆-Alkyl, C₁-C₆-Halogenalkyl, C₁-C₆-Alkoxy oder C₁-C₆-Halogenalkoxy;
- R⁴: Nitro, Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogrenalkylsulfonyl, Aminosulfonyl, N-(C₁-C₆-Alkyl)-aminosulfonyl, N,N-Di-(C₁-C₆-alkyl)-aminosulfonyl, N-(C₁-C₆-Alkylsulfonyl)-amino, N-(C₁-C₆-Halogenalkylsulfonyl)-amino, N-(C₁-C₆-Alkyl)-N-(C₁-C₆-alkylsulfonyl)-amino oder N-(C₁-C₆-Alky)-N-(C₁-C₆-halogenalkylsulfonyl)-amino;
- R⁵: Wasserstoff, C₁-C₆-Alkyl oder Halogen;
- m: 0, 1 oder 2;
- L³: Halogen, C₁-C₆-Alkylsulfonyloxy, C₁-C₆-Halogenalkylsulfonyoxy oder Phenylsulfonyloxy, wobei der Phenylring des letztgenannten Rests unsubstituiert sein kann oder partiell oder vollständig halogeniert und/oder einen bis drei der folgenden Reste tragen kann: Nitro, Cyano, C₁-C₆-Alkyl, C₁-C₆-Halogenalkyl, C₁-C₄-Alkoxy- oder C₂-C₄-Halogenalkoxy;

Bevorzugt sind Verbindungen der Formel XIII, wobei L³ für Halogen, insbesondere Brom steht.

Die besonders bevorzugten Ausführungsformen der Verbindungen der Formel XIII in Bezug auf die Variablen X, Y, R¹ bis R⁵ und m entsprechen denen der tricyclischen Benzoylcyclohexandion-Derivate der Formel I.

Insbesondere bevorzugt sind die Verbindungen der Formel XIII, wobei
- Y: gemeinsam mit den beiden Kohlenstoffen, an die es gebunden ist, folgende Heterocyclen ausbildet:

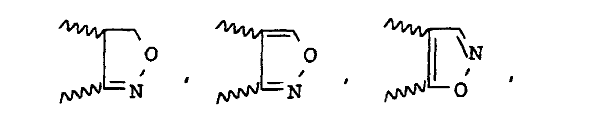

Hierbei sind die Verbindungen XIII außerordentlich bevorzugt, wobei
- R⁴: Nitro, Halogen, C₁-C₆-Alkyl, C₁-C₆-Halogenalkyl, C₁-C₆-Alkoxy, C₁-C₆-Alkylthio oder C₁-C₆-Alkylsulfonyl; insbesondere C₁-C₆-Alkylsulfonyl;
bedeutet.

Die Verbindungen der Formel XIII können auf verschiedene Art und Weise erhalten werden, beispielsweise kann das anellierte System analog zu den bei den Verbindungen der Formel VIc beschriebenen Verfahren aufgebaut werden.
Es ist aber auch möglich von einem geeigneten Grundkörper ausgehend das anellierte System aufzubauen (in Analogie zu den bei den Verbindungen der Formel VIc beschriebenen Verfahren) und anschließend L³ = Halogen durch übliche Halogenierungsreaktion einzuführen.

Ebenso sind die Aniline der Formel XIV und die Nitrile der Formel XV Gegenstand der vorliegenden Erfindung und neu, 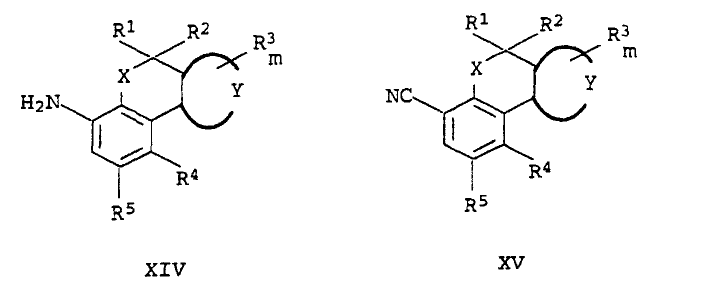 wobei die Variablen folgende Bedeutung haben:
- X: Sauerstoff, Schwefel, S=O oder S(=O)₂;
- Y: bildet gemeinsam mit den beiden Kohlenstoffen, an die es gebunden ist, einen gesättigten, partiell gesättigten oder ungesättigten 5-gliedrigen Heterocyclus, der ein bis drei gleiche oder verschiedene Heteroatome, ausgewählt aus folgender Gruppe: Sauerstoff, Schwefel oder Stickstoff, enthält;
- R¹,R²: Wasserstoff, C₁-C₆-Alkyl, C₁-C₆-Halogenalkyl, C₁-C₆-Alkoxy oder C₁-C₆-Halogenalkoxy;
- R³: Halogen, C₁-C₆-Alkyl, C₁-C₆-Halogenalkyl, C₁-C₆-Alkoxy oder C₁-C₆-Halogenalkoxy;
- R⁴: Nitro, Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, Aminosulfonyl, N-(C₁-C₆-Alkyl)-aminosulfonyl, N,N-Di-(C₁-C₆-alkyl)-aminosulfonyl, N-(C₁-C₆-Alkylsulfonyl)-amino, N-(C₁-C₆-Halogenalkylsulfonyl)-amino, N-(C₁-C₆-Alkyl)-N-(C₁-C₆-alkylsulfonyl)-amino oder N-(C₁-C₆-Alky)-N-(C₁-C₆-halogenalkylsulfonyl)-amino;
- R⁵: Wasserstoff, C₁-C₆-Alkyl oder Halogen;

Die besonders bevorzugten Ausführungsformen der Verbindungen der Formel XIV und XV in Bezug auf die Variablen X, Y, R¹ bis R⁵ und m entsprechen denen der tricyclischen Benzoylcyclohexandion-Derivate der Formel I.

Insbesondere bevorzugt sind die Verbindungen der Formel XV bzw. XVI, wobei
- Y: gemeinsam mit den beiden Kohlenstoffen, an die es gebunden ist, folgende Heterocyclen ausbildet:

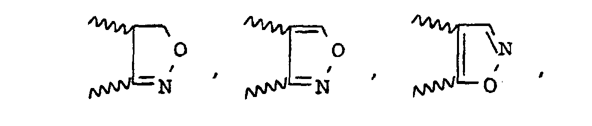

Hierbei sind die Verbindungen XIV bzw. XV außerordentlich bevorzugt, wobei
- R⁴: Nitro, Halogen, C₁-C₆-Alkyl, C₁-C₆-Halagenalkyl, C₁-C₆-Alkoxy, C₁-C₆-Alkylthio oder C₁-C₆-Alkylsulfonyl; insbesondere C₁-C₆-Alkylsulfonyl;
bedeutet.

Die Verbindungen der Formel XIV können auf verschiedene Art und Weise erhalten werden, beispielsweise kann das anellierte System analog zu den bei den Verbindungen der Formel VIc beschriebenen Verfahren aufgebaut werden.

Es ist aber auch möglich von einem geeigneten Grundkörper ausgehend das anellierte System aufzubauen (in Analogie zu den bei den Verbindungen der Formel VIC beschriebenen Verfahren) und anschließend durch Nitrierung para zu R⁴ in Analogie zu literaturbekannten Verfahren eine Nitrogruppe einzuführen und diese durch Reduktion auf an sich bekannte Art und Weise in die Aminogruppe überzuführen.

Gegenbenenfalls kann es von Vorteil sein, bei den voranstehend beschriebenen Synthesevarianten für bestimmte Funktionalitäten Schutzgruppen einzuführen, wenn die Kompatibilität der Funktionalitäten mit den erforderlichen Rekationsbedingungen nicht gegeben ist.

Die Wahl der Schutzgruppen richtet sich sowohl nach den Reaktionsbedingungen als auch nach der Molekülstruktur. Die Schutzgruppen, ihre Einführung und ihre Abspaltung sind in der Regel literaturbekannt (vgl. z.B. T.W. Greene et al., "Protective Groups in Organic Synthesis", 2^{nd} edition, Wiley, New York, 1991) und sie können in Analogie zu literaturbekannten Verfahren eingesetzt werden.

Weiterhin kann es notwendig sein eine Kombination der voranstehend beschriebenen Synthesevarianten durchzuführen.

Ebenso ist es möglich durch elektrophile, nukleophile, radikalische oder organometallische Rekationen sowie durch Oxidations- oder Reduktionsreaktionen weitere Substituenten einzuführen bzw. vorhandene Substituenten zu modifizieren.

### Herstellungsbeispiele:

### 1. (5,5-Dimethyl-1,3-dioxo-cyclohex-2-yl)-(6-methoxy-3a,4-dihydro-3H-chromeno[4,3-c]isoxazolin-9-yl)methanon (Verbindung 2.2)

### 2-Hydroxy-3-formyl-4-methoxy-benzoesäuremethylester

Zu einer Lösung von 50,1 g (0,275 mol) 2-Hydroxy-4-methoxybenzoesäuremethylester und 88 g (0,725 mol) Dichlormethoxymethan in 400 ml Methylenchlorid wurde bei 0 bis 5°C eine Lösung von 209,0 g (1,1 mol) Titantetrachlorid in 150 ml Methylenchlord getropft und über Nacht bei Raumtemperatur gerührt. Anschließend rührte man die Mischung in Eiswasser ein und extrahierte mit Methylenchlorid. Die vereinigten organischen Phasen wurden mit Natriumhydrogencarbonat-Lösung, Wasser und Natriumchlorid-Lösung gewaschen, getrocknet und anschließend das Lösungsmittel entfernt. Nach Chromatographie an Kieselgel mit Cyclohexan/Ethylacetat = 1:1 erhielt man 24,5 g (42 %) 2-Hydroxy-3-formyl-4-methoxy-benzoesäuremethylester in Form eines farblosen Feststoffes vom Fp.: 123 - 124°C.
¹H-NMR (CDCl₃, δ in ppm): 3,92 (s, 3H); 3,98 (s, 3H); 6,49 (d, 1H); 8,19 (d, 1H); 10,39 (s, 1H).

### 2-Allyloxy-3-formyl-4-methoxy-benzoesäuremethylester

Zu einer Mischung von 21,0 g (0,375 mol) Kaliumhydroxid und 20,2 g (0,096 mol) 2-Hydroxy-3-formyl-4-methoxy-benzoesäuremethylester in 500 ml Dimethylsulfoxid wurden bei Raumtemperatur 23,2 g (0,192 mol) Allylbromid getropft und 4 Stunden bei Raumtemperatur gerührt. Anschließend rührte man die Mischung in 1,5 1 3%ige wäßrige Salzsäure ein und extrahierte mit Ethylacetat. Die vereinigten organischen Phasen wurden mit Wasser gewaschen, getrocknet und das Lösungsmittel entfernt. Nach Chromatographie an Kieselgel mit Cyclohexan/Ethylacetat = 1:2 erhielt man 7,7 g (36 %) 2-Allyloxy-3-formyl-4-methoxy-benzoesäuremethylester in Form eines gelblichen Öls.
¹H-NMR (CDCl₃, δ in ppm): 3,86 (s, 3H); 3,93 (s, 3H); 4,58 (d, 2H); 5,32 (d, 1H); 5,39 (d, 1H); 6,15 (m, 1H); 6,79 (d, 1H); 8,04 (d, 1H); 10,41 (s, 1H).

### 6-Methoxy-9-methoxycarbonyl-3a,4-dihydro-3H-chromeno[4,3-c] isoxazolin

### Stufe a)

Zu einer Lösung von 2,25g (32,3 mmol) Hydroxylammoniumchlorid und 2,7 g Pyridin in 70 ml Wasser wurden bei Raumtemperatur 4,6 g (18,4 mmol) 2-Allyloxy-3-formyl-4-methoxy-benzoesäuremethylester in 70 ml Methanol zugetropft. Man ließ über Nacht bei Raumtemperatur rühren, gab 150 ml Wasser zu, extrahierte mit Methylenchlorid, wusch die vereinigten organischen Phasen mit 3-%iger wäßriger Salzsäure, trocknete und entfernte das Lösungsmittel. Das so erhaltene Oxim hat einen Festpunkt von 126 - 129°C.

### Stufe b)

Dieses Oxim wurde ohne weitere Aufreinigung weiter umgesetzt, indem man es in 40 ml Methylenchlorid löste und 15,0 ml (25,0 mmol) Natriumhypochloridlösung (12,5 % aktives Chlor) zutropfte. Man gab eine Spatenspitze Natriumacetat zu rührte 12 Stunden bei Raumtemperatur. Die organische Phase wurde abgetrennt, die wäßrige Phase mit Methylenchlorid extrahiert und die vereinigten organischen Phasen mit Wasser gewaschen. Man trocknete und entfernte das Lösungsmittel. Nach Chromatographie an Kieselgel mit Cyclohexan/Ethylacetat = 1:1 erhielt man 2,2 g (49 %) 6-Methoxy-9-methoxycarbonyl-3a,4-dihydro-3H-chromeno[4,3-c]isoxazolin in Form eines farblosen Feststoffe vom Fp.: 199 - 203°C.
¹H-NMR (CDCl₃, δ in ppm): 3,84 (s, 3H); 3,98 (s, 3H); 3,8 - 4,0 (m, 2H); 4,16 (dt, 1H); 4,63 (t, 1H); 4,84 (dd, 1H); 6,61 (d, 1H); 7,93 (d, 1H). 6-Methoxy-9-hydroxycarbonyl-3a,4-dihydro-3H-chromeno[4,3-c] isoxazolin

Zu einer Lösung von 2,1 g (8,0 mmol) 6-Methoxy-9-methoxycarbonyl-3a,4-dihydro-3H-chromeno[4,3-c]isoxazolin in 40 ml Methanol tropfte man bei Raumtemperatur eine Lösung von 0,8 g (20,0 mmol) Natriumhydroxid in 7 ml Wasser und erhitzte 6 Stunden unter Rückfluß. Nach dem Abkühlen entfernte man das Lösungsmittel, der Rückstand wurde in ca. 50 ml Waser aufgenommen und mit Methylenchlorid gewaschen. Anschließend säuerte man die wäßrige Phase mit 10%-iger Salzsäure an (pH = 1 - 2), saugte den Niederschlag ab, wusch mit Wasser und trocknete bei 60°C. Man erhielt 1,7 g (86 %) 6-Methoxy-9-hydroxycarbonyl-3a,4-dihydro-3H-chromeno[4,3-c]isoxazolin in Form farbloser Kristalle.
¹H-NMR (CDCl₃, δ in ppm): 3,73 (dd, 1H); 3,89 (s, 3H); 3,84 - 3,95 (m, 1H); 4,11 (dd, 1H); 4,54 (dd, 1H); 4,79 (dd, 1H); 6,61 (d, 1H); 7,81 (d, 1H).

### (5,5-Dimethyl-1,3-dioxo-cyclohex-2-yl)-(6-methoxy-3a,4-dihydro-3H-chromeno[4,3-c]isoxazolin-9-yl)methanon (Verbindung 2.2)

### Stufe a)

Zu einer Lösung von 0,50 g (2,0 mmol) 6-Methoxy-9-hydroxycarbonyl-3a,4-dihydro-3H-chromeno[(4,3-c)]isoxazolin in 30 ml Tetrachlorkohlenstoff gab man bei Raumtemperatur 0,26 g (2,2 mmol) Thionchlorid und einen Tropfen Dimethylformamid und rührte 3 Stunden bei 40 - 50°C. Anschließend entfernte man das Lösungsmittel im Vakuum. Man erhielt quantitativ (0,54 g) 6-Methoxy-9-chlorfomyl-3a,4-dihydro-3H-chromeno[4,3-c] isoxazolin als bräunliches Öl.

Man löste 0,54 g (2,0 mmol) 6-Methoxy-9-chlorformyl-3a,4-dihydro-3H-chromeno[4,3-c]isoxazolin in 30 ml Acetonitril und tropfte es bei 0°C zu einer Lösung von 0,28 g (2,0 mmol) 5,5-Dimethylcyclohexan-1,3-dion und 0,6 g (6,0 mmol) Triethylamin in 20 ml Acetonitril. Nach 12 Stunden Rühren bei Raumtemperatur entfernte man das Lösungsmittel, nahm in Methylenchlorid auf und wusch mit Wasser. Man trocknete und destillierte das Lösungsmittel ab. Der Rückstand wurde in 25 ml Acetonitril gelöst, mit 0,59 g (6,0 mmol) Trimethylsilylcyanid zersetzt und 5 Stunden bei 40°C gerührt. Nach Filtration wurde das Lösungsmittel entfernt und mit Cyclohexan/Essigsäureethylester chromatographiert. Man erhielt 0,59 g (79 %) der Verbindung 2.2 als farblose Kristalle mit einem Festpunkt von 203-206°C.
¹H-NMR (CDCl₃, g in ppm): 1,09 (s, 6H); 2,31 (s, 1H); 2,62 (s, 1H); 3,7-4,1 (m, 6H); 4,52 (dd, 1H); 4,61 (dd, 1H); 6,62 (d, 1H); 7,43 (d, 1H); 17,0 (Cs, 1H).

### 2. (5-Brom-8-methylsulfonyl-3a,4-diyhdro-3H-indeno[1,2-c]isoxazol

### 2-Allyl-6-chlor-benzaldehyd

Eine Lösung von 10,89 g (0,107 mol) Trimethylethylendiamin in 50 ml wasserfreiem Tetrahydrofuran wurde unter Schutzgasatmosphäre auf -10°C gekühlt und mit 66,6 ml 1,6 molarer n-Butyllithium-Lösung in Hexan (0,107 mol) tropfenweise versetzt. Nach 10 Minuten gab man 15 g (0,107 mol) 6-Chlorbenzaldehyd in 70 ml Tetrahydrofuran tropfenweise zu, versetzte mit weiteren 0,214 mol n-Butyllithium in Hexan (146,8 ml) und rührte 2,5 Stunden bei 0°C. Es wurde auf -20°C abgekühlt, 12,42 g (0,139 mol) Kupfer(I)cyanid zugegeben, 30 Minuten bei -10°C gerührt und anschließend 28,42 g Allylbromid in 100 ml Tetrahydrofuran zugetropft. Man rührte noch 2,5 Stunden bei 0°C, ehe 230 ml gesättigte Ammoniumchlorid-Lösung zugetropft wurden. Der dabei anfallende Feststoff wurde abgetrennt und die wäßrige Phase mit Diethylether extrahiert. Die vereinigten organischen Phasen wurden dann mit gesättigter Ammoniumchlorid-Lösung gewaschen, getrocknet und im Vakuum das Lösungsmittel entfernt. Man erhielt 17,0 g 2-Alkyl-6-chlorbenzaldehyd (89 %) in Form eines dunklen Öls.
¹H-NMR (CDCl₃, δ in ppm): 3,73 (d, 2H); 5,05 (dd, 2H); 5,96 (m, 1H); 7,05-7,48 (m, 3H); 10,58 (s, 1H).

### 2-Allyl-6-chlor-benzaldehyd-oxim

Zu einer Lösung von 4,62 g Hydroxylamin-Hydrochlorid in 50 ml Wasser wurden 5,58 g Natriumhydrogencarbonat gegeben und auf 0°C gekühlt. Dazu tropfte man eine Lösung von 9,7 g (44,32 mmol) 2-Allyl-6-chlor-benzaldehyd in 50 ml Methanol und rührte bei Raumtemperatur über Nacht. Anschließend wurde das Methanol im Vakuum entfernt und der Rückstand in 300 ml Wasser eingerührt. Die wäßrige Phase extrahierte man mit Diethylether, die vereinigten organischen Phasen wurden mit gesättigter Ammoniumchlorid-Lösung gewaschen, getrocknet und das Lösungmittel entfernt. Man erhielt 8,7 g (quantitativ) 2-Allyl-6-chlorbenzaldehyd-oxim in Form eines zähen Öls.
¹H-NMR (CDCl₃, δ in ppm): 3,58 (d, 2H); 5,02 (2d, 2H); 5,95 (m, 1H); 7,08-7,36 (m, 3H); 8,49 (s, 1H).

### 8-Chlor-3a,4-dihydro-3H-indeno[1,2-c]isoxazol

Zu einer Lösung von 8,4 g (42,9 mmol) 2-Allyl-6-chlorbenzaldehyd-oxim in 100 ml Methylenchlorid wurden bei Raumtemperatur 37,0 ml einer Natriumhypochloridlösung (12,5 % aktives Chlor) getropft und eine Spatelspitze Natriumacetat zugegeben. Man rührte 2 Stunden bei Raumtemperatur, trennte die organische Phase ab, extrahierte die wäßrige Phase mit Methylenchlorid und wusch die vereinigten organischen Phasen mit gesättigter Ammoniumchlorid-Lösung. Es wurde getrocknet und das Lösungsmittel entfernt. Man erhielt 7,0 g (94 %) 8-Chlor-3a,4-dihydro-3H-indeno-[1,2-c]isoxazol in Form eines zähen Öls.
¹H-NMR (CDCl₃, δ in ppm): 2,81 (dd, 1H); 3,24 (dd, 1H); 3,78-4,03 (s, 2H); 4,78 (t, 1H); 7,23-7,41 (m, 3H).

### 8-Methylthio-3a,4-dihydro-3H-indeno[1,2c]isoxazol

Zu einer Lösung von 5,0 g (25,8 mmol) 8-Chlor-3a,4-dihydro-3H-indeno-[1,2-c]isoxazol in 60 ml N-Methylpyrrolidon wurden bei Raumtemperatur 3,6 g (52,0 mmol) Natriumthiomethylat gegeben und über Nacht gerührt. Anschließend rührte man in 800 ml Wasser ein, extrahierte die wäßrige Phase mit Diethylether, wusch die vereinigten organischen Phasen mit gesättigter Ammoniumchlorid-Lösung, trocknete und entfernte das Lösungsmittel. Man erhielt 4,6 g (87 %) 8-Methylthio-3a,4-dihydro-3H-indeno[1,2-c]isoxazol in Form eines dunkelbraunen Feststoffs.
¹H-NMR (CDCl₃, δ in ppm): 2,54 (s, 3H); 2,78 (dd, 1H); 3,21 (dd, 1H); 3,72-3,93 (s, 2H); 4,64 (t, 1H); 7,09-7,38 (m, 3H).

### 5-Brom-8-methylthio-3a,4-dihydro-3H-indeno[1,2-c]isoxazol

Man kühlte 120 ml Schwefelsäure (98 proz.) auf 0°C und gab portionsweise 11,2 g (54,8 mmol) 8-Methylthio-3a,4-dihydro-3H-indeno[1,2-c]isoxazol zu. Anschließend tropfte man 9,2 g (57,5 mmol) Brom zu und rührte 2 Stunden bei 0°C weiter. Man goß die entstandene Lösung auf 2 1 eines Gemisches von Wasser und Eis, rührte 1,5 Stunden und saugte den ausgefallenen Feststoff ab, der anschließend gewaschen und dann getrocknet wurde. Man erhielt 11,4 g (73 %) 5-Brom-8-methylthio-3a,4-dihydro-3H-indeno[1,2-c]isoxazol eines braunen Feststoffs mit einem Fp. von 127-135°C.
¹H-NMR (CDCl₃, δ in ppm): 2,53 (s, 3H); 2,71 (dd, 1H); 3,24 (dd, 1H); 3,81-4,02 (s, 2H); 4,71 (t, 1H); 7,01 (d, 1H); 7,47 (d, 1H).

### 5-Brom-8-methylsulfonyl-3a,4-dihydro-3H-indeno[1,2-c]isoxazol

Eine Lösung von 11,2 g (39,4 mmol) 5-Brom-8-methylthio-3a,4-dihydro-3H-indeno[1,2-c]isoxazol und 1,55 g Natriumwolframat in 250 ml Toluol und 50 ml Eisessig wurde auf 70°C erhitzt und tropfenweise mit 10,73 g (39 proz., 86,8 mmol) Wasserstoffperoxid versetzt. Man rührte noch 3 Stunden bei 70°C weiter, wobei ein Feststoff ausfiel. Man ließ die Mischung auf Raumtemperatur abkühlen, rührte in 1 1 Wasser ein und saugte den weißen Feststoff ab. Die organische Phase des Filtrats wurde abgetrennt und die wäßrige mit Essigsäureethylester extrahiert. Die vereinigten organischen Phasen wurden mit Wasser gewaschen, getrocknet und das Lösungsmittel entfernt. Man erhielt ein zähes braunes Öl, welches mit Hexan/Essigsäureethylester (4:1) verrührt wurde. Der sich bildende Niederschlag wurde abgesaugt und mit den oben erhaltenen Feststoff vereinigt. Man erhielt 7,3 g (59 %) 5-Brom-8-methylsulfonyl-3a,4-dihydro-3H-indeno[1,2-c]-isoxazol.
¹H-NMR (d⁶-DMSO, δ in ppm): 2,93 (dd, 1H); 3,23 (dd, 1H); 3,41 (s, 3H); 3,94 (dd, 1H); 4,16 (m, 1H); 4,81 (t, 1H); 7,82 (d, 1H); 8,03 (d, 1H).

### 3. 2-Methyl-6-hydroxycarbonyl-9-chlor-[1]-thiochromano[4,3-c]pyrazol

### 2-Chlorsulfonyl-4-chlor-benzoesäuremethylester

Zu einer Lösung von 139 g (0,75 mol) 2-Amino-4-chlor-benzoesäuremethylester in 400 ml konzentrierter Salzsäure wurden bei 0 bis 5°C eine Lösung von 60,9 g (0,88 mol) Natriumnitrit in 100 ml Wasser zugetropft und noch 1 Stunde bei 0°C nachgerührt.
In einer zweiten Apparatur wurden 3 g Kupfer-(II)-chlorid, 3 g Benzyltriethylammoniumchlorid, 10 ml Wasser und 400 ml 1,2-Dichlorethan vereinigt und 64 g (1 mol) Schwefeldioxid eingeleitet.
Anschließend wurde das Diazoniumsalz wie oben beschrieben bei 10 bis 15°C zugegeben und langsam auf 50°C erwärmt. Dann leitete man weitere 54 g (0,84 mol) Schwefeldioxid ein und rührte noch 30 Minuten bei 50°C. Nach Abkühlen wurden bei Raumtemperatur dann 7,4 g (0,1 mol) Chlor eingegast, 15 Minuten nachgerührt und anschließend die sich bildenden Phasen getrennt. Die organische Phase wurde getrocknet und das Lösungsmittel entfernt. Man erhielt 207 g 2-Chlorsulfonyl-4-Chlor-benzoesäuremethylester.
¹H-NMR (CDCl₃, δ in ppm): 4,00 (s, 3H); 7,75 (m, 2H); 8,18 (m, 1H)

### 2-Mercapto-4-chlor-benzoesäuremethylester

Zu einer Suspension von 205 g (0,75 mol) 2-Chlorsulfonyl-4-chlor-benzoesäuremethylester in 1 1 konzentrierter Salzsäure und 375 g Eis wurde portionsweise innerhalb von 1,5 Stunden 243,5 g (3,7 mol) Zinkpulver gegeben. Man rührte 3 Stunden nach und erhitzte langsam auf 70°C. Nach 2 Stunden bei dieser Temperatur kühlte man ab. Nach 12 Stunden stehen bei Raumtemperatur wurde das Reaktionsgemisch mit Ethylacetat extrahiert, die vereinigten organischen Phasen und das Lösungsmittel entfernt. Man erhielt 125,4 g (83 %) 2-Mercapto-4-chlor-benzoesäuremethylester.
¹H-NMR (CDCl₃, δ in ppm): 3,95 (s, 3H); 4,88 (s, 1H); 7,10 (m, 1H); 7,30 (m, 1H); 7,96 (d, 1H).

### 2-(2-Hydroxycarbonyl-eth-1-yl)-thio-4-chlor-benzoesäuremethylester

Zu einer Lösung von 125,4 g (0,62 mol) 2-Mercapto-4-chlorbenzoesäuremethylester in 1,5 1 Aceton wurde 179,5 g (1,3 mol) Kaliumcarbonat und portionsweise 94,5 g (0,62 mol) 3-Brompropionsäure gegeben und das Reaktionsgemisch 12 Stunden bei Raumtemperatur gerührt. Man destillierte das Lösungsmittel ab, nahm den Rückstand in Wasser auf und extrahierte mit Diethylether. Dann wurde die wäßrige Phase mit konzentrierter Salzsäure sauer gestellt, der ausgefallene Niederschlag abgesaugt und getrocknet. Man erhielt 150 g (88 %) 2- (2-Hydroxycarbony-eth-1-yl)-thio-4-chlor-benzosäuremethylester
Fp.: 133 bis 136°C

### 5-Chlor-4-oxo-thiochroman-8-carbonsäuremethylester

50 g (0,18 mol) 2-(2-Hydroxycarbonyl-eth-1-yl)-thio-4-chlorbenzoesäuremethylester wurden bei 70°C zu 500 g Polyphosphorsäure gegen und noch 30 Minuten nachgerührt. Anschließend wurde das Reaktionsgemisch in Wasser eingerührt, der ausgefallene Niederschlag abgesaugt und getrocknet. Man erhielt 41,1 g (88 %) 5-Chlor-4-oxo-thiochroman-8-carbonsäuremethylester.
¹H-NMR (CDCl₃, δ in ppm): 3,08 (m, 4H); 3,96 (s, 3H); 7,14 (d, 1H); 7,95 (d, 1H).

### 5-chlor-3- (N,N-dimehtylaminomethyliden)-4-oxo-thiochroman-8-carbonsäuremethylester

30 g (0,078 mol) 5-Chlor-4-oxo-thiochroman-8-carbonsäuremethylester wurden in 300 ml N,N-Dimethylformamid-dimethylacetal 6 Stunden unter Rückfluß erhitzt. Dann wurden flüchtige Bestandteile abdestilliert, der Rückstand in Methylenchlorid aufgenommen und die organische Phase mit Wasser gewaschen. Nach Trocknung und Entfernen des Lösungsmittels erhielt man 35,3 g (97 %) 5-Chlor-3-(N,N-dimethylaminomethyliden)-4-oxothiochroman-8-carbonsäuremethylester.
¹H-NMR (CDCl₃, δ in ppm): 3,18 (s, 6H); 3,80 (s, 2H); 3,95 (s, 3H); 7,24 (d, 1H); 7,64 (s, 1H); 7,82 (d, 1H) .

### 2-Methyl-6-methoxycarbonyl-9-chlor-[1]-thiochromano[4,3-c] pyrazol

Zu einer Lösung von 7,0 g (22,5 mmol) 5-Chlor-3-(N,N-dimethylaminomethyliden)-4-oxo-thiochroman-8-carbonsäuremethylester in 700 ml Ethanol wurden 1,3 g (29,2 mmol) Methylhydrazin zugetropft und 2 Stunden unter Rückfluß erhitzt. Das Lösungsmittel wurde entfernt und der Rückstand an Kieselgel mit Ethylacetat/Cyclohexan (2:3) als Eluent chromatografiert. Man erhielt 4,0 g (60 %) 2-Methyl-6-methoxycarbonyl-9-chlor-[1]-thiochromano[4,3-c]pyrazol.
¹H-NMR (CDCl₃, δ in ppm) : 3,76 (s, 2H); 3,95 (s, 3H); 4,00 (s, 3H); 7,24 (s, 1H); 7,36 (d, 1H); 7,70 (d, 1H).

### 2-Methyl-6-hydroxycarbonyl-9-chlor-[1]-thiochromano[4,3-c] pyrazol

4,0 g (13,6 mmol) 2-Methyl-6-methoxycarbonyl-9-chlor-[1]-thiochromano[4,3-c]pyrazol wurden in 100 ml Methanol/Wasser (1:1) mit 0,8 g (20 mmol) Natriumhydroxid 1 Stunde unter Rückfluß erhitzt. Man entfernte das organische Lösungsmittel am Vakuum und extrahierte den Rückstand mit Ethylacetat. Die wäßrige Phase wurde mit konzentrierter Salzsäure angesäuert, der ausgefallene Niederschlag abgesaugt und getrocknet. Man erhielt 3,5 g (92 %) 2-Methyl-6-hydroxycarbonyl-9-chlor-[1]-thiochromano[4,3-c]pyrazol
¹H-NMR (CDCl₃, δ in ppm): 3,80 (s, 2H); 3,96 (s, 3H); 7,40 (d, 1H); 7,65 (m, 2H).

In den Tabellen 2 bis 4 sind neben den voranstehenden Verbindungen noch weitere tricyclische Benzoylcyclohexandion-Derivate der Formel I aufgeführt, die in analoger Weise hergestellt wurden oder herstellbar sind:

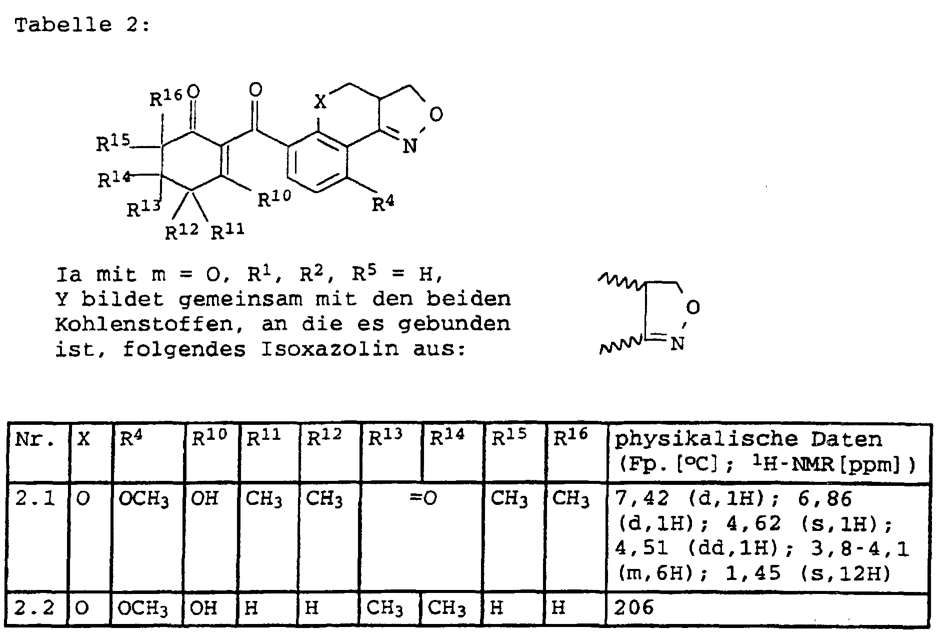

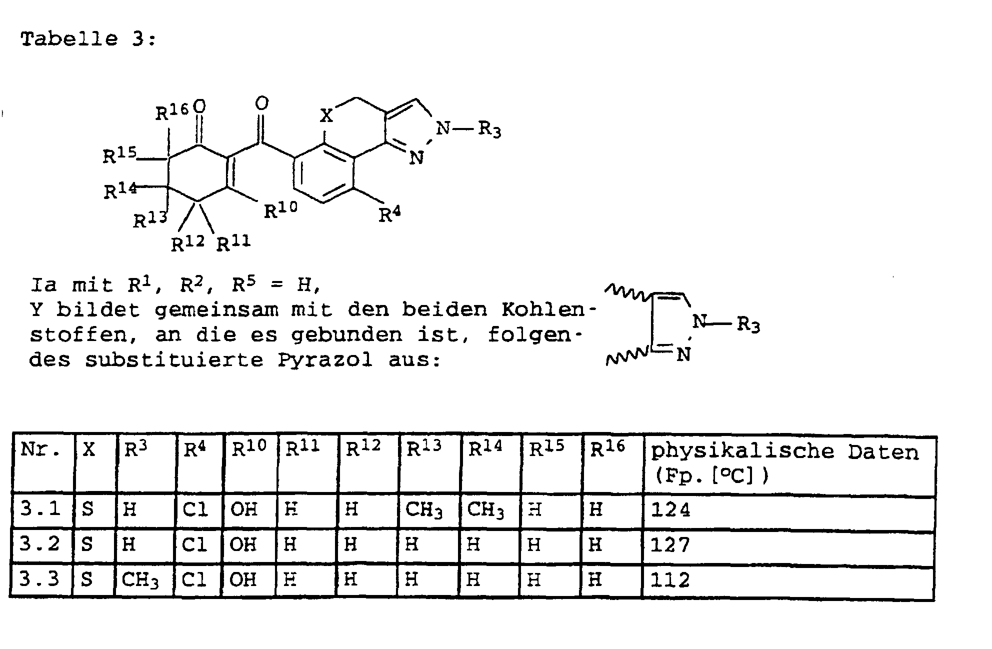

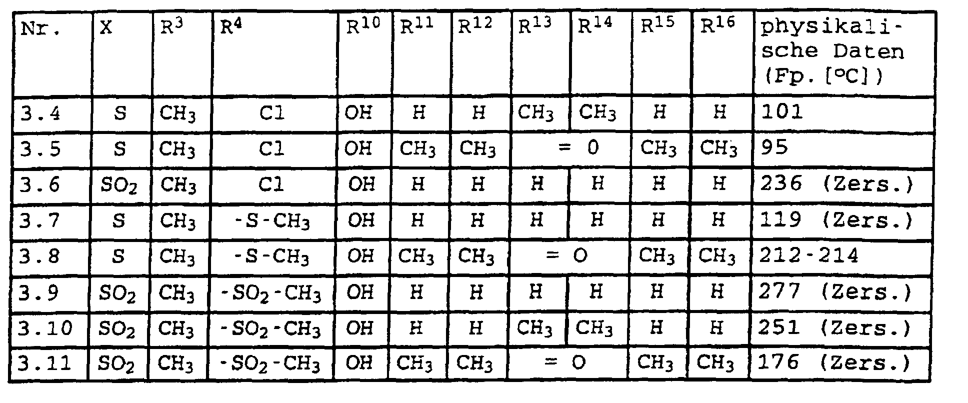

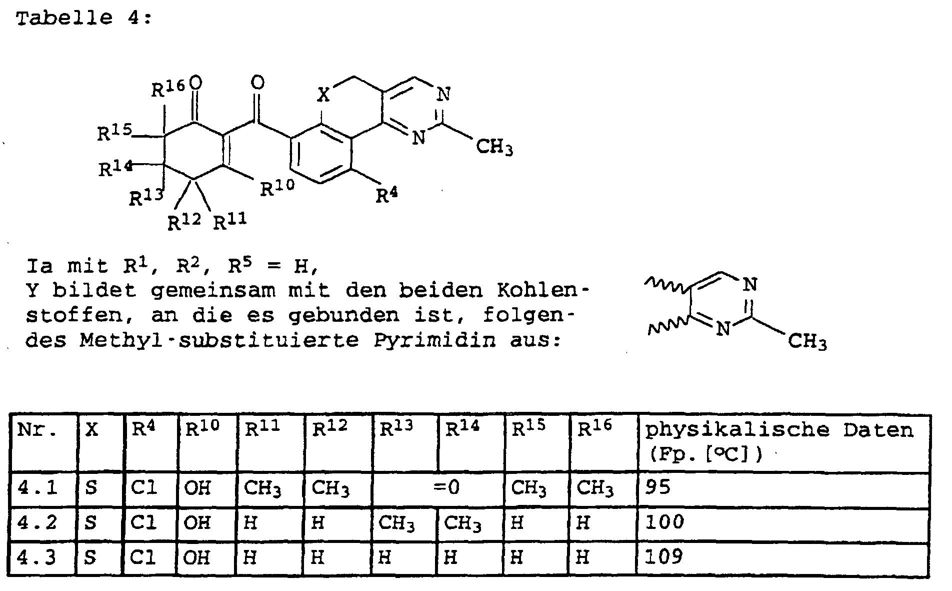

Die Verbindungen der Formel I und deren landwirtschaftlich brauchbaren Salze eignen sich sowohl als Isomerengemische als auch in Form der reinen Isomeren - als Herbizide. Die herbiziden Mittel, die Verbindungen der Formel I enthalten, bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen der Formel I bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus können die Verbindungen der Formel I auch in Kulturen, die durch Züchtung einschließlich gentechnischer Methoden gegen die Wirkung von Herbiziden tolerant sind, verwandt werden.

Die Verbindungen der Formel I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die herbiziden Mittel enthalten eine herbizid wirksame Menge mindestens einer Verbindung der Formel I oder eines landwirtschaftlich brauchbaren Salzes von I und für die Formulierung von Pflanzenschutzmitteln übliche Hilfsmittel.

Als inerte Hilfsstoffe kommen im Wesentlichen in Betracht:

Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline und deren Derivate, alkylierte Benzole oder deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die tricyclischen Benzoylcyclohexandion-Derivate der Formel I als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Lauryletherund Fettalkoholsulfaten, sowie Salze sulfatierter Hexa-, Heptaund Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Verbindungen der Formel I in den anwendungsfertigen Zubereitungen können in weiten Bereichen variiert werden. Im allgemeinen enthalten die Formulierungen etwa von 0,001 bis 98 Gew.-%, vorzugsweise 0,01 bis 95 Gew.-%, mindestens eines wirkstoffs. Die Wirkstoffe werden dabei in einer Reinheit von 90% bis 100%, vorzugsweise 95% bis 100% (nach NMR-Spektrum) eingesetzt.

Die folgenden Formulierungsbeispiele verdeutlichen die Herstellung solcher Zubereitungen:
I. 20 Gewichtsteile der Verbindung Nr. 3.1 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Rizinusöl besteht. Durch Ausgießen und feines Verteilen der Lösung in 100000 Gewichtsteilen Wasser erhält man eine wäßrige Dispersion, die 0,02 Gew.-% des Wirkstoffs enthält.
II. 20 Gewichtsteile der verbindung Nr. 3.2 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Rizinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
III. 20 Gewichtsteile der Verbindung Nr. 3.4 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
IV. 20 Gewichtsteile der Verbindung Nr. 3.5 werden mit 3 Gewichtsteilen des Natriumsalzes der Diisobutylnaphthalinsulfonsäure, 17 Gewichtsteilen des Natriumsalzes einer Ligninsulfonsäure aus einer Sulfit-Ablauge und 60 Gewichts-teilen pulverförmigem Kieselsäuregel gut vermischt und in einer Hammermühle vermahlen. Durch feines verteilen der Mischung in 20000 Gewichtsteilen Wasser erhält man eine Spritzbrühe, die 0,1 Gew.-% des Wirkstoffs enthält.
V. 3 Gewichtsteile der Verbindung Nr. 4.1 werden mit 97 Gewichtsteilen feinteiligem Kaolin vermischt. Man erhält auf diese Weise ein Stäubemittel, das 3 Gew.-% des Wirkstoffs enthält.
VI. 20 Gewichtsteile der Verbindung Nr. 4.2 werden mit 2 Gewichtsteilen Calciumsalz der Dodecylbenzolsulfonsäure, 8 Gewichtsteilen Fettalkoholpolyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII. 1 Gewichtsteil der Verbindung Nr. 3.3 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII. 1 Gewichtsteil der Verbindung Nr. 4.2 wird in einer Mischung gelöst, die aus 80 Gewichtsteilen Cyclohexanon und 20 Gewichtsteilen Wettol^{R} EM 31 (= nichtionischer Emulgator auf der Basis von ethoxyliertem Rizinusöl) besteht. Man erhält ein stabiles Emulsionskonzentrat.

Die Applikation der Verbindungen der Formel I bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Verbindung der Formel I betragen je nach Bekämpfungsziel, Jahreszeit, Zielpflanzen und Wachstumsstadium 0.001 bis 3.0, vorzugsweise 0.01 bis 1.0 kg/ha aktive Substanz (a.S.).

Zur Verbreiterung des Wirkungsspektrums und zur Erzielung syner-gistischer Effekte können die tricyclischen Benzoylcyclohexandion-Derivate der Formel I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-Aroyl-1,3-cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexenonoximetherderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Außerdem kann es von Nutzen sein, die Verbindungen der Formel I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Anwendungsbeispiele

Die herbizide Wirkung der tricyclischen Benzoylcyclohexandion-Derivate der Formel I ließ sich durch die folgenden Gewächshausversuche zeigen:

Als Kulturgefäße dienten Plastiktöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsich-5 tigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 0,5, 0,25 bzw. 0,125 kg/ha a.S..

Die Pflanzen wurden artenspezifisch bei Temperaturen von 10 bis 25°C bzw. 20 bis 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Abutilon theophrasti | Chinesischer Hanf | velvet leaf |
| Chenopodium album | Weißer Gänsefuß | lambsquaters (goose-foot) |
| Echinochloa crusgalli | Hühnerhirse | barnyardgrass |
| Ipomoea ssp. | Prunkwindearten | morningglory |
| Solanum nigrum | Schwarzer Nachtschatten | black nightshade |
| Sinapis alba | Weißer Senf | white mustard |
| Setaria viridis | Grüne Borstenhirse | green foxtail |

Bei Aufwandmengen von 0,25 bzw. 0,125 kg/ha zeigt die Verbindung 2.1 im Nachauflauf eine sehr gute Wirkung gegen die oben genannten unerwünschten Pflanzen Chenopodium album, Echinochloa crus-galli, Ipomoea ssp., Setaria viridis und Solanum nigrum. weiterhin werden die Schadpflanzen Abutilon theophrasti, Echinochloa crus-galli, Sinapis alba und Solanum nigrum im Nachauflauf bei Aufwandmengen von 0,5 bzw. 0,25 kg/ha sehr gut von Verbindung 4.2 bekämpft.

## Patentansprüche

1. Tricyclische Benzoylcyclohexandion-Derivate der Formel I 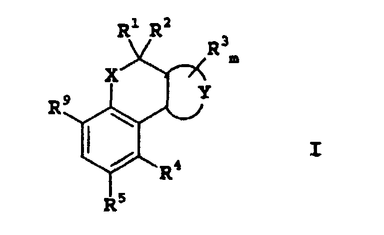 in der die Variablen folgende Bedeutungen haben:
X Sauerstoff, Schwefel, S=O oder S(=O)₂;
Y bildet gemeinsam mit den beiden Kohlenstoffen, an die es gebunden ist, einen gesättigten, partiell gesättigten oder ungesättigten 5-gliedrigen Heterocyclus, der ein bis drei gleiche oder verschiedene Heteroatome, ausgewählt aus folgender Gruppe: Sauerstoff, Schwefel oder Stickstoff, enthält;
R¹,R² Wasserstoff, C₁-C₆-Alkyl, C₁-C₆-Halogenalkyl, C₁-C₆-Alkoxy oder C₁-C₆-Halogenalkoxy;
R³ Halogen, C₁-C₆-Alkyl, C₁-C₆-Halogenalkyl, C₁-C₆-Alkoxy oder C₁-C₆-Halogenalkoxy;
R⁴ Nitro, Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, Aminosulfonyl, N-(C₁-C₆-Alkyl)-aminosulfonyl, N,N-Di-(C₁-C₆-alkyl)-aminosulfonyl, N- (C₁-C₆-Alkylsulfonyl)-amino, N-(C₁-C₆-Halogenalkylsulfonyl) -amino, N-(C₁-C₆-Alkyl)-N-(C₁-C₆-alkylsulfonyl) -amino oder N- (C₁-C₆-Alky)-N- (C₁-C₆halogenalkylsulfonyl)-amino;
R⁵ Wasserstoff, C₁-C₆-Alkyl oder Halogen;
m 0, 1 oder 2;
R⁹ ein Rest IIa oder IIb 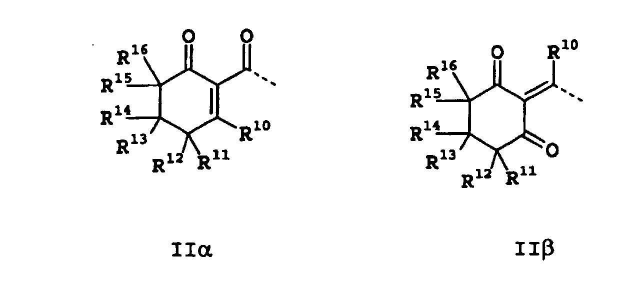 wobei die Variablen folgende Bedeutung haben:
R¹⁰ Hydroxy;
R¹¹, R¹⁵ Wasserstoff, C₁-C₄-Alkyl oder C₁-C₄-Alkoxycarbonyl;
R¹², R¹⁴, R¹⁶ Wasserstoff oder C₁-C₄-Alkyl;
R¹³ Wasserstoff, Halogen, Hydroxy, C₁-C₆-Alkyl, C₁-C₆-Halogenalkyl, Di-(C₁-C₆-alkoxy)-methyl, (C₁-C₆-Alkoxy)-(C₁-C₆-alkylthio)-methyl, Di- (C₁-C₆alkylthio) methyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, C₁-C₆-Alkoxycarbonyl, C₁-C₆-Halogenalkoxycarbonyl;
1,3-Dioxolan-2-yl, 1,3-Dioxan-2-yl, 1,3-Oxathiolan-2-yl, 1,3 Oxathian-2-yl, 1,3-Dithiolan-2-yl oder 1,3-Dithian-2-yl, wobei die sechs letztgenannten Reste durch einen bis drei C₁-C₄-Alkylreste substituiert sein können;
oder
R¹² und R¹³ oder R¹³ und R¹⁶ bilden gemeinsam eine π-Bindung oder eine C₁-C₅-Alkylkette, die einen bis drei Reste aus folgender Gruppe tragen kann: Halogen, Cyano, C₁-C₄-Alkyl, C₁-C₄-Halogenalkyl oder C₁-C₄-Alkoxycarbonyl, aus;
oder
R¹² und R¹⁶ bilden gemeinsam eine C₁-C₄-Alkylkette, die einen bis drei Reste aus folgender Gruppe tragen kann: Halogen, Cyano, C₁-C₄-Alkyl, C₁-C₄-Halogenalkyl oder C₁-C₄-Alkoxycarbonyl, aus;
R¹³ und R¹⁴ bilden gemeinsam eine -O-(CH₂)ₚ-O-, -O-(CH₂)ₚ-S-, -S-(CH₂)ₚ-S-, -O-(CH₂)_{q}- oder -S-(CH₂)_{q}-Kette, die durch einen bis drei Reste aus folgender Gruppe substituiert sein kann: Halogen, Cyano, C₁-C₄-Alkyl, C₁-C₄-Halogenalkyl oder C₁-C₄-Alkoxycarbonyl;
oder
R¹³ und R¹⁴ bilden gemeinsam mit dem Kohlenstoff, an dem sie gebunden sind eine Carbonylgruppe aus;
p 2, 3 oder 4;
q 1, 2, 3, 4 oder 5;
sowie deren landwirtschaftlich brauchbaren Salze.

2. Verfahren zur Herstellung von tricyclischen Benzoylcyclohexandion-Derivaten der Formel Iα (= I mit R¹⁰ = Hydroxy) gemäß Anspruch 1, **dadurch gekennzeichnet, daß** man ein Cyclohexandion der Formel V, in der die Variablen R¹¹ bis R¹⁶ die unter Anspruch 1 genannte Bedeutung haben, 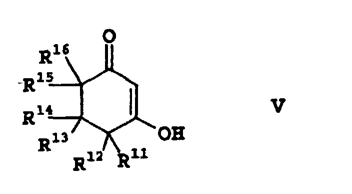 mit einer aktivierten tricyclischen Benzoesäure der Formel VIa oder mit einer tricyclischen Benzoesäure VIß, 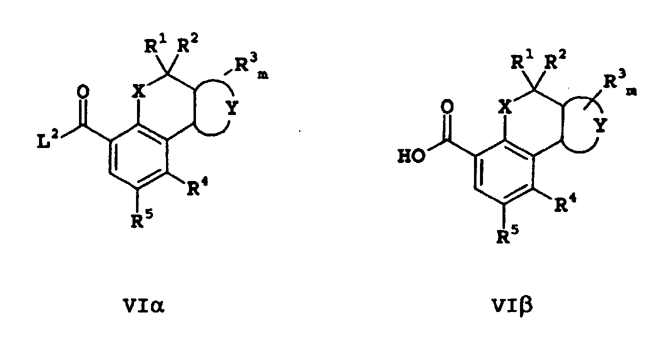 wobei die Variablen R¹ bis R⁵, X, Y und m die unter Anspruch 1 genannte Bedeutung haben und L² für eine nucleophil verdrängbare Abgangsgruppe steht, acyliert und das Acylierungsprodukt gegebenenfalls in Gegenwart eines Katalysators umlagert.

3. Mittel, enthaltend eine herbizid wirksame Menge mindestens eines tricyclischen Benzoylcyclohexandion-Derivates der Formel I oder eines landwirtschaftlich brauchbaren Salzes von I gemäß Anspruch 1 und für die Formulierung von Pflanzenschutzmitteln übliche Hilfsmittel.

4. Verfahren zur Herstellung von Mitteln gemäß Anspruch 3, **dadurch gekennzeichnet, daß** man eine herbizid wirksame Menge mindestens eines tricyclischen Benzoylcyclohexandion-Derivates der Formel I oder eines landwirtschaftlich brauchbaren Salzes von I gemäß Anspruch 1 und für die Formulierung von Pflanzenschutzmitteln übliche Hilfsmittel mischt.

5. Verfahren zur Bekämpfung von unerwünschtem Pflanzenwuchs, **dadurch gekennzeichnet, daß** man eine herbizid wirksame Menge mindestens eines tricyclischen Benzoylcyclohexandion-Derivates der Formel I oder eines landwirtschaftlich brauchbaren Salzes von I gemäß Anspruch 1, auf Pflanzen, deren Lebensraum und/oder auf Samen einwirken läßt.

6. Verwendung von tricyclischen Benzoylcyclohexandion-Derivaten der Formel I oder deren landwirtschaftlich brauchbaren Salze gemäß Anspruch 1 als Herbizide.

## Claims

1. A tricyclic benzoylcyclohexanedione derivative of the formula I 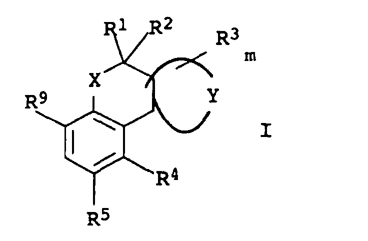 where:
X is oxygen, sulfur, S=O or S(=O)₂;
Y together with the two carbons to which it is attached forms a saturated, partially saturated or unsaturated 5-membered heterocycle which contains one to three identical or different heteroatoms selected from the following group: oxygen, sulfur or nitrogen;
R¹,R² are hydrogen, C₁-C₆-alkyl, C₁-C₆-haloalkyl, C₁-C₆-alkoxy or C₁-C₆-haloalkoxy;
R³ is halogen, C₁-C₆-alkyl, C₁-C₆-haloalkyl, C₁-C₆-alkoxy or C₁-C₆-haloalkoxy;
R⁴ is nitro, halogen, cyano, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₁-C₆-alkylsulfinyl, C₁-C₆-haloalkylsulfinyl, C₁-C₆-alkylsulfonyl, C₁-C₆-haloalkylsulfonyl, aminosulfonyl, N-(C₁-C₆-alkyl)-aminosulfonyl, N,N-di-(C₁-C₆-alkyl)-aminosulfonyl, N-(C₁-C₆-alkylsulfonyl)-amino, N-(C₁-C₆-haloalkylsulfonyl)-amino, N-(C₁-C₆-alkyl)-N-(C₁-C₆-alkylsulfonyl)-amino or N-(C₁-C₆-alkyl)-N-(C₁-C₆-haloalkylsulfonyl)-amino;
R⁵ is hydrogen, C₁-C₆-alkyl or halogen;
m is 0, 1 or 2;
R⁹ is a radical IIa or IIb 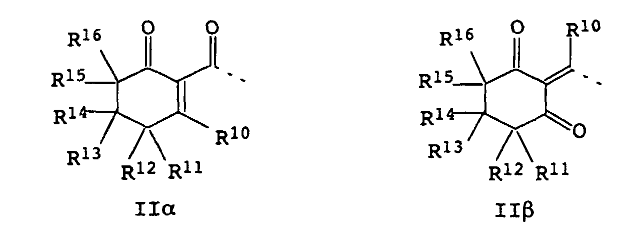
where:
R¹⁰ is hydroxyl;
R¹¹, R¹⁵ are hydrogen, C₁-C₄-alkyl or C₁-C₄-alkoxycarbonyl;
R¹², R¹⁴, R¹⁶ are hydrogen or C₁-C₄-alkyl;
R¹³ is hydrogen, halogen, hydroxyl, C₁-C₆-alkyl, C₁-C₆-haloalkyl, di-(C₁-C₆-alkoxy)methyl, (C₁-C₆-alkoxy)-(C₁-C₆-alkylthio)methyl, di-(C₁-C₆-alkylthio)methyl, C₁-C₆-alkoxy, C₁-C₆-haloalkoxy, C₁-C₆-alkylthio, C₁-C₆-haloalkylthio, C₁-C₆-alkylsulfinyl, C₁-C₆-haloalkylsulfinyl, C₁-C₆-alkylsulfonyl, C₁-C₆-haloalkylsulfonyl, C₁-C₆-alkoxycarbonyl, C₁-C₆-haloalkoxycarbonyl;
is 1,3-dioxolan-2-yl, 1,3-dioxan-2-yl, 1,3-oxathiolan-2-yl, 1,3-oxathian-2-yl, 1,3-dithiolan-2-yl or 1,3-dithian-2-yl, where the six last-mentioned radicals may be substituted by one to three C₁-C₄-alkyl radicals;
or
R¹² and R¹³ or R¹³ and R¹⁶ together form a π-bond or a C₁-C₅-alkyl chain which may carry one to three radicals from the following group: halogen, cyano, C₁-C₄-alkyl, C₁-C₄-haloalkyl or C₁-C₄-alkoxycarbonyl;
or
R¹² and R¹⁶ together form a C₁-C₄-alkyl chain which may carry one to three radicals from the following group: halogen, cyano, C₁-C₄-alkyl, C₁-C₄-haloalkyl or C₁-C₄-alkoxycarbonyl;
R¹³ and R¹⁴ together form an -O-(CH₂)ₚ-O-, -O-(CH₂)ₚ-S-, - S-(CH₂)ₚ-S-, -O-(CH₂)_{q}- or -S-(CH₂)_{q}- chain which may be substituted by one to three radicals from the following group: halogen, cyano, C₁-C₄-alkyl, C₁-C₄-haloalkyl or C₁-C₄-alkoxycarbonyl;
or
R¹³ and R¹⁴ together with the carbon to which they are attached form a carbonyl group;
p is 2, 3 or 4;
q is 1, 2, 3, 4 or 5;
and their agriculturally useful salts.

2. A process for preparing tricyclic benzoylcyclohexanedione derivatives of the formula Iα (= I where R¹⁰ = hydroxyl) as claimed in claim 1, which comprises acylating a cyclohexanedione of the formula V in which the variables R¹¹ to R¹⁶ are as defined in claim 1, 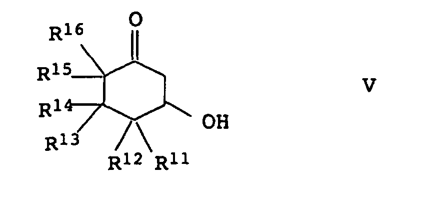 with an activated tricyclic benzoic acid of the formula VIα or with a tricyclic benzoic acid VIβ, 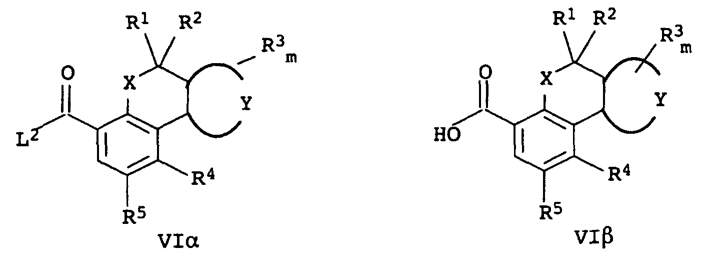 where the variables R¹ to R⁵, X, Y and m are as defined in claim 1 and L² is a nucleophilically replaceable leaving group and rearranging the acylation product, if appropriate in the presence of a catalyst.

3. A composition, comprising a herbicidally effective amount of at least one tricyclic benzoylcyclohexanedione derivative of the formula I or an agriculturally useful salt of I as claimed in claim 1 and auxiliaries which are customarily used for formulating the crop protection agents.

4. A process for preparing compositions as claimed in claim 3, which comprises mixing a herbicidally effective amount of at least one tricyclic benzoylcyclohexanedione derivative of the formula I or an agriculturally useful salt of I as claimed in claim 1 and auxiliaries which are customarily used for formulating crop protection agents.

5. A method for controlling undesirable vegetation, which comprises allowing a herbicidally effective amount of at least one tricyclic benzoylcyclohexanedione derivative of the formula I or an agriculturally useful salt of I as claimed in claim 1 to act on plants, their habitat and/or on seed.

6. The use of tricyclic benzoylcyclohexanedione derivatives of the formula I or their agriculturally useful salts as claimed in claim 1 as herbicides.

## Revendications

1. Dérivés de benzoylcyclohexanedione tricycliques de formule I 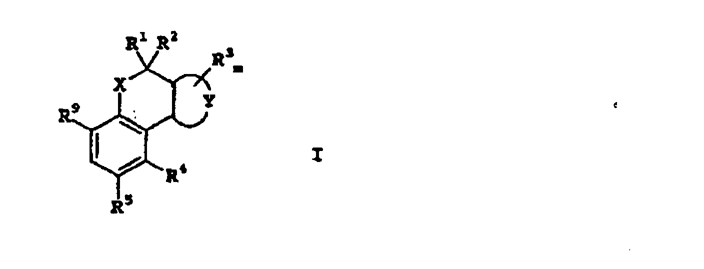 dans laquelle les variables ont les significations suivantes :
X représente un oxygène, un soufre, S=O ou S(=O)₂ ;
Y forme ensemble avec les deux atomes de carbone auxquels il est lié un hétérocycle à 5 chaînons saturé, partiellement saturé ou insaturé, qui contient de 1 à 3 hétéroatomes identiques ou différents, choisis dans le groupe suivant : oxygène, soufre ou azote ;
R¹, R² représentent un hydrogène, un alkyle en C₁ à C₆, un halogénalkyle en C₁ à C₆, un alcoxy en C₁ à C₆ ou un halogénalcoxy en C₁ à C₆ ;
R³ représente un halogène, un alkyle en C₁ à C₆, un halogénalkyle en C₁ à C₆, un alcoxy en C₁ à C₆ ou un halogénalcoxy en C₁ à C₆;
R⁴ représente un nitro, un halogène, un cyano, un alkyle en C₁ à C₆, un halogénalkyle en C₁ à C₆, un alcoxy en C₁ à C₆, un halogénalcoxy en C₁ à C₆, un alkylthio en C₁ à C₆, un halogénalkylthio en C₁ à C₆, un (alkyle en C₁ à C₆)sulfinyle, un (halogénalkyle en C₁ à C₆)sulfinyle, un (alkyle en C₁ à C₆)sulfonyle, un (halogénalkyle en C₁ à C₆)sulfonyle, un aminosulfonyle, un N-(alkyle en C₁ à C₆)aminosulfonyle, un N,N-di-(alkyle en C₁ à C₆)aminosulfonyle, un N-((alkyle en C₁ à C₆)sulfonyl)amino, un N-((halogénalkyle en C₁ à C₆)sulfonyl)amino, un N-(alkyle en C₁ à C₆)-N-((alkyle en C₁ à C₆)sulfonyl)amino ou un N-(alkyle en C₁ à C₆)-N-((halogénalkyle en C₁ à C₆)sulfonyl)amino ;
R⁵ représente un hydrogène, un alkyle en C₁ à C₆ ou un halogène ;
m vaut 0, 1 ou 2 ;
R⁹ représente un radical IIα ou IIβ 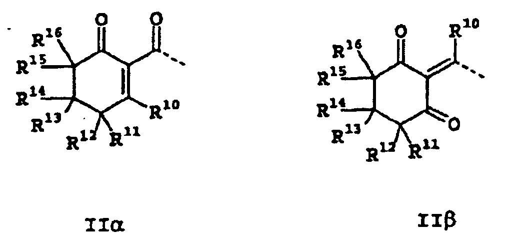
où les variables ont les significations suivantes :
R¹⁰ représente un hydroxy,
R¹¹, R¹⁵ représentent un hydrogène, un alkyle en C₁ à C₄ ou un (alcoxy en Ci à C₄)carbonyle ;
R¹², R¹⁴, R¹⁶ représentent un hydrogène ou un alkyle en C₁ à C₄ ;
R¹³ représente un hydrogène, un halogène, un hydroxy, un alkyle en C₁ à C₆, un halogénalkyle en C₁ à C₆, un di(alcoxy en C₁ à C₆)méthyle, un (alcoxy en C₁ à C₆)-(alkylthio en C₁ à C₆)méthyle, un di(alkylthio en C₁ à C₆)méthyle, un alcoxy en C₁ à C₆, un halogénalcoxy en C₁ à C₆, un alkylthio en C₁ à C₆, un halogénalkylthio en C₁ à C₆, un (alkyle en C₁ à C₆)sulfinyle, un (halogénalkyle en C₁ à C₆)sulfinyle, un (alkyle en C₁ à C₆)sulfonyle, un (halogénalkyle en C₁ à C₆)sulfonyle, un (alcoxy en C₁ à C₆)carbonyle, un (halogénalcoxy en C₁ à C₆)carbonyle ;
un 1,3-dioxolan-2-yle, un 1,3-dioxan-2-yle, un 1,3-oxathiolan-2-yle, un 1,3-oxathian-2-yle, un 1,3-dithiolan-2-yle ou un 1,3-dithian-2-yle, où les six derniers radicaux peuvent être substitués par de un à trois radicaux alkyle en C₁ à C₄ ; ou
R¹² et R¹³ ou R¹³ et R¹⁶ forment ensemble une liaison π ou une chaîne alkyle en C₁ à C₅ qui peut porter de un à trois radicaux du groupe suivant : halogène, cyano, alkyle en C₁ à C₄, halogénalkyle en C₁ à C₄ ou (alcoxy en C₁ à C₄)carbonyle ;
ou
R¹² et R¹⁶ forment ensemble une chaîne alkyle en C₁ à C₄ qui peut porter de un à trois radicaux des groupes suivants : halogène, cyano, alkyle en C₁ à C₄, halogénalkyle en C₁ à C₄ ou (alcoxy en C₁ à C₄)carbonyle ;
R¹³ et R¹⁴ forment ensemble une chaîne -O-(CH₂)ₚ-O-, -O-(CH₂)ₚ-S-, - S-(CH₂)ₚ-S-, -O-(CH₂)_{q}- ou -S-(CH₂)_{q}-, qui peut être substituée par de un à trois radicaux du groupe suivant :
halogène, cyano, alkyle en C₁ à C₄, halogénalkyle en C₁ à C₄ ou (alcoxy en C₁ à C₄)carbonyle ;
ou
R¹³ et R¹⁴ forment ensemble avec l'atome de carbone auquel ils sont liés un groupe carbonyle ;
p vaut 2, 3 ou 4 ;
q vaut 1, 2, 3, 4 ou 5 ;
ainsi que leurs sels utilisables en agriculture.

2. Procédé de préparation de dérivés de benzoylcyclohexanedione tricycliques de formule Iα (= I avec R¹⁰ = hydroxy) selon la revendication 1, **caractérisé en ce qu'**on acyle une cyclohexanedione de formule V dans laquelle les variables R¹¹ à R¹⁶ ont la signification donnée sous la revendication 1, 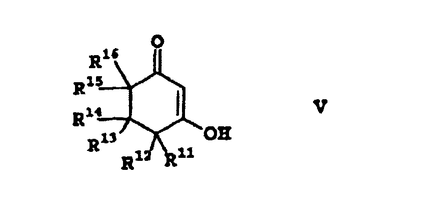 avec un acide benzoïque tricyclique activé de formule VIα ou avec un acide benzoïque tricyclique VIβ, 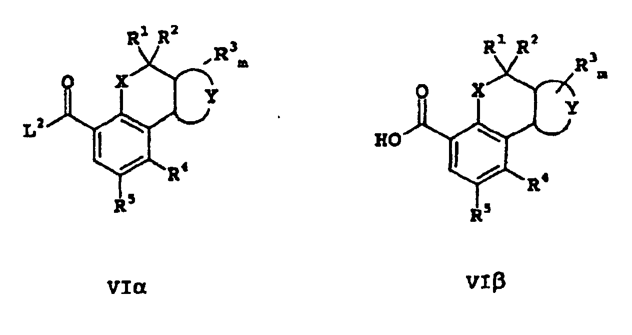 où les variables R¹ à R⁵, X, Y et m ont la signification mentionnée sous la revendication 1 et L² représente un groupe sortant apte à un déplacement nucléophile,
et **en ce que** le cas échéant on transpose le produit d'acylation en présence d'un catalyseur.

3. Agent contenant une quantité efficace comme herbicide d'au moins un dérivé de benzoylcyclohexanedione tricyclique de formule I ou d'un sel de I utilisable en agriculture selon la revendication 1 et des adjuvants habituels pour la formulation d'agents de protection des plantes.

4. Procédé de préparation d'agents selon la revendication 3, **caractérisé en ce qu'**on mélange une quantité efficace comme herbicide d'au moins un dérivé de benzoylcyclohexanedione tricyclique de formule I ou d'un sel de I utilisable en agriculture selon la revendication 1 et des adjuvants habituels pour la formulation d'agents de protection des plantes.

5. Procédé de lutte contre la croissance végétale indésirable, **caractérisé en ce qu'**on fait agir une quantité efficace comme herbicide d'au moins un dérivé de benzoylcyclohexanedione tricyclique de formule I ou d'un sel de I utilisable en agriculture selon la revendication 1, sur les plantes, leur habitat et/ou sur les semences.

6. Utilisation de dérivés de benzoylcyclohexanedione tricycliques de formule I ou de leurs sels utilisables en agriculture selon la revendication 1 comme herbicides.
